# EUROPEAN PATENT APPLICATION

(11) **EP 4 834 933 A1**
(43) Date of publication of application: **04.11.2026**
(21) Application number: 24911113.9
(22) Date of filing: 24.12.2024
(51) Int. Cl.: C07K 16/28, A61P 37/02, G01N 33/577, C12N 15/13, A61K 39/395, A61P 1/00, A61P 1/04

(54) **ANTI-TL1A ANTIBODY OR ANTIGEN-BINDING FRAGMENT THEREOF AND USE THEREOF**

(30) Priority: 29.12.2023 CN 202311855546
(71) Applicant: Chengdu Unovel Biopharma Co., Ltd., Chengdu, Sichuan 610000 (CN)
(72) Inventor: LI, Zhushi, Chengdu, Sichuan 610000 (CN); ZHU, Yan, Chengdu, Sichuan 610000 (CN); DENG, Tao, Chengdu, Sichuan 610000 (CN); ZHAO, Zhenhu, Chengdu, Sichuan 610000 (CN); LONG, Yangyang, Chengdu, Sichuan 610000 (CN); WANG, Ying, Chengdu, Sichuan 610000 (CN)
(74) Representative: Puchberger & Partner Patentanwälte
(86) International application number: PCT/CN2024/141823
(87) International publication number: WO 2025/140188

(57) **Abstract**

The present invention relates to an anti-TL1A antibody or an antigen-binding fragment thereof and the use thereof. The anti-TL1A antibody or the antigen-binding fragment thereof comprises complementarity determining regions HCDR1, HCDR2 and HCDR3 of a heavy chain variable region and/or complementarity determining regions LCDR1, LCDR2 and LCDR3 of a light chain variable region. The anti-TL1A antibody or the antigen-binding fragment thereof can block, inhibit or reduce the activity of TL1A regulation, and can specifically bind to TL1A, so as to prevent and/or treat TL1A-mediated related diseases or disorders.

## Description

### TECHNICAL FIELD

The present application relates to the technical field of biomedicine, and specifically to an anti-TL1A antibody or an antigen-binding fragment thereof and use thereof.

### BACKGROUND

Proteins structurally related to tumor necrosis factor (TNF) are collectively referred to as the TNF superfamily. Tumor necrosis factor-like ligand 1A (TL1A) is a member of the TNF family of cytokines, also known as TNFSF15. TL1A is the only known ligand for its receptor "death receptor 3" (DR3) (also known as TNFRSF25). TL1A expression on antigen-presenting cells (monocytes, macrophages, dendritic cells) and DR3 expression on effector cells (T cells, NK cells and NKT cells) are highly dependent on pro-inflammatory conditions. In vivo and in vitro evidence confirms the costimulatory role of the TL1A/DR3 pathway in enhancing T cell and effector cell functions, inflammatory cell expansion, and cytokine secretion. Furthermore, this pathway is involved in the regulation of pathogenic Th1, Th2 and Th17 T-helper cell responses and NK and NKT cell responses in immune-mediated diseases.

Current studies on DR3- or TL1A -deficient mice or mice treated with anti-TL1A antibodies have demonstrated the role of this pathway in various autoimmune disease models, such as inflammatory bowel disease (IBD), asthma, multiple sclerosis, and arthritis. IBD refers to a range of intestinal disorders that cause inflammatory conditions in the gastrointestinal tract. Severe forms of IBD may be characterized by intestinal fibrosis, which is the accumulation of scar tissue in the intestinal wall. The main types of IBD are ulcerative colitis (UC) and Crohn's disease (CD). Both UC and CD are chronic, relapsing, remitting inflammatory conditions of the gastrointestinal tract that most often begin during adolescence and young adulthood. UC affects the mucosal layer of the large intestine, and symptoms include abdominal pain and diarrhea, often accompanied by blood and mucus. CD can affect the entire thickness of the intestinal wall and all parts of the GI tract from the mouth to the anus. Symptoms of CD include abdominal pain, diarrhea, and other more insidious symptoms such as weight loss, nutritional deficiencies, and fever.

Available therapies for IBD patients are limited. Moreover, a considerable number of patients have no response or lose response to existing anti-inflammatory therapies. When patients receive such ineffective anti-inflammatory treatment, the disease worsens. Currently, the therapy for patients who do not respond to first-line treatment is surgery, namely strictureplasty (intestinal plastic surgery) or resection (bowel resection). Surgical treatment for IBD is invasive, and it is estimated that one-third of patients experience postoperative risks such as anastomotic leakage, infection, and bleeding after surgery.

The pathogenesis of IBD is believed to involve an uncontrolled immune response that may be triggered by certain environmental factors in a genetically susceptible host. The heterogeneity of disease pathogenesis and clinical course, combined with variable responses to treatments and their associated side effects, suggests that a targeted therapeutic approach for treating these diseases is an ideal treatment strategy. However, targeted therapies are very limited for IBD patients, especially those who may not respond to existing IBD therapies.

Therefore, the development of novel therapies for IBD and for treating other TL1A-mediated diseases and conditions remains an unmet clinical need.

### SUMMARY OF THE INVENTION

In view of the prior art, the present application provides an anti-TL1A antibody or an antigen-binding fragment thereof and a use thereof, wherein the anti-TL1A antibody or the antigen-binding fragment thereof comprises complementarity determining regions HCDR1, HCDR2, HCDR3 of a heavy chain variable region and/or complementarity determining regions LCDR1, LCDR2, LCDR3 of a light chain variable region; the anti-TL1A antibody or the antigen-binding fragment thereof can block, inhibit or reduce TL1A-regulated activity, and can specifically bind to TL1A, so as to prevent and/or treat TL1A-mediated related diseases or disorders.

A first aspect of the present application provides an anti-TL1A antibody or an antigen-binding fragment thereof, comprising:
(a) a heavy chain variable region comprising the following complementarity determining regions:
   HCDR1, comprising the amino acid sequence set forth in SEQ ID NO: 1, 7, 13, 19, 25, 28, 34, 40 or 46, or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 1, 7, 13, 19, 25, 28, 34, 40 or 46, or an amino acid sequence obtained by substitution, deletion or addition of one or more amino acids in the amino acid sequence set forth in SEQ ID NO: 1, 7, 13, 19, 25, 28, 34, 40 or 46 and having the same or similar function to the amino acid sequence set forth in SEQ ID NO: 1, 7, 13, 19, 25, 28, 34, 40 or 46;
   HCDR2, comprising the amino acid sequence set forth in SEQ ID NO: 2, 8, 14, 20, 29, 35, 41 or 47, or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 2, 8, 14, 20, 29, 35, 41 or 47, or an amino acid sequence obtained by substitution, deletion or addition of one or more amino acids in the amino acid sequence set forth in SEQ ID NO: 2, 8, 14, 20, 29, 35, 41 or 47 and having the same or similar function to the amino acid sequence set forth in SEQ ID NO: 2, 8, 14, 20, 29, 35, 41 or 47; and
   HCDR3, comprising the amino acid sequence set forth in SEQ ID NO: 3, 9, 15, 21, 26, 30, 36, 42 or 48, or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 3, 9, 15, 21, 26, 30, 36, 42 or 48, or an amino acid sequence obtained by substitution, deletion or addition of one or more amino acids in the amino acid sequence set forth in SEQ ID NO: 3, 9, 15, 21, 26, 30, 36, 42 or 48 and having the same or similar function to the amino acid sequence set forth in SEQ ID NO: 3, 9, 15, 21, 26, 30, 36, 42 or 48; and
(b) a light chain variable region comprising the following complementarity determining regions:
   LCDR1, comprising the amino acid sequence set forth in SEQ ID NO: 4, 10, 16, 22, 31, 37, 43 or 49, or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 4, 10, 16, 22, 31, 37, 43 or 49, or an amino acid sequence obtained by substitution, deletion or addition of one or more amino acids in the amino acid sequence set forth in SEQ ID NO: 4, 10, 16, 22, 31, 37, 43 or 49 and having the same or similar function to the amino acid sequence set forth in SEQ ID NO: 4, 10, 16, 22, 31, 37, 43 or 49;
   LCDR2, comprising the amino acid sequence set forth in SEQ ID NO: 5, 11, 17, 23, 32, 38, 44 or 50, or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 5, 11, 17, 23, 32, 38, 44 or 50, or an amino acid sequence obtained by substitution, deletion or addition of one or more amino acids in the amino acid sequence set forth in SEQ ID NO: 5, 11, 17, 23, 32, 38, 44 or 50 and having the same or similar function to the amino acid sequence set forth in SEQ ID NO: 5, 11, 17, 23, 32, 38, 44 or 50; and
   LCDR3, comprising the amino acid sequence set forth in SEQ ID NO: 6, 12, 18, 24, 27, 33, 39, 45 or 51, or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 6, 12, 18, 24, 27, 33, 39, 45 or 51, or an amino acid sequence obtained by substitution, deletion or addition of one or more amino acids in the amino acid sequence set forth in SEQ ID NO: 6, 12, 18, 24, 27, 33, 39, 45 or 51 and having the same or similar function to the amino acid sequence set forth in SEQ ID NO: 6, 12, 18, 24, 27, 33, 39, 45 or 51.

As used herein, the substitution, deletion or addition of one or more amino acids means substitution, deletion or addition of 1 or 2 amino acids. In some embodiments, the substitution, deletion or addition of one or more amino acids is a conservative modification.

In some embodiments, compared to the amino acid sequences set forth in SEQ ID NOs: 1-3, 7-9, 13-15, 19-21, 25-26, 28-30, 34-36, 40-42 and 46-48, the differing amino acids in the amino acid sequence having at least 80% identity thereto or the amino acid sequence having substitution, deletion or addition of one or more amino acids are located mainly or entirely in the FR regions of the heavy chain variable region. In some embodiments, compared to the amino acid sequences set forth in SEQ ID NOs: 4-6, 10-12, 16-18, 22-24, 27, 31-33, 37-39, 43-45 and 49-51, the differing amino acids in the amino acid sequence having at least 80% identity thereto or the amino acid sequence having substitution, deletion or addition of one or more amino acids are located mainly or entirely in the FR regions of the light chain variable region. In some embodiments, the anti-TL1A antibody or the antigen-binding fragment thereof comprises:
(1) HCDR1, HCDR2, HCDR3, LCDR1, LCDR2 and LCDR3, wherein the HCDR1, HCDR2 and HCDR3 have the amino acid sequences set forth in SEQ ID NOs: 1, 2 and 3, respectively, and the LCDR1, LCDR2 and LCDR3 have the amino acid sequences set forth in SEQ ID NOs: 4, 5 and 6, respectively;
(2) HCDR1, HCDR2, HCDR3, LCDR1, LCDR2 and LCDR3, wherein the HCDR1, HCDR2 and HCDR3 have the amino acid sequences set forth in SEQ ID NOs: 7, 8 and 9, respectively, and the LCDR1, LCDR2 and LCDR3 have the amino acid sequences set forth in SEQ ID NOs: 10, 11 and 12, respectively;
(3) HCDR1, HCDR2, HCDR3, LCDR1, LCDR2 and LCDR3, wherein the HCDR1, HCDR2 and HCDR3 have the amino acid sequences set forth in SEQ ID NOs: 13, 14 and 15, respectively, and the LCDR1, LCDR2 and LCDR3 have the amino acid sequences set forth in SEQ ID NOs: 16, 17 and 18, respectively;
(4) HCDR1, HCDR2, HCDR3, LCDR1, LCDR2 and LCDR3, wherein the HCDR1, HCDR2 and HCDR3 have the amino acid sequences set forth in SEQ ID NOs: 19, 20 and 21, respectively, and the LCDR1, LCDR2 and LCDR3 have the amino acid sequences set forth in SEQ ID NOs: 22, 23 and 24, respectively;
(5) HCDR1, HCDR2, HCDR3, LCDR1, LCDR2 and LCDR3, wherein the HCDR1, HCDR2 and HCDR3 have the amino acid sequences set forth in SEQ ID NOs: 25, 20 and 26, respectively, and the LCDR1, LCDR2 and LCDR3 have the amino acid sequences set forth in SEQ ID NOs: 22, 23 and 27, respectively;
(6) HCDR1, HCDR2, HCDR3, LCDR1, LCDR2 and LCDR3, wherein the HCDR1, HCDR2 and HCDR3 have the amino acid sequences set forth in SEQ ID NOs: 28, 29 and 30, respectively, and the LCDR1, LCDR2 and LCDR3 have the amino acid sequences set forth in SEQ ID NOs: 31, 32 and 33, respectively;
(7) HCDR1, HCDR2, HCDR3, LCDR1, LCDR2 and LCDR3, wherein the HCDR1, HCDR2 and HCDR3 have the amino acid sequences set forth in SEQ ID NOs: 34, 35 and 36, respectively, and the LCDR1, LCDR2 and LCDR3 have the amino acid sequences set forth in SEQ ID NOs: 37, 38 and 39, respectively;
(8) HCDR1, HCDR2, HCDR3, LCDR1, LCDR2 and LCDR3, wherein the HCDR1, HCDR2 and HCDR3 have the amino acid sequences set forth in SEQ ID NOs: 40, 41 and 42, respectively, and the LCDR1, LCDR2 and LCDR3 have the amino acid sequences set forth in SEQ ID NOs: 43, 44 and 45, respectively; or
(9) HCDR1, HCDR2, HCDR3, LCDR1, LCDR2 and LCDR3, wherein the HCDR1, HCDR2 and HCDR3 have the amino acid sequences set forth in SEQ ID NOs: 46, 47 and 48, respectively, and the LCDR1, LCDR2 and LCDR3 have the amino acid sequences set forth in SEQ ID NOs: 49, 50 and 51, respectively.

In preferred embodiments, the anti-TL1A antibody or the antigen-binding fragment thereof comprises:
(1) HCDR1, HCDR2, HCDR3, LCDR1, LCDR2 and LCDR3, wherein the HCDR1, HCDR2 and HCDR3 have the amino acid sequences set forth in SEQ ID NOs: 1, 2 and 3, respectively, and the LCDR1, LCDR2 and LCDR3 have the amino acid sequences set forth in SEQ ID NOs: 4, 5 and 6, respectively;
(2) HCDR1, HCDR2, HCDR3, LCDR1, LCDR2 and LCDR3, wherein the HCDR1, HCDR2 and HCDR3 have the amino acid sequences set forth in SEQ ID NOs: 7, 8 and 9, respectively, and the LCDR1, LCDR2 and LCDR3 have the amino acid sequences set forth in SEQ ID NOs: 10, 11 and 12, respectively; or
(3) HCDR1, HCDR2, HCDR3, LCDR1, LCDR2 and LCDR3, wherein the HCDR1, HCDR2 and HCDR3 have the amino acid sequences set forth in SEQ ID NOs: 13, 14 and 15, respectively, and the LCDR1, LCDR2 and LCDR3 have the amino acid sequences set forth in SEQ ID NOs: 16, 17 and 18, respectively.

In some embodiments, the anti-TL1A antibody or the antigen-binding fragment thereof is selected from 16H6, 12B3, 56C8, 5C6, 28G7, 70A3, 51G9, 69B1, 13B8 and humanized antibodies thereof (e.g., 16H6-H1L1, 16H6-H1L2, 16H6-H1L3, 16H6-H1L4, 16H6-H2L1, 16H6-H2L2, 16H6-H2L3, 16H6-H2L4, 16H6-H3L1, 16H6-H3L2, 16H6-H3L3, 16H6-H3L4, 16H6-H4L1, 16H6-H4L2, 16H6-H4L3, 16H6-H4L4, 12B3-H1L1, 12B3-H1L2, 12B3-H1L4, 12B3-H1L5, 12B3-H1L6, 12B3-H1L8, 12B3-H2L1, 12B3-H2L2, 12B3-H2L4, 12B3-H2L5, 12B3-H3L1, 12B3-H3L2, 12B3-H3L4, 12B3-H3L5, 12B3-H3L6, 12B3-H3L8, 12B3-H4L1, 12B3-H4L2, 12B3-H4L4, 12B3-H4L5, 12B3-H4L6, 12B3-H4L8, 12B3-H6L1, 12B3-H6L4, 12B3-H6L6, 12B3-H6L8, 12B3-H7L1, 12B3-H7L4, 12B3-H7L6, 12B3-H7L8, 12B3-H8L1, 12B3-H8L6, 12B3-H8L8) or an antigen-binding fragment thereof.

In some embodiments, the antibody is a murine antibody, a monkey antibody, a rabbit antibody, a chimeric antibody, a humanized antibody, or a fully human antibody.

In some embodiments, the antibody is a murine antibody.

In some embodiments, the antibody is a chimeric antibody comprising an extracellular antigen-binding domain, a transmembrane domain and an intracellular signal transduction domain, wherein the extracellular antigen-binding domain comprises the anti-TL1A antibody or the antigen-binding fragment thereof described herein.

In some embodiments, the anti-TL1A antibody or the antigen-binding fragment thereof is at least one selected from a monoclonal antibody, a polyclonal antibody, a neutralizing antibody, an antagonistic antibody, an anti-idiotypic antibody, an afucosylated antibody, a monospecific antibody, a multispecific antibody, a nanobody, a Fab fragment, a Fab' fragment, a F(ab')2 fragment, an Fd fragment, an Fv fragment, a dAb fragment, an isolated CDR region, and an scFv; the anti-TL1A antibody is preferably a monoclonal antibody or a multispecific antibody; preferably, the multispecific antibody is a bispecific antibody, a trispecific antibody or a tetraspecific antibody; or preferably, the multispecific antibody further comprises at least one second antibody having a second binding specificity for a second target.

In some embodiments, the antibody is a monoclonal antibody.

In some embodiments, the anti-TL1A antibody or the antigen-binding fragment thereof further comprises a heavy chain constant region and/or a light chain constant region.

Preferably, the heavy chain constant region is a heavy chain constant region of IgG, IgA, IgM, IgE or IgD or a variant thereof; the heavy chain constant region is preferably a heavy chain constant region of IgG (e.g., human IgG) or a variant thereof, for example, a constant region of IgG1, IgG2, IgG3 or IgG4 or a variant thereof; the heavy chain constant region is more preferably a heavy chain constant region of human IgG1 or a variant thereof, for example, a heavy chain constant region of human IgG1 or a variant thereof.

Preferably, the light chain constant region is a constant region of a human κ chain or λ chain or a variant thereof.

In some embodiments, the heavy chain constant region comprises a full-length heavy chain constant region or a fragment thereof, and the fragment can be selected from a CH1 region, a CH2 region, an Fc region and a CH3 region.

As non-limiting examples, the anti-TL1A antibody or the antigen-binding fragment thereof has one or more of the following characteristics:
(1) capable of binding to human TL1A;
(2) capable of binding to non-human mammalian TL1A, e.g., murine TL1A, rabbit TL1A, feline TL1A , canine TL1A or porcine TL1A; preferably, capable of binding to murine TL1A;
(3) capable of binding to non-human primate TL1A; preferably, capable of binding to cynomolgus monkey TL1A;
(4) capable of cross-reacting with human TL1A, non-human mammalian TL1A and non-human primate TL1A; preferably, capable of cross-reacting with human TL1A, murine TL1A and cynomolgus monkey TL1A;
(5) having a lower affinity for a human homologue of TL1A than for human TL1A, wherein the human homologue of TL1A is selected from TNFSF6, TNFSF10, TNFSF14, TNF-β, TNF-α, lymphotoxin α2-β1 and lymphotoxin α1-β2;
(6) blocking, inhibiting or reducing the interaction between TL1A and the receptor DR3 (TNFRSF25);
(7) blocking, inhibiting or reducing a signaling pathway triggered by TL1A;
(8) blocking, inhibiting or reducing TL1A-regulated activity; and
(9) blocking, inhibiting or reducing a TL1A-mediated cellular response; for example, lymphocyte proliferation, cytokine expression or lymphocyte survival.

In some embodiments, the anti-TL1A antibody or the antigen-binding fragment thereof has a blocking effect on TL1A-induced caspase activity in TF-1 cells.

In some embodiments, the anti-TL1A antibody or the antigen-binding fragment thereof is capable of cross-binding to human TL1A, cynomolgus monkey TL1A, mouse TL1A and rat TL1A.

In some embodiments, the anti-TL1A antibody or the antigen-binding fragment thereof is capable of specifically binding to human TL1A, mouse TL1A, rat TL1A and cynomolgus monkey TL1A.

A second aspect of the present application provides a nucleic acid molecule, wherein the nucleic acid molecule encodes the anti-TL1A antibody or the antigen-binding fragment thereof according to the first aspect.

A third aspect of the present application provides a recombinant vector comprising the nucleic acid molecule according to the first aspect.

A fourth aspect of the present application provides a host cell comprising the nucleic acid molecule according to the second aspect or the recombinant vector according to the third aspect.

A fifth aspect of the present application provides a method for preparing an anti-TL1A antibody or an antigen-binding fragment thereof, comprising culturing the host cell according to the fourth aspect. The method may further comprise isolating and/or purifying the antibody or the fragment thereof from the cell culture after the expression or culturing step, for example, purifying using a Protein A affinity column.

In some embodiments, the host cell is a prokaryotic cell. In other embodiments, the host cell is a eukaryotic cell.

In some embodiments, the host cell is selected from yeast cells, mammalian cells, and other cells suitable for preparing an antibody or an antigen-binding fragment thereof, or a moiety thereof. The mammalian cell is, for example, a Chinese hamster ovary (CHO) cell, a human embryonic kidney cell line 293 cell or a 293T cell. In some embodiments, the host cell is a recombinant CHO cell. In some embodiments, the method further comprises isolating and purifying the expression product of the host cell, for example, purifying using a Protein A affinity column.

A sixth aspect of the present application provides the anti-TL1A antibody or the antigen-binding fragment thereof according to the first aspect, the nucleic acid molecule according to the second aspect, the recombinant vector according to the third aspect, or the host cell according to the fourth aspect, for use in preventing and/or treating a TL1A-mediated related disease or disorder.

In some embodiments, the present application relates to use of the anti-TL1A antibody or the antigen-binding fragment thereof according to the first aspect, the nucleic acid molecule according to the second aspect, the recombinant vector according to the third aspect, or the host cell according to the fourth aspect in the manufacture of a medicament for preventing and/or treating a TL1A-mediated related disease or disorder. Alternatively, the present application relates to a method for preventing and/or treating a TL1A-mediated related disease or disorder, comprising administering the anti-TL1A antibody or the antigen-binding fragment thereof according to the first aspect, the nucleic acid molecule according to the second aspect, the recombinant vector according to the third aspect, or the host cell according to the fourth aspect to a subject in need thereof. Alternatively, the present application relates to the anti-TL1A antibody or the antigen-binding fragment thereof according to the first aspect, the nucleic acid molecule according to the second aspect, the recombinant vector according to the third aspect, or the host cell according to the fourth aspect, for use in preventing and/or treating a TL1A-mediated related disease or disorder.

In some embodiments, the disease or disorder is prevented, alleviated, ameliorated or inhibited by eliminating, inhibiting or reducing TL1A activity.

In some embodiments, the disease or disorder is an immune-related or inflammatory disease.

In some embodiments, the disease or disorder is at least one selected from the group consisting of inflammatory bowel disease (IBD), irritable bowel syndrome (IBS), systemic sclerosis-associated interstitial lung disease, idiopathic pulmonary fibrosis, virus-induced pulmonary fibrosis, asthma, chronic obstructive pulmonary disease (COPD), allergic pulmonary inflammation, neurodegenerative disease, cancer, diabetes, arthritis, rheumatoid arthritis, juvenile rheumatoid arthritis, osteoarthritis, psoriatic arthritis, ankylosing spondylitis, multiple sclerosis, transplant rejection, graft-versus-host disease (GVHD), spondyloarthropathy, primary sclerosing cholangitis, primary biliary cirrhosis, atherosclerosis, bladder syndrome, interstitial cystitis, intestinal and urinary dysfunction, sepsis, uveitis, encephalomyelitis, myasthenia gravis, systemic lupus erythematosus, cutaneous lupus erythematosus, autoimmune thyroiditis, atopic dermatitis, eczematous dermatitis, psoriasis, Sjögren's syndrome, scleroderma, and vasculitis.

In the present application, the inflammatory bowel disease comprises Crohn's disease and ulcerative colitis.

In some embodiments, the disease or disorder is inflammatory bowel disease.

A seventh aspect of the present application provides a kit for detecting TL1A, comprising the anti-TL1A antibody or the antigen-binding fragment thereof according to the first aspect, and/or the nucleic acid molecule according to the second aspect.

Beneficial effects of the present application:
The anti-TL1A antibody or the antigen-binding fragment thereof of the present application has high affinity for TL1A and high biological activity, and can effectively block, inhibit or reduce the interaction between TL1A and its receptor DR3. The anti-TL1A antibody or the antigen-binding fragment thereof of the present application can block, inhibit or reduce TL1A-regulated activity, and has a blocking effect on TL1A-induced caspase activity in TF-1 cells.

The anti-TL1A antibody or the antigen-binding fragment thereof of the present application can cross-bind to TL1A from human, non-human primates (e.g., cynomolgus monkey), and non-human mammals (e.g., mouse, rat), can specifically bind to TL1A, specifically to TL1A from human, non-human primates (e.g., cynomolgus monkey), and non-human mammals (e.g., mouse, rat), and has good stability.

The anti-TL1A antibody or the antigen-binding fragment thereof of the present application has strong binding to both human TL1A monomer and multimer (e.g., trimer).

### BRIEF DESCRIPTION OF THE DRAWINGS

Figure 1 is a graph showing the results of TL1A-induced reporter gene signal in Example 1.
Figure 2 is a graph showing the results of the activity detection of positive control antibody 1D1 by reporter gene assay in Example 1.
Figure 3-1 is a graph showing the results of the activity detection of chimeric antibody 5C6 by reporter gene assay in Example 4.
Figure 3-2 is a graph showing the results of the activity detection of chimeric antibodies 28G7 and 16H6 by reporter gene assay in Example 4.
Figure 3-3 is a graph showing the results of the activity detection of chimeric antibodies 51G9, 69B1, 12B3 and 56C8 by reporter gene assay in Example 4.

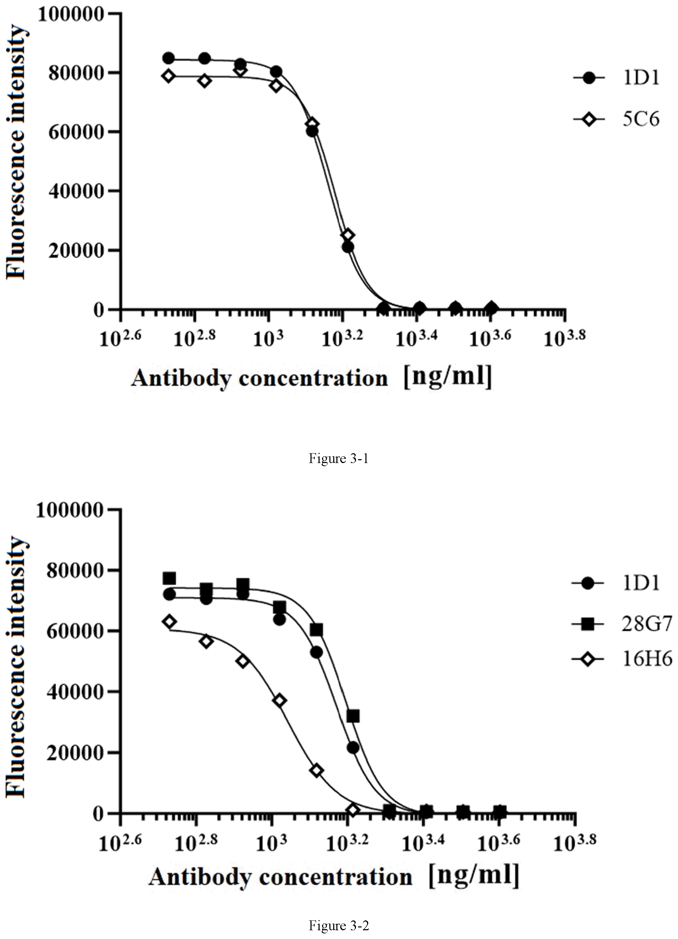

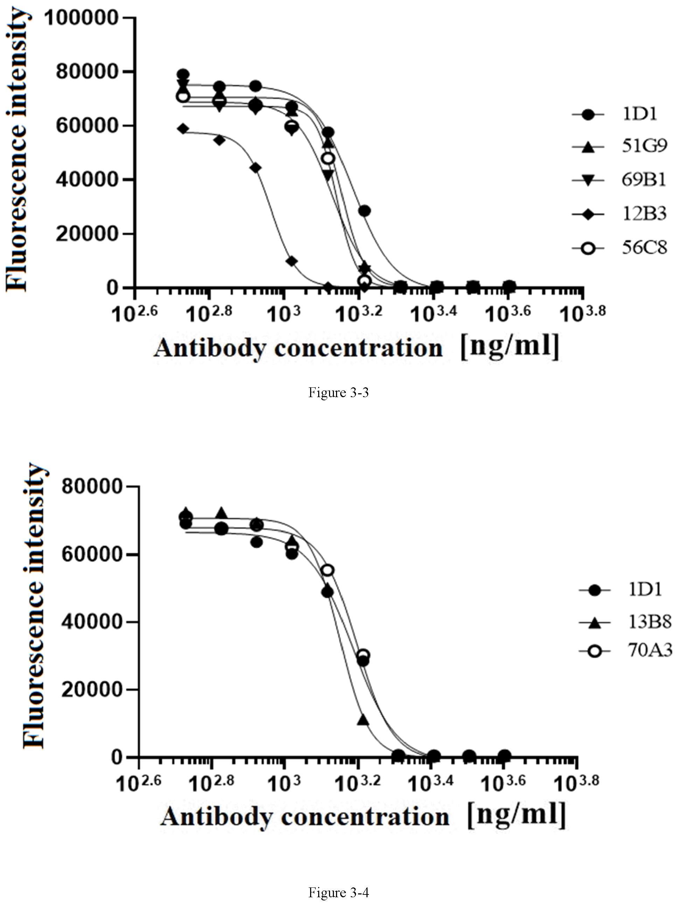

Figure 3-4 is a graph showing the results of the activity detection of chimeric antibodies 13B8 and 70A3 by reporter gene assay in Example 4.

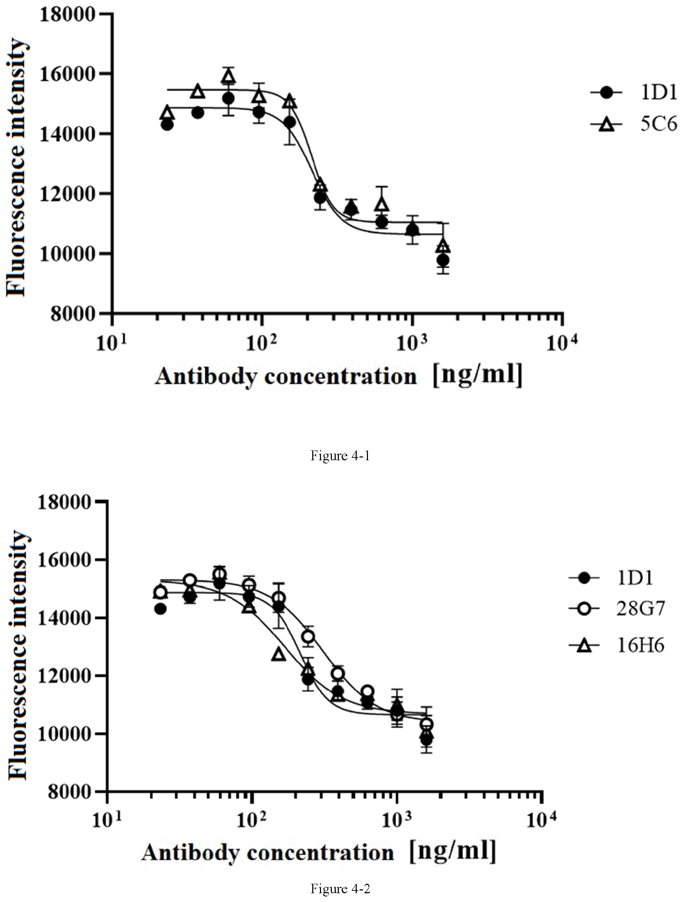

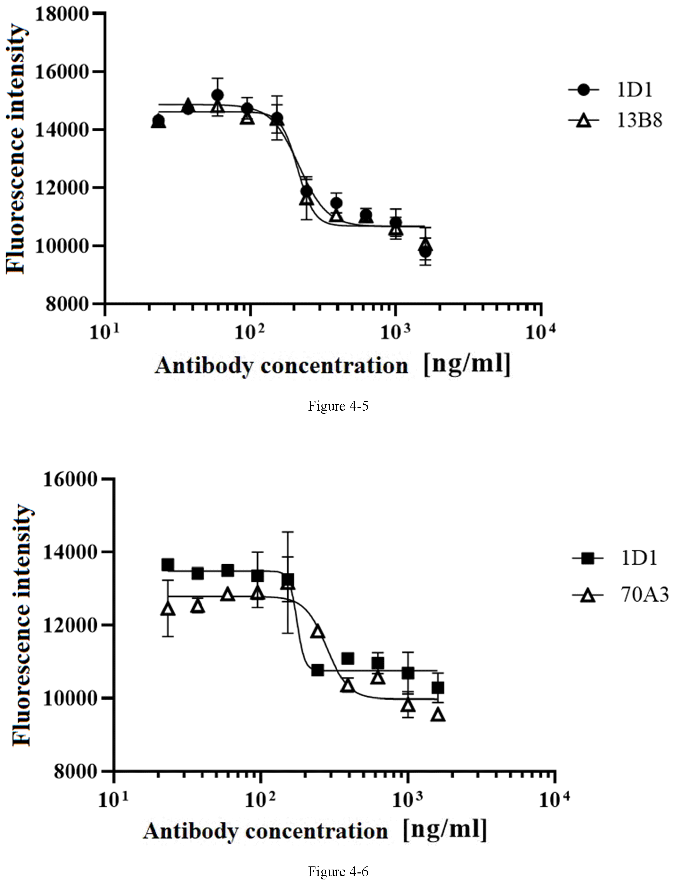

Figure 4-1 is a graph showing the results of the activity detection of chimeric antibody 5C6 by caspase assay in Example 5.
Figure 4-2 is a graph showing the results of the activity detection of chimeric antibodies 28G7 and 16H6 by caspase assay in Example 5.

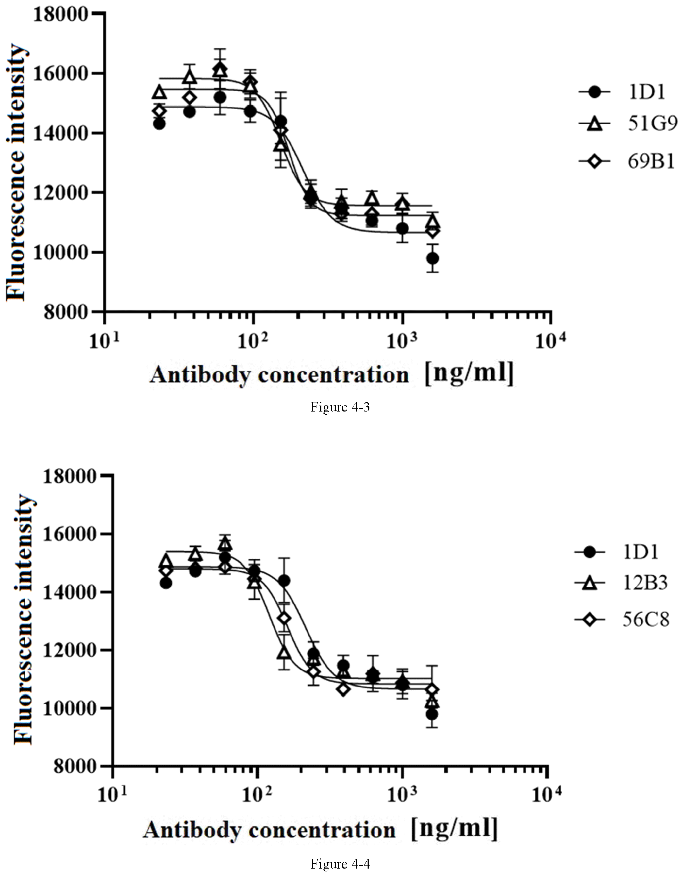

Figure 4-3 is a graph showing the results of the activity detection of chimeric antibodies 51G9 and 69B1 by caspase assay in Example 5.
Figure 4-4 is a graph showing the results of the activity detection of chimeric antibodies 12B3 and 56C8 by caspase assay in Example 5.
Figure 4-5 is a graph showing the results of the activity detection of chimeric antibody 13B8 by caspase assay in Example 5.
Figure 4-6 is a graph showing the results of the activity detection of chimeric antibody 70A3 by caspase assay in Example 5.

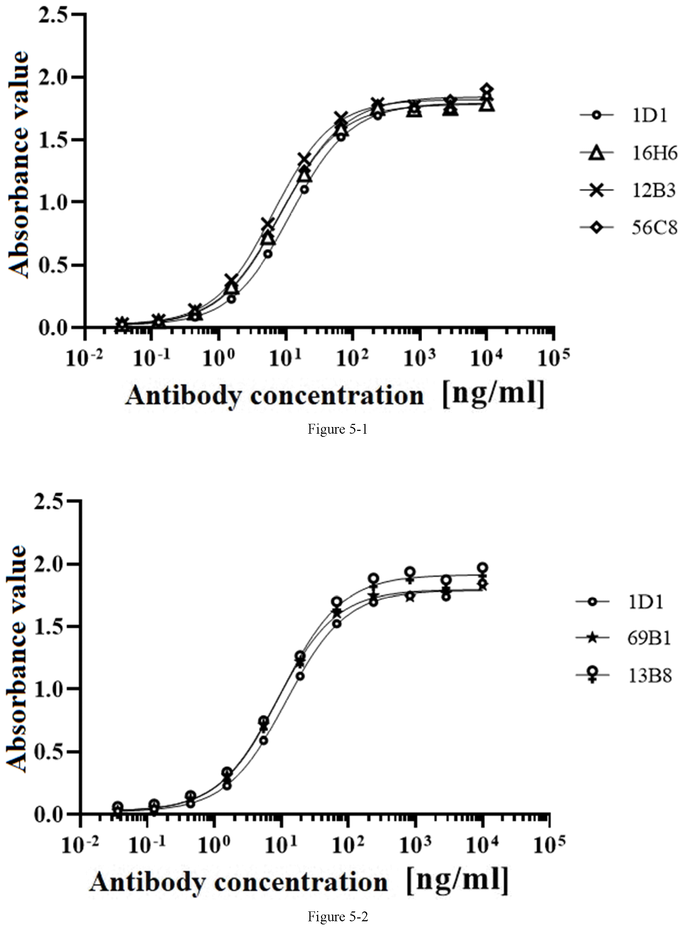

Figure 5-1 is a graph showing the results of the binding activity detection of chimeric antibodies 16H6, 12B3 and 56C8 to human TL1A monomer in Example 6.
Figure 5-2 is a graph showing the results of the binding activity detection of chimeric antibodies 69B1 and 13B8 to human TL1A monomer in Example 6.

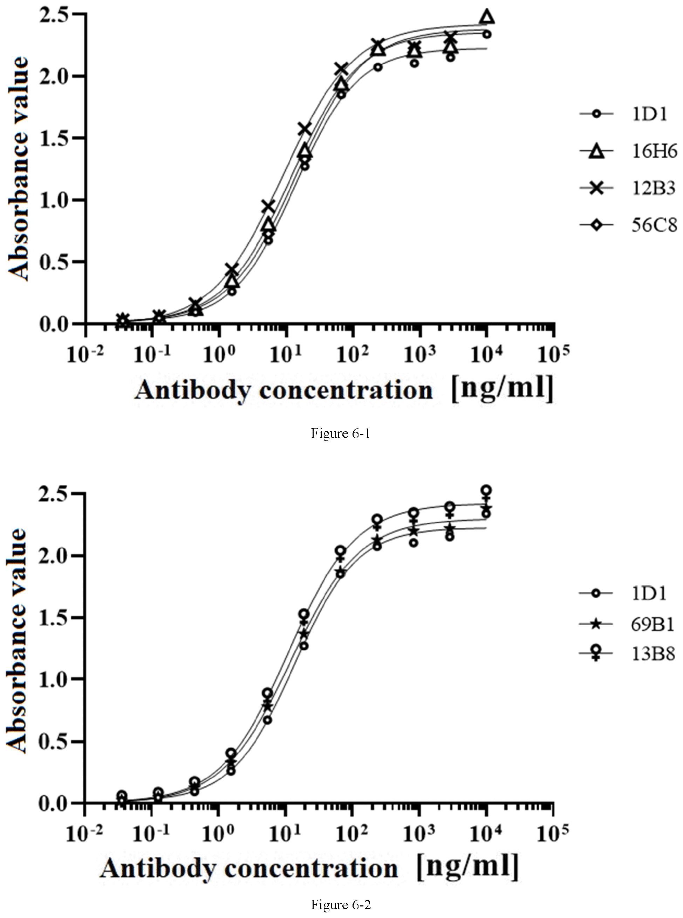

Figure 6-1 is a graph showing the results of the binding activity detection of chimeric antibodies 16H6, 12B3 and 56C8 to human TL1A trimer in Example 6.
Figure 6-2 is a graph showing the results of the binding activity detection of chimeric antibodies 69B1 and 13B8 to human TL1A trimer in Example 6.

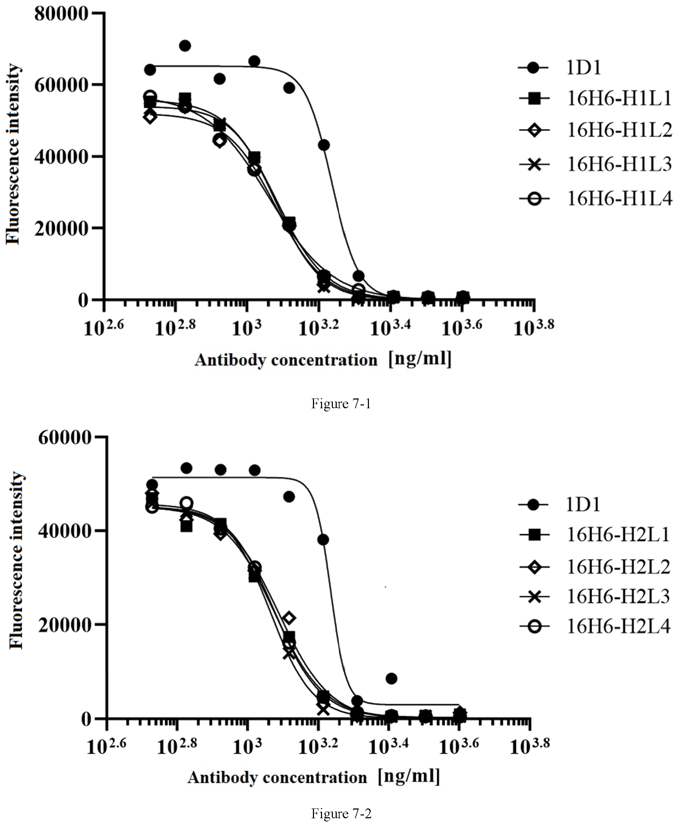

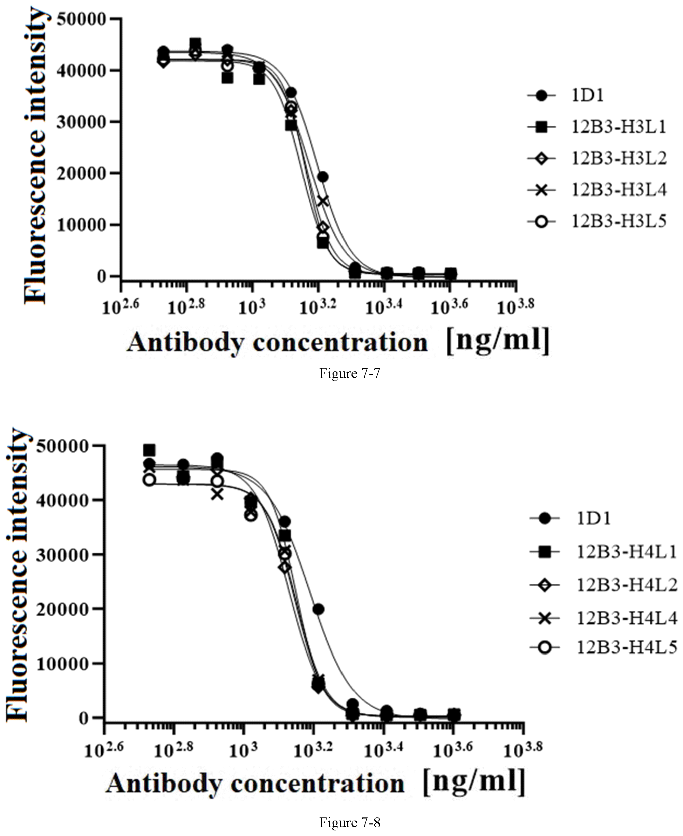

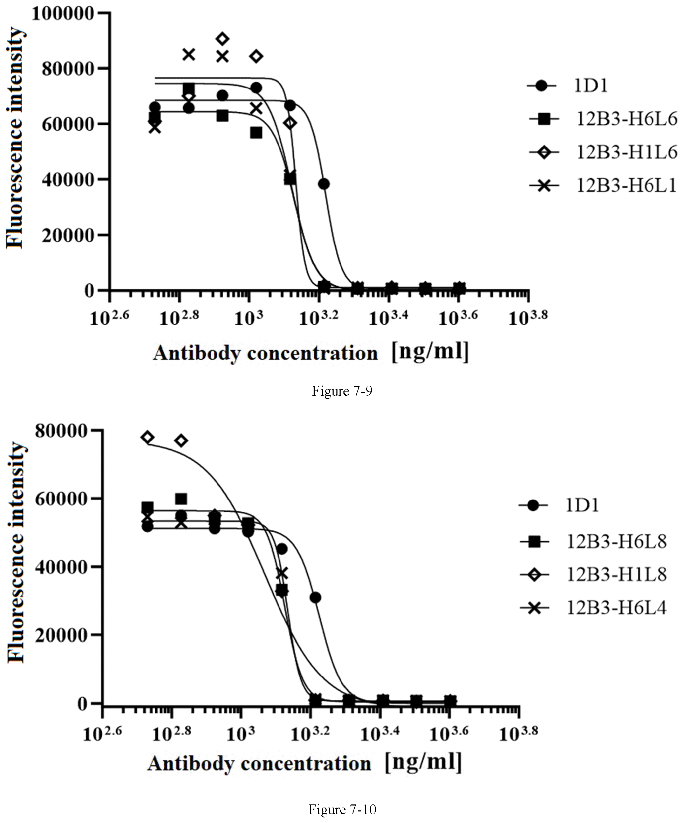

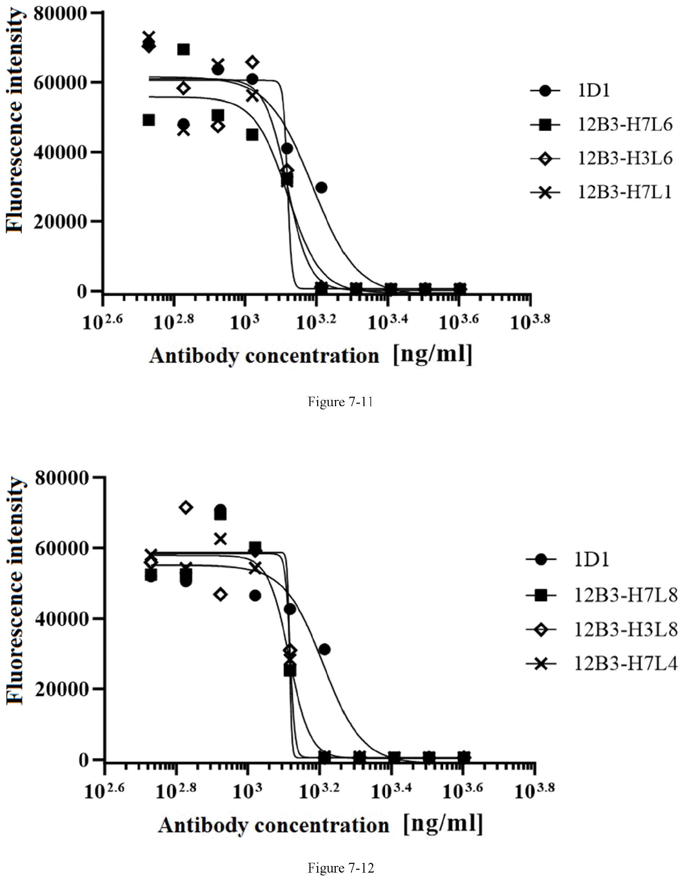

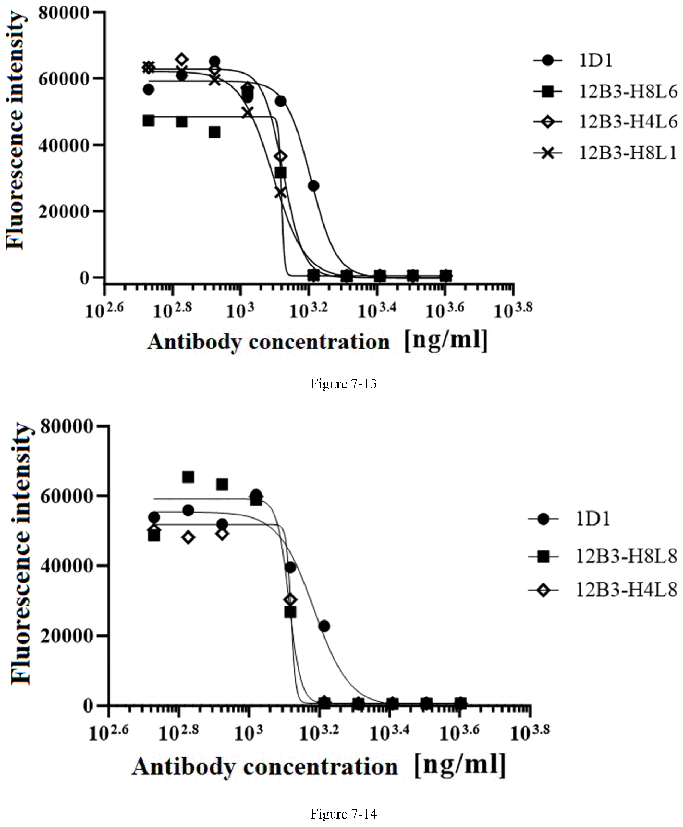

Figure 7-1 is a graph showing the results of the activity detection of humanized antibodies 16H6-H1L1, 16H6-H1L2, 16H6-H1L3 and 16H6-H1L4 against human TL1A trimer by reporter gene assay in Example 8.
Figure 7-2 is a graph showing the results of the activity detection of humanized antibodies 16H6-H2L1, 16H6-H2L2, 16H6-H2L3 and 16H6-H2L4 against human TL1A trimer by reporter gene assay in Example 8.

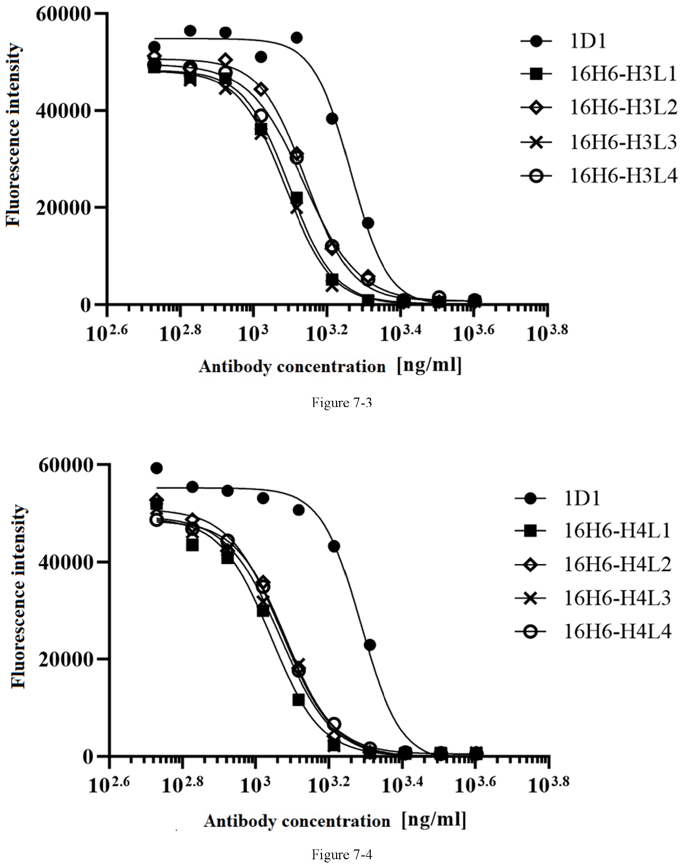

Figure 7-3 is a graph showing the results of the activity detection of humanized antibodies 16H6-H3L1, 16H6-H3L2, 16H6-H3L3 and 16H6-H3L4 against human TL1A trimer by reporter gene assay in Example 8.
Figure 7-4 is a graph showing the results of the activity detection of humanized antibodies 16H6-H4L1, 16H6-H4L2, 16H6-H4L3 and 16H6-H4L4 against human TL1A trimer by reporter gene assay in Example 8.

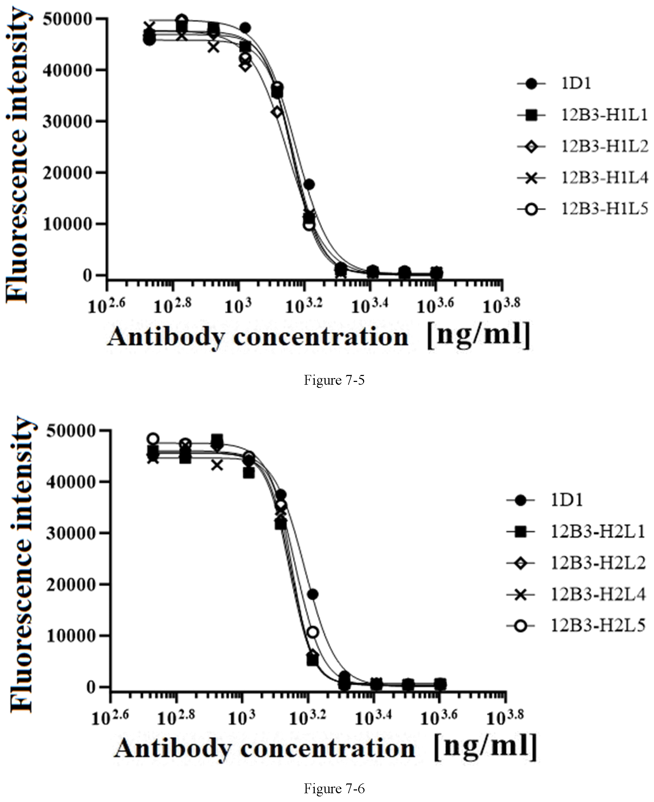

Figure 7-5 is a graph showing the results of the activity detection of humanized antibodies 12B3-H1L1, 12B3-H1L2, 12B3-H1L4 and 12B3-H1L5 against human TL1A trimer by reporter gene assay in Example 8.
Figure 7-6 is a graph showing the results of the activity detection of humanized antibodies 12B3-H2L1, 12B3-H2L2, 12B3-H2L4 and 12B3-H2L5 against human TL1A trimer by reporter gene assay in Example 8.
Figure 7-7 is a graph showing the results of the activity detection of humanized antibodies 12B3-H3L1, 12B3-H3L2, 12B3-H3L4 and 12B3-H3L5 against human TL1A trimer by reporter gene assay in Example 8.
Figure 7-8 is a graph showing the results of the activity detection of humanized antibodies 12B3-H4L1, 12B3-H4L2, 12B3-H4L4 and 12B3-H4L5 against human TL1A trimer by reporter gene assay in Example 8.
Figure 7-9 is a graph showing the results of the activity detection of humanized antibodies 12B3-H1L6, 12B3-H6L1 and 12B3-H6L6 against human TL1A trimer by reporter gene assay in Example 8.
Figure 7-10 is a graph showing the results of the activity detection of humanized antibodies 12B3-H1L8, 12B3-H6L4 and 12B3-H6L8 against human TL1A trimer by reporter gene assay in Example 8.
Figure 7-11 is a graph showing the results of the activity detection of humanized antibodies 12B3-H3L6, 12B3-H7L1 and 12B3-H7L6 against human TL1A trimer by reporter gene assay in Example 8.
Figure 7-12 is a graph showing the results of the activity detection of humanized antibodies 12B3-H3L8, 12B3-H7L4 and 12B3-H7L8 against human TL1A trimer by reporter gene assay in Example 8.
Figure 7-13 is a graph showing the results of the activity detection of humanized antibodies 12B3-H4L6, 12B3-H8L1 and 12B3-H8L6 against human TL1A trimer by reporter gene assay in Example 8.
Figure 7-14 is a graph showing the results of the activity detection of humanized antibodies 12B3-H4L8 and 12B3-H8L8 against human TL1A trimer by reporter gene assay in Example 8.

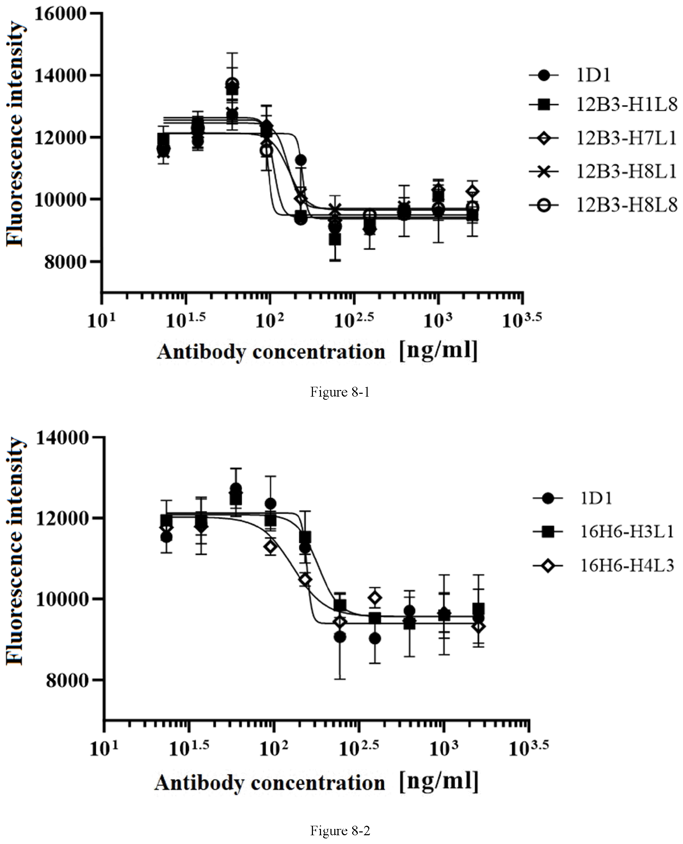

Figure 8-1 is a graph showing the results of the activity detection of humanized antibodies 12B3-H1L8, 12B3-H7L1, 12B3-H8L1 and 12B3-H8L8 against human TL1A trimer by caspase assay in Example 9.
Figure 8-2 is a graph showing the results of the activity detection of humanized antibodies 16H6-H3L1 and 16H6-H4L3 against human TL1A trimer by caspase assay in Example 9.

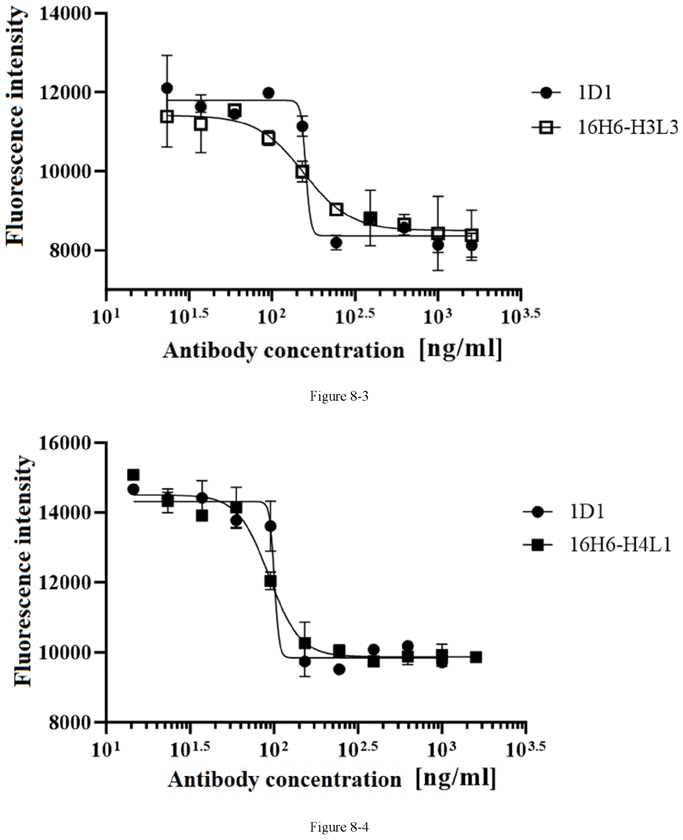

Figure 8-3 is a graph showing the results of the activity detection of humanized antibody 16H6-H3L3 against human TL1A trimer by caspase assay in Example 9.
Figure 8-4 is a graph showing the results of the activity detection of humanized antibody 16H6-H4L1 against human TL1A trimer by caspase assay in Example 9.

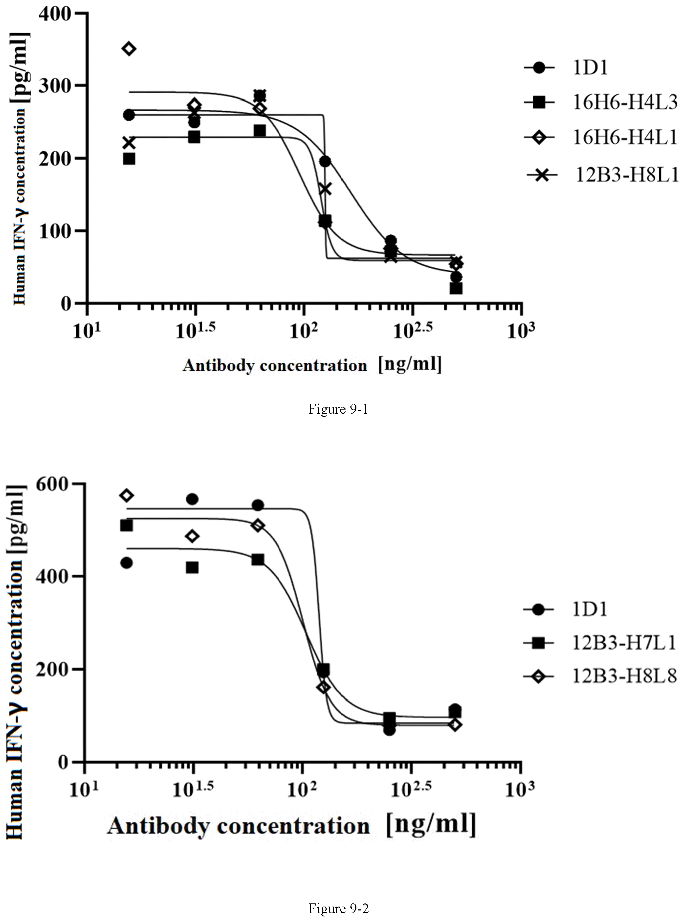

Figure 9-1 is a graph showing the results of the activity detection of humanized antibodies 16H6-H4L1, 16H6-H4L3 and 12B3-H8L1 against human TL1A trimer by cytokine assay in Example 10.
Figure 9-2 is a graph showing the results of the activity detection of humanized antibodies 12B3-H8L8 and 12B3-H7L1 against human TL1A trimer by cytokine assay in Example 10.

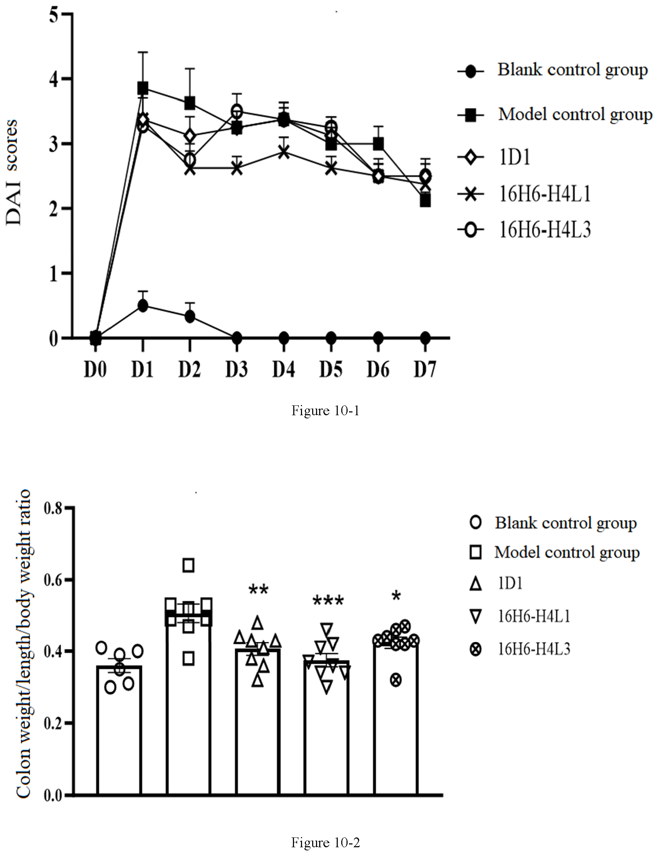

Figure 10-1 is a graph of the DAI scores of humanized antibodies 16H6-H4L1 and 16H6-H4L3 in a rat model in Example 11.
Figure 10-2 is a graph showing the results of the colon weight/length/body weight ratios of humanized antibodies 16H6-H4L1 and 16H6-H4L3 in a rat model in Example 11.

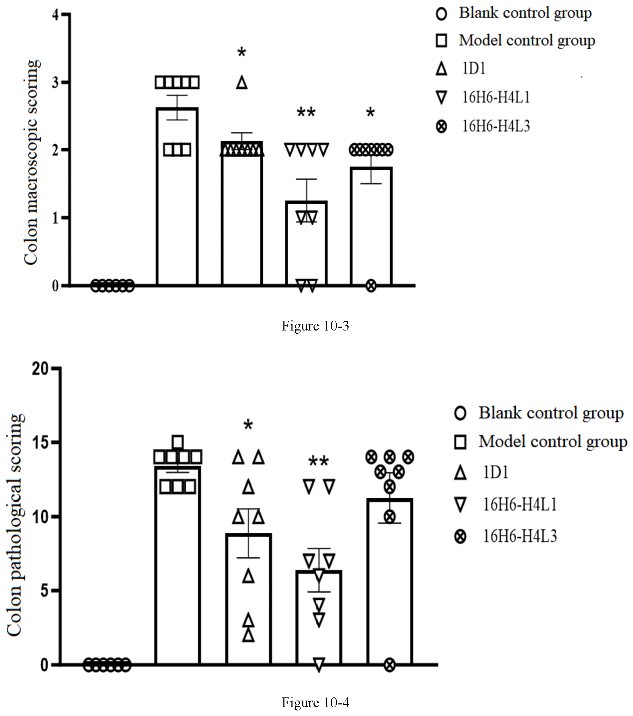

Figure 10-3 is a graph showing the results of the colon macroscopic scoring of humanized antibodies 16H6-H4L1 and 16H6-H4L3 in a rat model in Example 11.
Figure 10-4 is a graph showing the results of the colon pathological scoring of humanized antibodies 16H6-H4L1 and 16H6-H4L3 in a rat model in Example 11.

### DETAILED DESCRIPTION OF THE INVENTION

In order to make the present application easier to understand, the present application will be described in detail below with reference to the examples. These examples are for illustrative purposes only and are not intended to limit the scope of the present application.

Unless otherwise defined, all technical and scientific terms used herein have the same meaning as commonly understood by those skilled in the art to which the present disclosure belongs. See, e.g., Singleton et al., Dictionary of Microbiology and Molecular Biology 2nd ed., J. Wiley & Sons (New York, NY 1994); Sambrook et al., Molecular Cloning, A Laboratory Manual, Cold Springs Harbor Press (Cold Springs Harbor, NY 1989); Davis et al., Basic Methods in Molecular Biology, Elsevier Science Publishing Inc., New York, USA (2012); Abbas et al., Cellular and Molecular Immunology, Elsevier Science Health Science div (2009); and He Wei et al., Medical Immunology (2nd Edition), People's Medical Publishing House, 2010.

Unless otherwise stated, the terms "comprise", "comprises" and "comprising" or their equivalents (such as "contain", "contains", "containing", "include", "includes", and "including") as used herein are open-ended expressions, meaning that in addition to the listed elements, components and steps, other unstated elements, components and steps may also be encompassed.

Unless otherwise stated, the term "optional" or "optionally" as used herein means that the object or event it modifies may exist or may not exist, or may occur or may not occur. As used herein, the word "or" is intended to include "and" unless the context clearly indicates otherwise.

As used herein, "about" means within an acceptable error range for the particular value as determined by one of ordinary skill in the art, which depends in part on how the value is measured or determined, i.e., the limitations of the measurement system. For example, "about" can mean within one or more standard deviations, as practiced in the art. Alternatively, "about" can mean a range of up to 20% (i.e., ±20%), for example, fluctuating within a range of ±10%, ±5%, ±2%, ±1% or ±0.5% of a given specific value. Furthermore, particularly for biological systems or methods, the term can mean up to an order of magnitude or up to 5 times that value. When a specific value is given in the present application or claims, unless otherwise stated, the meaning of "about" should be considered to be within an acceptable error range for that specific value. Herein, unless otherwise stated, each measured value, step parameter or condition is defaulted to be modified by "about".

Herein, unless the context clearly dictates otherwise, singular terms encompass plural referents and vice versa.

Unless otherwise stated, the term "treatment" as used herein means that after administration, the disease or its related symptoms can be inhibited, suppressed, alleviated, ameliorated, slowed, relieved or eliminated, the progression of the disease or its related symptoms can be retarded, delayed, slowed, stopped or terminated, or the recurrence of the disease or its related symptoms can be prevented, controlled or reduced. For the prevention and treatment of diseases, the therapeutically effective amount or prophylactically effective amount can be determined by a clinician through conventional methods or experience based on the individual condition of the subject, severity of the disease, gender, age, body weight, route of administration, etc.

Unless otherwise stated, the term "subject" as used herein encompasses any vertebrate, e.g., mammals such as humans, non-human primates, sheep, dogs, cats, horses, cows, chickens, pigs, mice, etc. Preferably, the subject in the present application is a human.

The term "antibody" refers to a polypeptide encoded by immunoglobulin genes and capable of binding to an antigen. The term "complementarity determining region" may refer to the portion of a variable region of an antibody that confers antigen-binding specificity, and may refer to the amino acid sequence found in the hypervariable region of a heavy or light chain of an immunoglobulin. A heavy chain may include three complementarity determining regions (CDRs): HCDR1, HCDR2 and HCDR3; and a light chain may include three complementarity determining regions (CDRs): LCDR1, LCDR2 and LCDR3. These CDRs provide contact residues that play an important role in the binding of an antibody to its antigen or antigenic epitope.

The term "humanized antibody" refers to a genetically engineered non-human antibody whose amino acid sequence has been modified to increase homology to the sequence of a human antibody. Generally, all or part of the CDR regions of a humanized antibody are derived from a non-human antibody (donor antibody), and all or part of the non-CDR regions (e.g., variable region FR and/or constant region) are derived from a human immunoglobulin (acceptor antibody). A humanized antibody typically retains or partially retains the expected properties of the donor antibody, including but not limited to, antigen specificity, affinity, reactivity, ability to enhance immune cell activity, ability to enhance immune response, etc.

The term "chimeric antibody" refers to an antibody in which the variable region sequences are derived from one species and the constant region sequences are derived from another species, for example, an antibody obtained by fusing the variable region of a murine antibody with the constant region of a human antibody.

Herein, the percent identity (degree of homology) between sequences can be determined by comparing the two sequences using, for example, a freely available computer program (e.g., BLASTp or BLASTn with default settings) commonly used for this purpose on the World Wide Web (e.g., www.ncbi.nlm.nih.gov).

In some embodiments, the present application provides an anti-TL1A antibody or an antigen-binding fragment thereof, comprising:
(a) a heavy chain variable region comprising the following complementarity determining regions:
   HCDR1, comprising the amino acid sequence set forth in SEQ ID NO: 1, 7, 13, 19, 25, 28, 34, 40 or 46, or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 1, 7, 13, 19, 25, 28, 34, 40 or 46, or an amino acid sequence obtained by substitution, deletion or addition of one or more amino acids in the amino acid sequence set forth in SEQ ID NO: 1, 7, 13, 19, 25, 28, 34, 40 or 46 and having the same or similar function to the amino acid sequence set forth in SEQ ID NO: 1, 7, 13, 19, 25, 28, 34, 40 or 46;
   HCDR2, comprising the amino acid sequence set forth in SEQ ID NO: 2, 8, 14, 20, 29, 35, 41 or 47, or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 2, 8, 14, 20, 29, 35, 41 or 47, or an amino acid sequence obtained by substitution, deletion or addition of one or more amino acids in the amino acid sequence set forth in SEQ ID NO: 2, 8, 14, 20, 29, 35, 41 or 47 and having the same or similar function to the amino acid sequence set forth in SEQ ID NO: 2, 8, 14, 20, 29, 35, 41 or 47; and
   HCDR3, comprising the amino acid sequence set forth in SEQ ID NO: 3, 9, 15, 21, 26, 30, 36, 42 or 48, or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 3, 9, 15, 21, 26, 30, 36, 42 or 48, or an amino acid sequence obtained by substitution, deletion or addition of one or more amino acids in the amino acid sequence set forth in SEQ ID NO: 3, 9, 15, 21, 26, 30, 36, 42 or 48 and having the same or similar function to the amino acid sequence set forth in SEQ ID NO: 3, 9, 15, 21, 26, 30, 36, 42 or 48; and
(b) a light chain variable region comprising the following complementarity determining regions:
   LCDR1, comprising the amino acid sequence set forth in SEQ ID NO: 4, 10, 16, 22, 31, 37, 43 or 49, or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 4, 10, 16, 22, 31, 37, 43 or 49, or an amino acid sequence obtained by substitution, deletion or addition of one or more amino acids in the amino acid sequence set forth in SEQ ID NO: 4, 10, 16, 22, 31, 37, 43 or 49 and having the same or similar function to the amino acid sequence set forth in SEQ ID NO: 4, 10, 16, 22, 31, 37, 43 or 49;
   LCDR2, comprising the amino acid sequence set forth in SEQ ID NO: 5, 11, 17, 23, 32, 38, 44 or 50, or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 5, 11, 17, 23, 32, 38, 44 or 50, or an amino acid sequence obtained by substitution, deletion or addition of one or more amino acids in the amino acid sequence set forth in SEQ ID NO: 5, 11, 17, 23, 32, 38, 44 or 50 and having the same or similar function to the amino acid sequence set forth in SEQ ID NO: 5, 11, 17, 23, 32, 38, 44 or 50; and
   LCDR3, comprising the amino acid sequence set forth in SEQ ID NO: 6, 12, 18, 24, 27, 33, 39, 45 or 51, or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 6, 12, 18, 24, 27, 33, 39, 45 or 51, or an amino acid sequence obtained by substitution, deletion or addition of one or more amino acids in the amino acid sequence set forth in SEQ ID NO: 6, 12, 18, 24, 27, 33, 39, 45 or 51 and having the same or similar function to the amino acid sequence set forth in SEQ ID NO: 6, 12, 18, 24, 27, 33, 39, 45 or 51.

The CDRs described above have been defined according to the ImMunoGenTics (IMGT) numbering system, but those skilled in the art will appreciate that CDR sequences numbered according to any one or more of the AbM numbering system, the Kabat numbering system, the Chothia numbering system, and the Contact numbering system (based on analysis of available complex crystal structures) also fall within the protection scope of the present application.

As used herein, the substitution, deletion or addition of one or more amino acids means substitution, deletion or addition of 1 or 2 amino acids. In some embodiments, the substitution, deletion or addition of one or more amino acids is a conservative modification. In some embodiments, compared to the amino acid sequences set forth in SEQ ID NOs: 1-3, 7-9, 13-15, 19-21, 25-26, 28-30, 34-36, 40-42 and 46-48, the differing amino acids in the amino acid sequence having at least 80% identity thereto or the amino acid sequence having substitution, deletion or addition of one or more amino acids are located mainly or entirely in the FR regions of the heavy chain variable region. In some embodiments, compared to the amino acid sequences set forth in SEQ ID NOs: 4-6, 10-12, 16-18, 22-24, 27, 31-33, 37-39, 43-45 and 49-51, the differing amino acids in the amino acid sequence having at least 80% identity thereto or the amino acid sequence having substitution, deletion or addition of one or more amino acids are located mainly or entirely in the FR regions of the light chain variable region.

In some embodiments, the anti-TL1A antibody or the antigen-binding fragment thereof comprises:
(1) HCDR1, HCDR2, HCDR3, LCDR1, LCDR2 and LCDR3, wherein the HCDR1, HCDR2 and HCDR3 have the amino acid sequences set forth in SEQ ID NOs: 1, 2 and 3, respectively, and the LCDR1, LCDR2 and LCDR3 have the amino acid sequences set forth in SEQ ID NOs: 4, 5 and 6, respectively;
(2) HCDR1, HCDR2, HCDR3, LCDR1, LCDR2 and LCDR3, wherein the HCDR1, HCDR2 and HCDR3 have the amino acid sequences set forth in SEQ ID NOs: 7, 8 and 9, respectively, and the LCDR1, LCDR2 and LCDR3 have the amino acid sequences set forth in SEQ ID NOs: 10, 11 and 12, respectively;
(3) HCDR1, HCDR2, HCDR3, LCDR1, LCDR2 and LCDR3, wherein the HCDR1, HCDR2 and HCDR3 have the amino acid sequences set forth in SEQ ID NOs: 13, 14 and 15, respectively, and the LCDR1, LCDR2 and LCDR3 have the amino acid sequences set forth in SEQ ID NOs: 16, 17 and 18, respectively;
(4) HCDR1, HCDR2, HCDR3, LCDR1, LCDR2 and LCDR3, wherein the HCDR1, HCDR2 and HCDR3 have the amino acid sequences set forth in SEQ ID NOs: 19, 20 and 21, respectively, and the LCDR1, LCDR2 and LCDR3 have the amino acid sequences set forth in SEQ ID NOs: 22, 23 and 24, respectively;
(5) HCDR1, HCDR2, HCDR3, LCDR1, LCDR2 and LCDR3, wherein the HCDR1, HCDR2 and HCDR3 have the amino acid sequences set forth in SEQ ID NOs: 25, 20 and 26, respectively, and the LCDR1, LCDR2 and LCDR3 have the amino acid sequences set forth in SEQ ID NOs: 22, 23 and 27, respectively;
(6) HCDR1, HCDR2, HCDR3, LCDR1, LCDR2 and LCDR3, wherein the HCDR1, HCDR2 and HCDR3 have the amino acid sequences set forth in SEQ ID NOs: 28, 29 and 30, respectively, and the LCDR1, LCDR2 and LCDR3 have the amino acid sequences set forth in SEQ ID NOs: 31, 32 and 33, respectively;
(7) HCDR1, HCDR2, HCDR3, LCDR1, LCDR2 and LCDR3, wherein the HCDR1, HCDR2 and HCDR3 have the amino acid sequences set forth in SEQ ID NOs: 34, 35 and 36, respectively, and the LCDR1, LCDR2 and LCDR3 have the amino acid sequences set forth in SEQ ID NOs: 37, 38 and 39, respectively;
(8) HCDR1, HCDR2, HCDR3, LCDR1, LCDR2 and LCDR3, wherein the HCDR1, HCDR2 and HCDR3 have the amino acid sequences set forth in SEQ ID NOs: 40, 41 and 42, respectively, and the LCDR1, LCDR2 and LCDR3 have the amino acid sequences set forth in SEQ ID NOs: 43, 44 and 45, respectively; or
(9) HCDR1, HCDR2, HCDR3, LCDR1, LCDR2 and LCDR3, wherein the HCDR1, HCDR2 and HCDR3 have the amino acid sequences set forth in SEQ ID NOs: 46, 47 and 48, respectively, and the LCDR1, LCDR2 and LCDR3 have the amino acid sequences set forth in SEQ ID NOs: 49, 50 and 51, respectively.

In some embodiments, the anti-TL1A antibody or the antigen-binding fragment thereof comprises:
(1) HCDR1, HCDR2, HCDR3, LCDR1, LCDR2 and LCDR3, wherein the HCDR1, HCDR2 and HCDR3 have the amino acid sequences set forth in SEQ ID NOs: 1, 2 and 3, respectively, and the LCDR1, LCDR2 and LCDR3 have the amino acid sequences set forth in SEQ ID NOs: 4, 5 and 6, respectively;
(2) HCDR1, HCDR2, HCDR3, LCDR1, LCDR2 and LCDR3, wherein the HCDR1, HCDR2 and HCDR3 have the amino acid sequences set forth in SEQ ID NOs: 7, 8 and 9, respectively, and the LCDR1, LCDR2 and LCDR3 have the amino acid sequences set forth in SEQ ID NOs: 10, 11 and 12, respectively;
(3) HCDR1, HCDR2, HCDR3, LCDR1, LCDR2 and LCDR3, wherein the HCDR1, HCDR2 and HCDR3 have the amino acid sequences set forth in SEQ ID NOs: 13, 14 and 15, respectively, and the LCDR1, LCDR2 and LCDR3 have the amino acid sequences set forth in SEQ ID NOs: 16, 17 and 18, respectively;
(4) HCDR1, HCDR2, HCDR3, LCDR1, LCDR2 and LCDR3, wherein the HCDR1, HCDR2 and HCDR3 have the amino acid sequences set forth in SEQ ID NOs: 40, 41 and 42, respectively, and the LCDR1, LCDR2 and LCDR3 have the amino acid sequences set forth in SEQ ID NOs: 43, 44 and 45, respectively; or
(5) HCDR1, HCDR2, HCDR3, LCDR1, LCDR2 and LCDR3, wherein the HCDR1, HCDR2 and HCDR3 have the amino acid sequences set forth in SEQ ID NOs: 46, 47 and 48, respectively, and the LCDR1, LCDR2 and LCDR3 have the amino acid sequences set forth in SEQ ID NOs: 49, 50 and 51, respectively.

In preferred embodiments, the anti-TL1A antibody or the antigen-binding fragment thereof comprises:
(1) HCDR1, HCDR2, HCDR3, LCDR1, LCDR2 and LCDR3, wherein the HCDR1, HCDR2 and HCDR3 have the amino acid sequences set forth in SEQ ID NOs: 1, 2 and 3, respectively, and the LCDR1, LCDR2 and LCDR3 have the amino acid sequences set forth in SEQ ID NOs: 4, 5 and 6, respectively;
(2) HCDR1, HCDR2, HCDR3, LCDR1, LCDR2 and LCDR3, wherein the HCDR1, HCDR2 and HCDR3 have the amino acid sequences set forth in SEQ ID NOs: 7, 8 and 9, respectively, and the LCDR1, LCDR2 and LCDR3 have the amino acid sequences set forth in SEQ ID NOs: 10, 11 and 12, respectively; or
(3) HCDR1, HCDR2, HCDR3, LCDR1, LCDR2 and LCDR3, wherein the HCDR1, HCDR2 and HCDR3 have the amino acid sequences set forth in SEQ ID NOs: 13, 14 and 15, respectively, and the LCDR1, LCDR2 and LCDR3 have the amino acid sequences set forth in SEQ ID NOs: 16, 17 and 18, respectively.

In some embodiments, the anti-TL1A antibody or the antigen-binding fragment thereof comprises:
(1) HCDR1, HCDR2, HCDR3, LCDR1, LCDR2 and LCDR3, wherein the HCDR1, HCDR2 and HCDR3 have the amino acid sequences set forth in SEQ ID NOs: 1, 2 and 3, respectively, and the LCDR1, LCDR2 and LCDR3 have the amino acid sequences set forth in SEQ ID NOs: 4, 5 and 6, respectively; or
(2) HCDR1, HCDR2, HCDR3, LCDR1, LCDR2 and LCDR3, wherein the HCDR1, HCDR2 and HCDR3 have the amino acid sequences set forth in SEQ ID NOs: 7, 8 and 9, respectively, and the LCDR1, LCDR2 and LCDR3 have the amino acid sequences set forth in SEQ ID NOs: 10, 11 and 12, respectively.

In some embodiments, the anti-TL1A antibody or the antigen-binding fragment thereof comprises: a heavy chain variable region comprising an amino acid sequence set forth in any one of SEQ ID NOs: 61, 54, 59, 60, 62, 69, 70, 71, 72, 73 and 74 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto; and
a light chain variable region comprising an amino acid sequence set forth in any one of SEQ ID NOs: 55, 56, 57, 58, 63, 64, 65, 66, 67 and 68 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto.

In some embodiments, the anti-TL1A antibody or the antigen-binding fragment thereof comprises:
(1) a heavy chain variable region and a light chain variable region, wherein the heavy chain variable region comprises the amino acid sequence set forth in SEQ ID NO: 61 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 61, and the light chain variable region comprises the amino acid sequence set forth in SEQ ID NO: 55 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 55;
(2) a heavy chain variable region and a light chain variable region, wherein the heavy chain variable region comprises the amino acid sequence set forth in SEQ ID NO: 54 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 54, and the light chain variable region comprises the amino acid sequence set forth in SEQ ID NO: 55 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 55;
(3) a heavy chain variable region and a light chain variable region, wherein the heavy chain variable region comprises the amino acid sequence set forth in SEQ ID NO: 54 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 54, and the light chain variable region comprises the amino acid sequence set forth in SEQ ID NO: 56 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 56;
(4) a heavy chain variable region and a light chain variable region, wherein the heavy chain variable region comprises the amino acid sequence set forth in SEQ ID NO: 54 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 54, and the light chain variable region comprises the amino acid sequence set forth in SEQ ID NO: 57 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 57;
(5) a heavy chain variable region and a light chain variable region, wherein the heavy chain variable region comprises the amino acid sequence set forth in SEQ ID NO: 54 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 54, and the light chain variable region comprises the amino acid sequence set forth in SEQ ID NO: 58 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 58;
(6) a heavy chain variable region and a light chain variable region, wherein the heavy chain variable region comprises the amino acid sequence set forth in SEQ ID NO: 59 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 59, and the light chain variable region comprises the amino acid sequence set forth in SEQ ID NO: 55 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 55;
(7) a heavy chain variable region and a light chain variable region, wherein the heavy chain variable region comprises the amino acid sequence set forth in SEQ ID NO: 59 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 59, and the light chain variable region comprises the amino acid sequence set forth in SEQ ID NO: 56 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 56;
(8) a heavy chain variable region and a light chain variable region, wherein the heavy chain variable region comprises the amino acid sequence set forth in SEQ ID NO: 59 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 59, and the light chain variable region comprises the amino acid sequence set forth in SEQ ID NO: 57 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 57;
(9) a heavy chain variable region and a light chain variable region, wherein the heavy chain variable region comprises the amino acid sequence set forth in SEQ ID NO: 59 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 59, and the light chain variable region comprises the amino acid sequence set forth in SEQ ID NO: 58 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 58;
(10) a heavy chain variable region and a light chain variable region, wherein the heavy chain variable region comprises the amino acid sequence set forth in SEQ ID NO: 60 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 60, and the light chain variable region comprises the amino acid sequence set forth in SEQ ID NO: 55 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 55;
(11) a heavy chain variable region and a light chain variable region, wherein the heavy chain variable region comprises the amino acid sequence set forth in SEQ ID NO: 60 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 60, and the light chain variable region comprises the amino acid sequence set forth in SEQ ID NO: 56 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 56;
(12) a heavy chain variable region and a light chain variable region, wherein the heavy chain variable region comprises the amino acid sequence set forth in SEQ ID NO: 60 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 60, and the light chain variable region comprises the amino acid sequence set forth in SEQ ID NO: 57 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 57;
(13) a heavy chain variable region and a light chain variable region, wherein the heavy chain variable region comprises the amino acid sequence set forth in SEQ ID NO: 60 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 60, and the light chain variable region comprises the amino acid sequence set forth in SEQ ID NO: 58 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 58;
(14) a heavy chain variable region and a light chain variable region, wherein the heavy chain variable region comprises the amino acid sequence set forth in SEQ ID NO: 61 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 61, and the light chain variable region comprises the amino acid sequence set forth in SEQ ID NO: 56 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 56;
(15) a heavy chain variable region and a light chain variable region, wherein the heavy chain variable region comprises the amino acid sequence set forth in SEQ ID NO: 61 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 61, and the light chain variable region comprises the amino acid sequence set forth in SEQ ID NO: 57 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 57;
(16) a heavy chain variable region and a light chain variable region, wherein the heavy chain variable region comprises the amino acid sequence set forth in SEQ ID NO: 61 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 61, and the light chain variable region comprises the amino acid sequence set forth in SEQ ID NO: 58 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 58;
(17) a heavy chain variable region and a light chain variable region, wherein the heavy chain variable region comprises the amino acid sequence set forth in SEQ ID NO: 62 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 62, and the light chain variable region comprises the amino acid sequence set forth in SEQ ID NO: 63 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 63;
(18) a heavy chain variable region and a light chain variable region, wherein the heavy chain variable region comprises the amino acid sequence set forth in SEQ ID NO: 62 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 62, and the light chain variable region comprises the amino acid sequence set forth in SEQ ID NO: 64 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 64;
(19) a heavy chain variable region and a light chain variable region, wherein the heavy chain variable region comprises the amino acid sequence set forth in SEQ ID NO: 62 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 62, and the light chain variable region comprises the amino acid sequence set forth in SEQ ID NO: 65 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 65;
(20) a heavy chain variable region and a light chain variable region, wherein the heavy chain variable region comprises the amino acid sequence set forth in SEQ ID NO: 62 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 62, and the light chain variable region comprises the amino acid sequence set forth in SEQ ID NO: 66 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 66;
(21) a heavy chain variable region and a light chain variable region, wherein the heavy chain variable region comprises the amino acid sequence set forth in SEQ ID NO: 62 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 62, and the light chain variable region comprises the amino acid sequence set forth in SEQ ID NO: 67 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 67;
(22) a heavy chain variable region and a light chain variable region, wherein the heavy chain variable region comprises the amino acid sequence set forth in SEQ ID NO: 62 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 62, and the light chain variable region comprises the amino acid sequence set forth in SEQ ID NO: 68 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 68;
(23) a heavy chain variable region and a light chain variable region, wherein the heavy chain variable region comprises the amino acid sequence set forth in SEQ ID NO: 69 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 69, and the light chain variable region comprises the amino acid sequence set forth in SEQ ID NO: 63 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 63;
(24) a heavy chain variable region and a light chain variable region, wherein the heavy chain variable region comprises the amino acid sequence set forth in SEQ ID NO: 69 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 69, and the light chain variable region comprises the amino acid sequence set forth in SEQ ID NO: 64 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 64;
(25) a heavy chain variable region and a light chain variable region, wherein the heavy chain variable region comprises the amino acid sequence set forth in SEQ ID NO: 69 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 69, and the light chain variable region comprises the amino acid sequence set forth in SEQ ID NO: 65 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 65;
(26) a heavy chain variable region and a light chain variable region, wherein the heavy chain variable region comprises the amino acid sequence set forth in SEQ ID NO: 69 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 69, and the light chain variable region comprises the amino acid sequence set forth in SEQ ID NO: 66 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 66;
(27) a heavy chain variable region and a light chain variable region, wherein the heavy chain variable region comprises the amino acid sequence set forth in SEQ ID NO: 70 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 70, and the light chain variable region comprises the amino acid sequence set forth in SEQ ID NO: 63 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 63;
(28) a heavy chain variable region and a light chain variable region, wherein the heavy chain variable region comprises the amino acid sequence set forth in SEQ ID NO: 70 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 70, and the light chain variable region comprises the amino acid sequence set forth in SEQ ID NO: 64 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 64;
(29) a heavy chain variable region and a light chain variable region, wherein the heavy chain variable region comprises the amino acid sequence set forth in SEQ ID NO: 70 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 70, and the light chain variable region comprises the amino acid sequence set forth in SEQ ID NO: 65 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 65;
(30) a heavy chain variable region and a light chain variable region, wherein the heavy chain variable region comprises the amino acid sequence set forth in SEQ ID NO: 70 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 70, and the light chain variable region comprises the amino acid sequence set forth in SEQ ID NO: 66 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 66;
(31) a heavy chain variable region and a light chain variable region, wherein the heavy chain variable region comprises the amino acid sequence set forth in SEQ ID NO: 70 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 70, and the light chain variable region comprises the amino acid sequence set forth in SEQ ID NO: 67 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 67;
(32) a heavy chain variable region and a light chain variable region, wherein the heavy chain variable region comprises the amino acid sequence set forth in SEQ ID NO: 70 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 70, and the light chain variable region comprises the amino acid sequence set forth in SEQ ID NO: 68 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 68;
(33) a heavy chain variable region and a light chain variable region, wherein the heavy chain variable region comprises the amino acid sequence set forth in SEQ ID NO: 71 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 71, and the light chain variable region comprises the amino acid sequence set forth in SEQ ID NO: 63 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 63;
(34) a heavy chain variable region and a light chain variable region, wherein the heavy chain variable region comprises the amino acid sequence set forth in SEQ ID NO: 71 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 71, and the light chain variable region comprises the amino acid sequence set forth in SEQ ID NO: 64 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 64;
(35) a heavy chain variable region and a light chain variable region, wherein the heavy chain variable region comprises the amino acid sequence set forth in SEQ ID NO: 71 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 71, and the light chain variable region comprises the amino acid sequence set forth in SEQ ID NO: 65 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 65;
(36) a heavy chain variable region and a light chain variable region, wherein the heavy chain variable region comprises the amino acid sequence set forth in SEQ ID NO: 71 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 71, and the light chain variable region comprises the amino acid sequence set forth in SEQ ID NO: 66 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 66;
(37) a heavy chain variable region and a light chain variable region, wherein the heavy chain variable region comprises the amino acid sequence set forth in SEQ ID NO: 71 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 71, and the light chain variable region comprises the amino acid sequence set forth in SEQ ID NO: 67 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 67;
(38) a heavy chain variable region and a light chain variable region, wherein the heavy chain variable region comprises the amino acid sequence set forth in SEQ ID NO: 71 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 71, and the light chain variable region comprises the amino acid sequence set forth in SEQ ID NO: 68 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 68;
(39) a heavy chain variable region and a light chain variable region, wherein the heavy chain variable region comprises the amino acid sequence set forth in SEQ ID NO: 72 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 72, and the light chain variable region comprises the amino acid sequence set forth in SEQ ID NO: 63 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 63;
(40) a heavy chain variable region and a light chain variable region, wherein the heavy chain variable region comprises the amino acid sequence set forth in SEQ ID NO: 72 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 72, and the light chain variable region comprises the amino acid sequence set forth in SEQ ID NO: 65 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 65;
(41) a heavy chain variable region and a light chain variable region, wherein the heavy chain variable region comprises the amino acid sequence set forth in SEQ ID NO: 72 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 72, and the light chain variable region comprises the amino acid sequence set forth in SEQ ID NO: 67 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 67;
(42) a heavy chain variable region and a light chain variable region, wherein the heavy chain variable region comprises the amino acid sequence set forth in SEQ ID NO: 72 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 72, and the light chain variable region comprises the amino acid sequence set forth in SEQ ID NO: 68 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 68;
(43) a heavy chain variable region and a light chain variable region, wherein the heavy chain variable region comprises the amino acid sequence set forth in SEQ ID NO: 73 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 73, and the light chain variable region comprises the amino acid sequence set forth in SEQ ID NO: 63 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 63;
(44) a heavy chain variable region and a light chain variable region, wherein the heavy chain variable region comprises the amino acid sequence set forth in SEQ ID NO: 73 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 73, and the light chain variable region comprises the amino acid sequence set forth in SEQ ID NO: 65 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 65;
(45) a heavy chain variable region and a light chain variable region, wherein the heavy chain variable region comprises the amino acid sequence set forth in SEQ ID NO: 73 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 73, and the light chain variable region comprises the amino acid sequence set forth in SEQ ID NO: 67 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 67;
(46) a heavy chain variable region and a light chain variable region, wherein the heavy chain variable region comprises the amino acid sequence set forth in SEQ ID NO: 73 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 73, and the light chain variable region comprises the amino acid sequence set forth in SEQ ID NO: 68 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 68;
(47) a heavy chain variable region and a light chain variable region, wherein the heavy chain variable region comprises the amino acid sequence set forth in SEQ ID NO: 74 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 74, and the light chain variable region comprises the amino acid sequence set forth in SEQ ID NO: 63 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 63;
(48) a heavy chain variable region and a light chain variable region, wherein the heavy chain variable region comprises the amino acid sequence set forth in SEQ ID NO: 74 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 74, and the light chain variable region comprises the amino acid sequence set forth in SEQ ID NO: 67 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 67; or
(49) a heavy chain variable region and a light chain variable region, wherein the heavy chain variable region comprises the amino acid sequence set forth in SEQ ID NO: 74 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 74, and the light chain variable region comprises the amino acid sequence set forth in SEQ ID NO: 68 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 68.

In some embodiments, compared to the amino acid sequence set forth in any one of SEQ ID NOs: 61, 54, 59, 60, 62, 69, 70, 71, 72, 73 and 74 or any one of SEQ ID NOs: 55, 56, 57, 58, 63, 64, 65, 66, 67 and 68, the differing amino acids in the amino acid sequence having at least 80% identity are located mainly or entirely in an FR region.

In some embodiments, the anti-TL1A antibody or the antigen-binding fragment thereof comprises:
(1) a heavy chain variable region and a light chain variable region, wherein the heavy chain variable region comprises the amino acid sequence set forth in SEQ ID NO: 61 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 61, and the light chain variable region comprises the amino acid sequence set forth in SEQ ID NO: 55 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 55;
(2) a heavy chain variable region and a light chain variable region, wherein the heavy chain variable region comprises the amino acid sequence set forth in SEQ ID NO: 54 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 54, and the light chain variable region comprises the amino acid sequence set forth in SEQ ID NO: 55 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 55;
(3) a heavy chain variable region and a light chain variable region, wherein the heavy chain variable region comprises the amino acid sequence set forth in SEQ ID NO: 54 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 54, and the light chain variable region comprises the amino acid sequence set forth in SEQ ID NO: 56 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 56;
(4) a heavy chain variable region and a light chain variable region, wherein the heavy chain variable region comprises the amino acid sequence set forth in SEQ ID NO: 54 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 54, and the light chain variable region comprises the amino acid sequence set forth in SEQ ID NO: 57 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 57;
(5) a heavy chain variable region and a light chain variable region, wherein the heavy chain variable region comprises the amino acid sequence set forth in SEQ ID NO: 54 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 54, and the light chain variable region comprises the amino acid sequence set forth in SEQ ID NO: 58 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 58;
(6) a heavy chain variable region and a light chain variable region, wherein the heavy chain variable region comprises the amino acid sequence set forth in SEQ ID NO: 59 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 59, and the light chain variable region comprises the amino acid sequence set forth in SEQ ID NO: 55 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 55;
(7) a heavy chain variable region and a light chain variable region, wherein the heavy chain variable region comprises the amino acid sequence set forth in SEQ ID NO: 59 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 59, and the light chain variable region comprises the amino acid sequence set forth in SEQ ID NO: 56 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 56;
(8) a heavy chain variable region and a light chain variable region, wherein the heavy chain variable region comprises the amino acid sequence set forth in SEQ ID NO: 59 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 59, and the light chain variable region comprises the amino acid sequence set forth in SEQ ID NO: 57 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 57;
(9) a heavy chain variable region and a light chain variable region, wherein the heavy chain variable region comprises the amino acid sequence set forth in SEQ ID NO: 59 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 59, and the light chain variable region comprises the amino acid sequence set forth in SEQ ID NO: 58 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 58;
(10) a heavy chain variable region and a light chain variable region, wherein the heavy chain variable region comprises the amino acid sequence set forth in SEQ ID NO: 60 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 60, and the light chain variable region comprises the amino acid sequence set forth in SEQ ID NO: 55 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 55;
(11) a heavy chain variable region and a light chain variable region, wherein the heavy chain variable region comprises the amino acid sequence set forth in SEQ ID NO: 60 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 60, and the light chain variable region comprises the amino acid sequence set forth in SEQ ID NO: 57 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 57;
(12) a heavy chain variable region and a light chain variable region, wherein the heavy chain variable region comprises the amino acid sequence set forth in SEQ ID NO: 61 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 61, and the light chain variable region comprises the amino acid sequence set forth in SEQ ID NO: 56 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 56;
(13) a heavy chain variable region and a light chain variable region, wherein the heavy chain variable region comprises the amino acid sequence set forth in SEQ ID NO: 61 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 61, and the light chain variable region comprises the amino acid sequence set forth in SEQ ID NO: 57 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 57;
(14) a heavy chain variable region and a light chain variable region, wherein the heavy chain variable region comprises the amino acid sequence set forth in SEQ ID NO: 61 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 61, and the light chain variable region comprises the amino acid sequence set forth in SEQ ID NO: 58 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 58;
(15) a heavy chain variable region and a light chain variable region, wherein the heavy chain variable region comprises the amino acid sequence set forth in SEQ ID NO: 62 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 62, and the light chain variable region comprises the amino acid sequence set forth in SEQ ID NO: 68 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 68;
(16) a heavy chain variable region and a light chain variable region, wherein the heavy chain variable region comprises the amino acid sequence set forth in SEQ ID NO: 73 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 73, and the light chain variable region comprises the amino acid sequence set forth in SEQ ID NO: 63 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 63;
(17) a heavy chain variable region and a light chain variable region, wherein the heavy chain variable region comprises the amino acid sequence set forth in SEQ ID NO: 74 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 74, and the light chain variable region comprises the amino acid sequence set forth in SEQ ID NO: 63 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 63; or
(18) a heavy chain variable region and a light chain variable region, wherein the heavy chain variable region comprises the amino acid sequence set forth in SEQ ID NO: 74 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 74, and the light chain variable region comprises the amino acid sequence set forth in SEQ ID NO: 68 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 68.

In some embodiments, the anti-TL1A antibody or the antigen-binding fragment thereof comprises:
(1) a heavy chain variable region and a light chain variable region, wherein the heavy chain variable region comprises the amino acid sequence set forth in SEQ ID NO: 61 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 61, and the light chain variable region comprises the amino acid sequence set forth in SEQ ID NO: 55 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 55;
(2) a heavy chain variable region and a light chain variable region, wherein the heavy chain variable region comprises the amino acid sequence set forth in SEQ ID NO: 61 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 61, and the light chain variable region comprises the amino acid sequence set forth in SEQ ID NO: 56 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 56;
(3) a heavy chain variable region and a light chain variable region, wherein the heavy chain variable region comprises the amino acid sequence set forth in SEQ ID NO: 61 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 61, and the light chain variable region comprises the amino acid sequence set forth in SEQ ID NO: 57 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 57;
(4) a heavy chain variable region and a light chain variable region, wherein the heavy chain variable region comprises the amino acid sequence set forth in SEQ ID NO: 61 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 61, and the light chain variable region comprises the amino acid sequence set forth in SEQ ID NO: 58 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 58;
(5) a heavy chain variable region and a light chain variable region, wherein the heavy chain variable region comprises the amino acid sequence set forth in SEQ ID NO: 73 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 73, and the light chain variable region comprises the amino acid sequence set forth in SEQ ID NO: 63 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 63;
(6) a heavy chain variable region and a light chain variable region, wherein the heavy chain variable region comprises the amino acid sequence set forth in SEQ ID NO: 74 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 74, and the light chain variable region comprises the amino acid sequence set forth in SEQ ID NO: 63 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 63; or
(7) a heavy chain variable region and a light chain variable region, wherein the heavy chain variable region comprises the amino acid sequence set forth in SEQ ID NO: 74 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 74, and the light chain variable region comprises the amino acid sequence set forth in SEQ ID NO: 68 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 68.

In some embodiments, the antibody is a murine antibody, a monkey antibody, a rabbit antibody, a chimeric antibody, a humanized antibody, or a fully human antibody.

In some embodiments, the antibody is a murine antibody.

In some embodiments, the antibody is a chimeric antibody comprising an extracellular antigen-binding domain, a transmembrane domain and an intracellular signal transduction domain, wherein the extracellular antigen-binding domain comprises the anti-TL1A antibody or the antigen-binding fragment thereof described herein.

In some embodiments, the anti-TL1A antibody or the antigen-binding fragment thereof is at least one selected from a monoclonal antibody, a polyclonal antibody, a neutralizing antibody, an antagonistic antibody, an anti-idiotypic antibody, an afucosylated antibody, a monospecific antibody, a multispecific antibody, a nanobody, a Fab fragment, a Fab' fragment, a F(ab')2 fragment, an Fd fragment, an Fv fragment, a dAb fragment, an isolated CDR region, and an scFv; the anti-TL1A antibody is preferably a monoclonal antibody or a multispecific antibody; preferably, the multispecific antibody is a bispecific antibody, a trispecific antibody or a tetraspecific antibody; or preferably, the multispecific antibody further comprises at least one second antibody having a second binding specificity for a second target.

In some embodiments, the antibody is a monoclonal antibody.

In the present application, the antibody may be monovalent, bivalent or multivalent, i.e., the antibody can bind to one, two or more antigen molecules at a time. An antibody binds "monovalently" to a specific protein, i.e., one molecule of the antibody binds to only one molecule of the protein; however, the antibody may also bind to different proteins. When an antibody binds to only one molecule of each of two different proteins, the antibody binds "monovalently" to each protein, and the antibody is "bispecific" and binds "monovalently" to each of the two different proteins. An antibody may be "monomeric", i.e., it comprises a single polypeptide chain. An antibody may comprise multiple polypeptide chains ("multimeric") or may comprise two ("dimeric"), three ("trimeric") or four ("tetrameric") polypeptide chains. If the antibody is multimeric, the antibody may be a homomultimer, i.e., the antibody comprises more than one molecule of a single polypeptide chain, including homodimers, homotrimers or homotetramers. Optionally, the multimeric antibody may be a heteromultimer, i.e., the antibody comprises more than one different polypeptide chain, including heterodimers, heterotrimers or heterotetramers.

In the present application, conservative modifications may also be made to the anti-TL1A antibody or the antigen-binding fragment thereof. Conservative modification refers to an amino acid modification that does not significantly affect or alter the binding characteristics of the antibody. For example, a conservative amino acid substitution may involve replacing an amino acid with another amino acid of the same class (having similar chemical properties or functions). As an example, amino acids may be grouped according to the properties of their side chains as follows: (1) non-polar amino acids: Ala (A), Val (V), Leu (L), Ile (I), Pro (P), Phe (F), Trp (W), Met (M); (2) uncharged polar amino acids: Gly (G), Ser (S), Thr (T), Cys (C), Tyr (Y), Asn (N), Gln (Q); (3) acidic amino acids: Asp (D), Glu (E); and (4) basic amino acids: Lys (K), Arg (R), His (H). Alternatively, amino acids may be grouped based on common side chain properties as: (1) hydrophobic amino acids: Met, Ala, Val, Leu, Ile; (2) neutral hydrophilic amino acids: Cys, Ser, Thr, Asn, Gln; (3) acidic amino acids: Asp, Glu; (4) basic amino acids: His, Lys, Arg; (5) amino acids affecting chain orientation: Gly, Pro; and (6) aromatic amino acids: Trp, Tyr, Phe

In some embodiments, the anti-TL1A antibody or the antigen-binding fragment thereof further comprises a heavy chain constant region and/or a light chain constant region.

Preferably, the heavy chain constant region is a heavy chain constant region of IgG, IgA, IgM, IgE or IgD or a variant thereof; the heavy chain constant region is preferably a heavy chain constant region of IgG (e.g., human IgG) or a variant thereof, for example, a constant region of IgG1, IgG2, IgG3 or IgG4 (e.g., human IgG1, IgG2, IgG3 or IgG4) or a variant thereof; the heavy chain constant region is more preferably a heavy chain constant region of human IgG1 or a variant thereof, for example, a heavy chain constant region of human IgG1 or a variant thereof.

Preferably, the light chain constant region is a constant region of a human κ chain or λ chain or a variant thereof, preferably a constant region of a human κ chain. In some embodiments, the light chain constant region comprises the amino acid sequence set forth in SEQ ID NO: 76 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity thereto.

In some embodiments, the heavy chain constant region comprises a full-length heavy chain constant region or a fragment thereof, and the fragment can be selected from a CH1 region, a CH2 region, an Fc region and a CH3 region.

In some embodiments, the Fc region comprises a human IgG1 Fc region. Preferably, the human IgG1 Fc region may further comprise any one or more of the following mutations: (a) 297A, 297Q, 297G or 297D; (b) 279F, 279K or 279L; (c) 228P; (d) 235A, 235E, 235G, 235Q, 235R or 235S; (e) 237A, 237E, 237K, 237N or 237R; (f) 234A, 234V or 234F; (g) 233P; (h) 328A; (i) 327Q or 327T; (j) 329A, 329G, 329Y or 329R; (k) 331S; (l) 236F or 236R; (m) 238A, 238E, 238G, 238H, 238I, 238V, 238W or 238Y; (n) 248A; (o) 254D, 254E, 254G, 254H, 254I, 254N, 254P, 254Q, 254T or 254V; (p) 255N; (q) 256H, 256K, 256R or 256V; (r) 264S; (s) 265H, 265K, 265S, 265Y or 265A; (t) 267G, 267H, 267I or 267K; (u) 268K; (v) 269N or 269Q; (w) 270A, 270G, 270M or 270N; (x) 271T; (y) 272N; (z) 292E, 292F, 292G or 292I; (aa) 293S; (bb) 301W; (cc) 304E; (dd) 311E, 311G or 311S; (ee) 316F; (ff) 328V; (gg) 330R; (hh) 339E or 339L; (ii) 343I or 343V; (jj) 373A, 373G or 373S; (kk) 376E, 376W or 376Y; (ll) 380D; (mm) 382D or 382P; (nn) 385P; (oo) 424H, 424M or 424V; (pp) 434I; (qq) 438G; (rr) 439E, 439H or 439Q; (ss) 440A, 440D, 440E, 440F, 440M, 440T or 440V; (tt) E233P; (uu) L235E; (vv) L234A and L235A; (ww) L234A, L235A and G237A; (xx) L234A, L235A and P329G; (yy) L234F, L235E and P331S; (zz) L234A, L235E and G237A; (aaa) L234A, L235E, G237A and P331S; (bbb) L234A, L235A, G237A, P238S, H268A, A330S and P331S (IgG1σ); (ccc) L234A, L235A and P329A; (ddd) G236R and L328R; (eee) G237A; (fff) F241A; (ggg) V264A; (hhh) D265A; (iii) D265A and N297A; (jjj) D265A and N297G; (kkk) D270A; (lll) A330L; (mmm) P331A or P331S; or (nnn) any combination of (a) to (uu), according to Kabat numbering.

In some embodiments, the antibody or antigen-binding fragment comprises: (i) a human IgG4 Fc region, or (ii) a human IgG4 Fc region comprising any one or more mutations selected from (a) S228P, (b) S228P and L235E, and (c) S228P, F234A and L235A, according to Kabat numbering.

In some embodiments, the antibody or antigen-binding fragment comprises a human IgG2 Fc region; an IgG2-IgG4 cross-subclass Fc region; an IgG2-IgG3 cross-subclass Fc region; an IgG2 Fc region comprising any one or more mutations selected from H268Q, V309L, A330S, and P331S (IgG2m4); or an IgG2 Fc region comprising any one or more mutations selected from V234A, G237A, P238S, H268A, V309L, A330S, and P331S (IgG2σ).

In some embodiments, the Fc region comprises a human IgG1 Fc region. Preferably, the human IgG1 Fc region may further have any one or more substitution mutations selected from 329A, 329G, 329Y, 331S, 236F, 236R, 238A, 238E, 238G, 238H, 238I, 238V, 238W, 238Y, 248A, 254D, 254E, 254G, 254H, 254I, 254N, 254P, 254Q, 254T, 254V, 264S, 265H, 265K, 265S, 265Y, 265A, 267G, 267H, 267I, 267K, 434I, 438G, 439E, 439H, 439Q, 440A, 440D, 440E, 440F, 440M, 440T and 440V, according to Kabat numbering.

In some embodiments, the heavy chain constant region is a heavy chain constant region of human IgG1 or a variant thereof, such as those having the L234A/L235A/G237A mutation (referred to as the "AAA mutation"), the LALA mutation (L234A/L235A), the LALA-PG mutation (L234A/L235A/P329G), the YTE mutation, or the LS mutation. In some embodiments, the heavy chain constant region comprises the amino acid sequence set forth in SEQ ID NO: 77 or 75, or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 77 or 75.

In some embodiments, the anti-TL1A antibody or the antigen-binding fragment thereof comprises a heavy chain and a light chain, wherein:
the heavy chain comprises: a heavy chain variable region comprising an amino acid sequence set forth in any one of SEQ ID NOs: 61, 54, 59, 60, 62, 69, 70, 71, 72, 73 and 74 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in any one of SEQ ID NOs: 61, 54, 59, 60, 62, 69, 70, 71, 72, 73 and 74, and a heavy chain constant region comprising the amino acid sequence set forth in SEQ ID NO: 75 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 75; and
the light chain comprises: a light chain variable region comprising an amino acid sequence set forth in any one of SEQ ID NOs: 55, 56, 57, 58, 63, 64, 65, 66, 67 and 68 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in any one of SEQ ID NOs: 55, 56, 57, 58, 63, 64, 65, 66, 67 and 68, and a light chain constant region comprising the amino acid sequence set forth in SEQ ID NO: 76 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 76.

In some embodiments, the anti-TL1A antibody or the antigen-binding fragment thereof comprises a heavy chain and a light chain, wherein:
the heavy chain comprises a sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in any one of SEQ ID NOs: 78, 81 and 83; and
the light chain comprises a sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in any one of SEQ ID NOs: 79, 80, 82 and 84.

In some embodiments, the anti-TL1A antibody or the antigen-binding fragment thereof comprises:
(1) a heavy chain comprising the amino acid sequence set forth in SEQ ID NO: 78 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 78, and
   a light chain comprising the amino acid sequence set forth in SEQ ID NO: 79 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 79;
(2) a heavy chain comprising the amino acid sequence set forth in SEQ ID NO: 78 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 78, and
   a light chain comprising the amino acid sequence set forth in SEQ ID NO: 80 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 80;
(3) a heavy chain comprising the amino acid sequence set forth in SEQ ID NO: 81 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 81, and
   a light chain comprising the amino acid sequence set forth in SEQ ID NO: 82 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 82;
(4) a heavy chain comprising the amino acid sequence set forth in SEQ ID NO: 83 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 83, and
   a light chain comprising the amino acid sequence set forth in SEQ ID NO: 82 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 82;
(5) a heavy chain comprising the amino acid sequence set forth in SEQ ID NO: 81 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 81, and
a light chain comprising the amino acid sequence set forth in SEQ ID NO: 84 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to the amino acid sequence set forth in SEQ ID NO: 84.

In some embodiments, compared to the amino acid sequence set forth in any one of SEQ ID NOs: 78-84, the differing amino acids in the amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity are all located in the FR regions or constant regions.

As non-limiting examples, the anti-TL1A antibody or the antigen-binding fragment thereof has one or more of the following characteristics:
(1) capable of binding to human TL1A;
(2) capable of binding to non-human mammalian TL1A, e.g., murine TL1A, rabbit TL1A, feline TL1A, canine TL1A or porcine TL1A, preferably, capable of binding to murine TL1A;
(3) capable of binding to non-human primate TL1A; preferably, capable of binding to cynomolgus monkey TL1A;
(4) capable of cross-reacting with human TL1A, non-human mammalian TL1A and non-human primate TL1A; preferably, capable of cross-reacting with human TL1A, murine TL1A and cynomolgus monkey TL1A;
(5) having a lower affinity for a human homologue of TL1A than for human TL1A, wherein the human homologue of TL1A is selected from TNFSF6, TNFSF10, TNFSF14, TNF-β, TNF-α, lymphotoxin α2-β1 and lymphotoxin α1-β2;
(6) blocking, inhibiting or reducing the interaction between TL1A and the receptor DR3 (TNFRSF25);
(7) blocking, inhibiting or reducing a signaling pathway triggered by TL1A;
(8) blocking, inhibiting or reducing TL1A-regulated activity; and
(9) blocking, inhibiting or reducing a TL1A-mediated cellular response; for example, lymphocyte proliferation, cytokine expression or lymphocyte survival.

In some embodiments, the anti-TL1A antibody or the antigen-binding fragment thereof has a blocking effect on TL1A-induced caspase activity in TF-1 cells.

In some embodiments, the anti-TL1A antibody or the antigen-binding fragment thereof is capable of cross-binding to human TL1A, cynomolgus monkey TL1A, mouse TL1A and rat TL1A.

In some embodiments, the anti-TL1A antibody or the antigen-binding fragment thereof is capable of specifically binding to human TL1A, mouse TL1A, rat TL1A and cynomolgus monkey TL1A.

In some embodiments of the present application, a nucleic acid molecule is provided, wherein the nucleic acid molecule encodes the anti-TL1A antibody or the antigen-binding fragment thereof described above.

In some embodiments of the present application, a recombinant vector is provided, comprising the nucleic acid molecule described above.

The term "vector" refers to a tool for expressing a target gene in a host cell, for example, plasmid vectors, cosmid vectors, and viral vectors such as phage vectors, lentiviral vectors, adenoviral vectors, retroviral vectors, and adeno-associated viral vectors. Recombinant vectors can be constructed from or by manipulating: plasmids (e.g., pSC101, pGV1106, pACYC177, ColE1, pKT230, pME290, pBR322, pUC8/9, pUC6, pBD9, pHC79, pIJ61, pLAFR1, pHV14, pGEX series, pET series, pUC19, etc.), phages (e.g., λgt4λB, λ-Charon, λΔz1, M13, etc.), or viral vectors (e.g., SV40, etc.), as commonly used in the art.

In a recombinant vector, a nucleic acid molecule may be operably linked to a promoter. The term "operably linked" is intended to refer to a functional linkage between a target nucleotide sequence and an expression regulatory sequence (e.g., a promoter sequence). When "operably linked", the regulatory element can control the transcription and/or translation of the target polynucleotide.

Recombinant vectors can generally be constructed as cloning vectors or expression vectors. For recombinant expression vectors, vectors commonly available in the relevant art for expressing foreign proteins in plant, animal or microbial cells may be used. Various methods well known in the art can be used to construct recombinant vectors.

In order to be used in a host, such as a prokaryotic or eukaryotic cell, a recombinant vector can be constructed accordingly. For example, when constructing a vector as an expression vector for a prokaryotic host, the vector typically comprises a strong promoter for transcription (e.g., pLλ promoter, CMV promoter, trp promoter, lac promoter, tac promoter, T7 promoter, etc.), a ribosome binding site for initiating translation, and transcription/translation termination sequences. On the other hand, an expression vector for a eukaryotic host includes an origin of replication that are operable in a eukaryotic cell, such as an f1 origin of replication, an SV40 origin of replication, a pMB1 origin of replication, an adenoviral origin of replication, an AAV origin of replication, and a BBV origin of replication, but is not limited thereto. Additionally, the expression vector typically includes a promoter derived from a mammalian cell genome (e.g., a metallothionein promoter) or promoter derived from a mammalian virus (e.g., an adenovirus late promoter, a vaccinia virus 7.5K promoter, an SV40 promoter, a cytomegalovirus promoter, a tk promoter of HSV, etc.), as well as a polyadenylation sequence as a transcription termination sequence. Recombinant cells can be prepared by introducing the recombinant vector into a suitable host cell. Any host cell known in the art can be used in the present disclosure, as long as it allows the recombinant vector to be continuously cloned and expressed in a stable manner. Examples of prokaryotic host cells useful in the present disclosure can be selected from Escherichia coli (*E. coli*) such as *E. coli JM109*, *E. coli BL21*, *E. coli RR1*, *E. coli LE392*, *E. coli B*, *E. coli X1776*, and *E. coli W3110*, Bacillus species such as *Bacillus subtilis* and *Bacillus thuringiensis*, and Enterobacteriaceae strains such as *Salmonella typhimurium*, *Serratia marcescens*, and various *Pseudomonas* species. Eukaryotic host cells useful for transformation can be selected from, but are not limited to, *Saccharomyces cerevisiae*, insect cells, and animal cells such as Sp2/0, CHO (Chinese hamster ovary) K1, CHO DG44, CHO S, CHO DXB11, CHO GS-KO, PER.C6, W138, BHK, COS-7, 293, HepG2, Huh7, 3T3, RIN, MDCK, etc. The nucleic acid molecule or the recombinant vector carrying the nucleic acid molecule can be introduced (transfected) into a host cell using methods well known in the relevant art. For example, when the host cell is a prokaryotic cell, such transfection can be performed using CaCl₂ or electroporation. For eukaryotic host cells, gene introduction can be achieved using, but not limited to, microinjection, calcium phosphate precipitation, electroporation, liposome-mediated transfection, or particle bombardment.

To select transformed host cells, a phenotype associated with a selectable marker can be utilized according to methods well known in the art. For example, when the selectable marker is a gene conferring resistance to certain antibiotics, the host cells can be grown in a culture medium containing the antibiotic to select for the target transformant.

In some embodiments of the present application, a host cell is provided, comprising the nucleic acid molecule described above or the recombinant vector described above.

In some embodiments of the present application, a method for preparing an anti-TL1A antibody or an antigen-binding fragment thereof is provided, comprising culturing the host cell described above. The method may further comprise isolating and/or purifying the antibody or the fragment thereof from the cell culture after the expression or culturing step, for example, purifying using a Protein A affinity column.

In some embodiments, the host cell is a prokaryotic cell. In other embodiments, the host cell is a eukaryotic cell.

In some embodiments, the host cell is selected from a yeast cell, a mammalian cell, or other cells suitable for preparing an antibody or an antigen-binding fragment thereof, or a moiety thereof. The mammalian cell is, for example, a Chinese hamster ovary (CHO) cell, a human embryonic kidney cell line 293 cell or a 293T cell. In some embodiments, the host cell is a recombinant CHO cell. In some embodiments, the method further comprises isolating and purifying the expression product of the host cell, for example, purifying using a Protein A affinity column.

A sixth aspect of the present application provides the anti-TL1A antibody or the antigen-binding fragment thereof according to the first aspect, the nucleic acid molecule according to the second aspect, the recombinant vector according to the third aspect, or the host cell according to the fourth aspect, for use in preventing and/or treating a TL1A-mediated related disease or disorder.

In some embodiments, the present application relates to use of the anti-TL1A antibody or the antigen-binding fragment thereof according to the first aspect, the nucleic acid molecule according to the second aspect, the recombinant vector according to the third aspect, or the host cell according to the fourth aspect in the manufacture of a medicament for preventing and/or treating a TL1A-mediated related disease or disorder. Alternatively, the present application relates to a method for preventing and/or treating a TL1A-mediated related disease or disorder, comprising administering to a subject in need thereof the anti-TL1A antibody or the antigen-binding fragment thereof according to the first aspect, the nucleic acid molecule according to the second aspect, the recombinant vector according to the third aspect, or the host cell according to the fourth aspect. Alternatively, the present application relates to the anti-TL1A antibody or the antigen-binding fragment thereof according to the first aspect, the nucleic acid molecule according to the second aspect, the recombinant vector according to the third aspect, or the host cell according to the fourth aspect, for use in preventing and/or treating a TL1A-mediated related disease or disorder.

In some embodiments, the disease or disorder is prevented, alleviated, ameliorated or inhibited by eliminating, inhibiting or reducing TL1A activity.

In some embodiments, the disease or disorder is an immune-related or inflammatory disease.

In some embodiments, the disease or disorder is at least one selected from the group consisting of inflammatory bowel disease (IBD), irritable bowel syndrome (IBS), systemic sclerosis-associated interstitial lung disease, idiopathic pulmonary fibrosis, virus-induced pulmonary fibrosis, asthma, chronic obstructive pulmonary disease (COPD), allergic pulmonary inflammation, neurodegenerative disease, cancer, diabetes, arthritis, rheumatoid arthritis, juvenile rheumatoid arthritis, osteoarthritis, psoriatic arthritis, ankylosing spondylitis, multiple sclerosis, transplant rejection, graft-versus-host disease (GVHD), spondyloarthropathy, primary sclerosing cholangitis, primary biliary cirrhosis, atherosclerosis, bladder syndrome, interstitial cystitis, intestinal and urinary dysfunction, sepsis, uveitis, encephalomyelitis, myasthenia gravis, systemic lupus erythematosus, cutaneous lupus erythematosus, autoimmune thyroiditis, atopic dermatitis, eczematous dermatitis, psoriasis, Sjögren's syndrome, scleroderma, and vasculitis.

In the present application, the inflammatory bowel disease comprises Crohn's disease and ulcerative colitis.

In some embodiments, the disease or disorder is inflammatory bowel disease.

A seventh aspect of the present application provides a kit for detecting TL1A, comprising the anti-TL1A antibody or the antigen-binding fragment thereof according to the first aspect, and/or the nucleic acid molecule according to the second aspect.

The dosage and frequency of administration of the anti-TL1A antibody or the antigen-binding fragment thereof described in the present application can be determined by a clinician based on factors such as the age, weight, sex, general condition, and severity of the disease of the subject; they can be administered by any conventional route known in the art, such as parenteral, intravenous, intramuscular, intra-arterial, intrathecal, intracapsular, intraorbital, intracardiac, intradermal, intraperitoneal, transtracheal, subcutaneous, subepidermal, intra-articular, subcapsular, subarachnoid, intraspinal, epidural, and intrasternal administration.

### Examples

The present application will be further described below with reference to the examples. However, those skilled in the art will appreciate that the protection scope of the present application is not limited to these examples.

Unless otherwise specified, the reagents, materials or instruments used in the following examples are commercially available.

### Example 1: Construction of a Reporter Gene Cell Line for Functional Activity Evaluation

The anti-TL1A antibody 1D1 from Pfizer was used as a positive control antibody. The amino acid sequence of 1D1 was derived from patent WO2021260577 (the heavy chain sequence and the light chain sequence of 1D1 are set forth in SEQ ID NO: 52 and SEQ ID NO: 53, respectively).

The light chain and heavy chain genes of 1D1 were synthesized and then subcloned into the pcDNA3.4 expression vector, respectively. CHO-K1 cells were transfected using BIOHUB 78 PEI transfection reagent (purchased from Shanghai BioHub) and cultured at 37°C with 5% CO₂. Subsequently, the cell culture was collected, centrifuged, and purified using a Protein A affinity column.

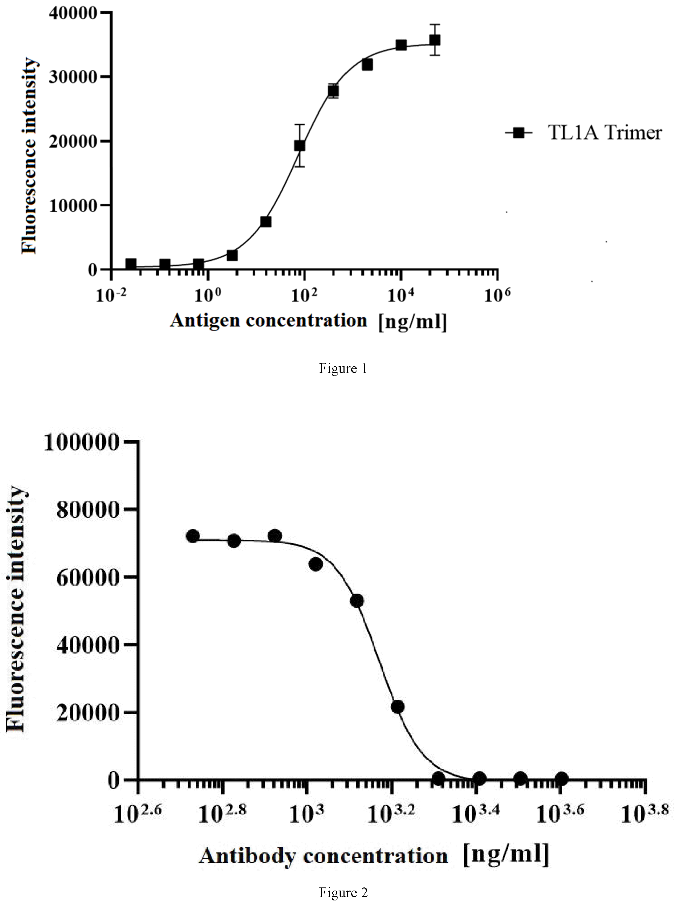

TF-1 cells in the logarithmic growth phase were seeded into 6-well plates at 1.5×10⁵ cells/3 mL per well, and were infected with PCDH-NFkB-Luc-CMV-EGFP-Hygro lentivirus (MOI = 20). Then, 500 µg/mL Hygromycin B was added to select for stably transfected cell strains, and a TF-1-NFκB-Luc reporter gene cell line was obtained. The activation effect of human TL1A on this reporter gene cell line was verified according to the following steps.
(1) TF-1-NFκB-Luc reporter gene cells in the logarithmic growth phase were taken, washed twice with RPMI 1640 + 10% FBS medium, and adjusted to a density of 4×10⁵ cells/mL. Then, 10 µL of the cell suspension was aspirated and added to a 384-well white plate, with 4×10³ cells per well.
(2) Human TL1A trimer protein (purchased from KACTUS) was subjected to 5-fold serial dilutions starting from a maximum concentration of 200 µg/mL using RPMI 1640 + 10% FBS medium, for a total of 10 dilution gradients. Then, 10 µL of the diluted protein at each gradient concentration was added to the above 384-well white plate, with two replicate wells per concentration gradient. The final concentrations of TL1A trimer per well were 200 µg/mL, 40 µg/mL, 8 µg/mL, 1.6 µg/mL, 0.32 µg/mL, 0.064 µg/mL, 0.0128 µg/mL, 0.00256 µg/mL, 0.000512 µg/mL, and 0.000102 µg/mL.
(3) The reaction system was supplemented with medium to a final volume of 40 µL per well. The plate was placed in an incubator and cultured at 37°C with 5% CO₂ for 18 hours.
(4) After the culture, Bio Lite^{TM} and the plate were equilibrated to room temperature for 30 minutes. Then, 20 µL of Bio Lite^{TM} detection reagent (purchased from Vazyme) was added to each well. The Luciferase fluorescence value was read using a BMG PLOARstar Omega multimode microplate reader. The data are shown in Figure 1 (a graph showing the results of TL1A-induced reporter gene signal), indicating that the reporter gene cell line can be activated by human TL1A trimer in a dose-dependent manner.

The blocking effect of the positive control antibody 1D1 on human TL1A-induced signaling was verified according to the following steps.
(1) The positive control antibody 1D1 was subjected to 1.25-fold serial dilutions starting from a maximum concentration of 6 µg/mL using RPMI 1640 + 10% FBS medium, for a total of 10 dilution gradients. Then, 20 µL of the diluted antibody at each concentration gradient was added to a 384-well white plate, with two replicate wells per concentration gradient, so that the antibody concentrations in the 384-well white plate were 8 µg/mL, 6.4 µg/mL, 5.12 µg/mL, 4.096 µg/mL, 3.277 µg/mL, 2.621 µg/mL, 2.097 µg/mL, 1.678 µg/mL, 1.342 µg/mL, and 1.074 µg/mL, respectively. Then, 10 µL of 2 µg/mL human TL1A trimer protein (purchased from KACTUS) was added to each corresponding well, resulting in a final concentration of 0.5 µg/mL. The plate was incubated at 37°C with 5% CO₂ for 1 hour.
(2) TF-1-NFκB-Luc cells in the logarithmic growth phase were taken, washed twice with RPMI 1640 + 10% FBS medium, and adjusted to a density of 4×10⁵ cells/mL with RPMI 1640 + 10% FBS medium. Then, 10 µL of the cell suspension was aspirated and added to the above 384-well white plate, with 4×10³ cells per well. The plate was cultured at 37°C with 5% CO₂ for 18 hours.
(3) After the culture, Bio Lite^{TM} and the plate were equilibrated to room temperature for 30 minutes. Then, 20 µL of Bio Lite^{TM} detection reagent (purchased from Vazyme) was added to each well. The Luciferase fluorescence value was read using a BMG PLOARstar Omega multimode microplate reader. The data are shown in Figure 2 (a graph showing the activity of positive control antibody 1D1 detected by reporter gene assay), indicating that the positive control antibody 1D1 can block TL1A-induced reporter gene signaling in a dose-dependent manner, and that this functional assay system can be used for activity evaluation of candidate antibodies.

### Example 2: Hybridoma Screening

Ten Balb/c mice and ten CD1 mice (SPF grade) were selected and immunized three times at 14-day intervals. For the first immunization, 50 µg of human TL1A (purchased from KACTUS) emulsified with Freund's adjuvant was administered subcutaneously at multiple sites. For the second and third immunizations, 25 µg of human TL1A and 25 µg of mouse TL1A (both purchased from KACTUS) emulsified with Freund's adjuvant were administered subcutaneously at multiple sites. Mice with good immune titer were selected for booster immunization. Three days later, the mice were euthanized by cervical dislocation. The spleens were aseptically removed, and single-cell suspensions of B cells were prepared. The prepared B cells were mixed with SP2/0 myeloma cells at a ratio of 2:1 and fused using a BTX cell electrofusion apparatus. After electrofusion, all cells were immediately suspended in DMEM medium containing 20% FBS and HAT, and seeded into 96-well plates. Approximately 10 days after fusion, the medium was replaced with HT medium, and the cells were cultured for two additional days. Subsequently, the culture supernatants were collected and assayed for specific antibodies. 100 µL of culture supernatant was taken from each well of the 96-well plate, and each well was simultaneously supplemented with fresh HT-containing DMEM medium. The collected culture supernatant was tested for binding to human TL1A (purchased from KACTUS), mouse TL1A (purchased from KACTUS), rat TL1A (purchased from Sino Biological), and cynomolgus monkey TL1A (purchased from KACTUS) by ELISA (HRP Anti-Mouse IgG H&L was purchased from Biodragon, and TMB chromogenic solution was purchased from Huzhou InnoReagents). Clones capable of cross-binding to human TL1A, cynomolgus monkey TL1A, mouse TL1A and rat TL1A were preferred. Monoclonal antibodies that specifically bound to human TL1A, mouse TL1A, rat TL1A and cynomolgus monkey TL1A were obtained through two rounds of subcloning.

The hybridomas of the monoclonal antibodies obtained from the two rounds of subcloning were expanded and cultured, and then the murine antibodies were purified using a Protein A affinity column. The purified murine antibodies and the positive control antibody 1D1 were subjected to reporter gene assay according to the procedure described in Example 1. Nine murine antibodies with activity superior to or comparable to that of the positive control antibody 1D1 were selected. The light chain and heavy chain variable region amino acid sequences of these antibodies were obtained through hybridoma sequencing.

### Example 3: Construction and Expression of Chimeric Antibodies

Based on the light chain and heavy chain variable region amino acid sequences of the nine murine antibodies obtained by sequencing, light chain and heavy chain variable region genes were synthesized. The heavy chain variable region genes were subcloned into the pcDNA3.4-hIgG1 expression vector, in which the heavy chain constant region was modified by L234A/L235A/G237A mutations to eliminate effector function. The light chain variable region genes were subcloned into the pcDNA3.4-hIgKc expression vector. Thus, heavy chain expression vectors and light chain expression vectors were obtained. After the constructed vectors were verified by sequencing, CHO-K1 cells were transiently transfected with the vectors. 120 µg of BIOHUB 78 PEI transfection reagent (purchased from Shanghai BioHub) was added to 1.5 mL of OPM-Trans CHO transfection medium (purchased from OPM), and mixed thoroughly. The mixture was then added to 1.5 mL of OPM-Trans CHO transfection medium containing 30 µg of the above expression vectors, mixed thoroughly, and allowed to stand at room temperature for 20 minutes. The resulting complex was then added to 30 mL of CHO-K1 cells at a density of 4×10⁶ cells/mL, gently shaken to mix thoroughly, and cultured at 37°C with 5% CO₂ at 100 rpm. The temperature was reduced to 32°C on the first day after transfection, and 2.5% OPM-CHO ProFeed medium (purchased from OPM) was added on days 1, 3, 5 and 7. The cells were cultured until day 8, and then the culture supernatant was collected by centrifugation. The chimeric antibodies were purified by Protein A affinity column chromatography. Table 1 shows the CDR sequences of the chimeric antibodies.

**Table 1: CDR sequences of chimeric antibodies**

| Chimeric antibody number | | CDR1 | | CDR2 | | CDR3 | |
|---|---|---|---|---|---|---|---|
| | | SEQ ID NO. | Sequence | SEQ ID NO. | Sequence | SEQ ID NO. | Sequence |
| 16H6 | heavy chain | 1 | GYTFTSYW | 2 | IFPGSGST | 3 | SEGDYSFAY |
| | light chain | 4 | QDINKY | 5 | STS | 6 | QQGSMLPWT |
| 12B3 | heavy chain | 7 | GYPFTDFY | 8 | VNPDNGDT | 9 | ARERVGYWFAY |
| | light chain | 10 | QNVGSA | 11 | SAS | 12 | QQYSSFPLT |
| 56C8 | heavy chain | 13 | GFNIKDDY | 14 | IDPANGNT | 15 | ASGRNYGTYWHFDV |
| | light chain | 16 | QDINRY | 17 | RAN | 18 | LQYDEFPYT |
| 5C6 | heavy chain | 19 | GYSFTGYT | 20 | INPYSGGT | 21 | ARISYRYEAMDC |
| | light chain | 22 | SSVSY | 23 | DTS | 24 | QQWSSDPYT |
| 28G7 | heavy chain | 25 | SYSFTGYT | 20 | INPYSGGT | 26 | ARIGGRVDAMDY |
| | light chain | 22 | SSVSY | 23 | DTS | 27 | QQWSSNPYT |
| 70A3 | heavy chain | 28 | GYSITSDY | 29 | IYYNGGT | 30 | AKAGSSYVGLDY |
| | light chain | 31 | QDISNY | 32 | YTS | 33 | QQGRTLPWT |
| 51G9 | heavy chain | 34 | GYSITSGYY | 35 | ISSDGRS | 36 | VRGGLRWDFES |
| | light chain | 37 | KSVSTSGYNY | 38 | LAS | 39 | QHSRELPPT |
| 69B1 | heavy chain | 40 | GYTFTGYW | 41 | ILPGDDSA | 42 | ARRAYYPYDAMDY |
| | light chain | 43 | QSIVHRNTYTY | 44 | GVS | 45 | FQSTHVPYT |
| 13B8 | heavy chain | 46 | GFSLTDYA | 47 | IYRSGST | 48 | AKNGDGFSWYFDV |
| | light chain | 49 | GNIHTY | 50 | NAK | 51 | QHFWGNPPT |

### Example 4: Activity Detection of Chimeric Antibodies by Reporter Gene Assay

The activity of the nine chimeric antibodies and the positive control antibody 1D1 was detected by reporter gene assay according to the procedure described in Example 1. Table 2 and Figures 3-1 to 3-4 (graphs showing the activity of chimeric antibodies detected by reporter gene assay) show the activity detection results of the reporter gene assay. It can be seen that both the chimeric antibodies and the positive control antibody could block the fluorescence signal of the TL1A-induced reporter gene cell line in a dose-dependent manner. From the curves and IC₅₀ values, the activities of chimeric antibodies 12B3 and 16H6 were significantly better than that of the positive control antibody 1D1, while the activities of the remaining chimeric antibodies were comparable to that of 1D1.

**Table 2: Activity detection results of chimeric antibodies by reporter gene assay**

| Chimeric antibody number | IC₅₀ (ng/mL) | |
|---|---|---|
| | Chimeric antibody | 1D1 |
| 5C6 | 1511 | 1450 |
| 28G7 | 1568 | 1482 |
| 70A3 | 1576 | 1533 |
| 16H6 | 1102 | 1482 |
| 51G9 | 1421 | 1522 |
| 69B1 | 1345 | 1522 |
| 12B3 | 923.9 | 1522 |
| 56C8 | 1381 | 1522 |
| 13B8 | 1414 | 1533 |

### Example 5: Activity Detection of Chimeric Antibodies by Caspase Assay

The activity of the nine chimeric antibodies and the positive control antibody 1D1 was detected by caspase assay according to the following steps, in order to evaluate the blocking effect of the candidate antibodies on TL1A and Cycloheximide co-induced caspase activity in TF-1 cells.
(1) Cycloheximide (purchased from MCE) was diluted to 10 µg/mL with RPMI 1640 + 10% FBS medium, and 10 µL of the diluted solution was added per well to a 384-well white plate.
(2) TL1A trimer (purchased from KACTUS) was diluted to 200 ng/mL with RPMI 1640 + 10% FBS medium, and 10 µL of the diluted solution was added per well to the above 384-well white plate.
(3) The positive control antibody 1D1 and the nine chimeric antibodies were subjected to 1.6-fold serial dilutions starting from a maximum concentration of 6.4 µg/mL using RPMI 1640 + 10% FBS medium, for a total of 10 dilution gradients. The antibody concentrations were 6.4 µg/mL, 4 µg/mL, 2.5 µg/mL, 1.562 µg/mL, 0.976 µg/mL, 0.610 µg/mL, 0.381 µg/mL, 0.238 µg/mL, 0.149 µg/mL, and 0.0931 µg/mL, respectively. Then, 10 µL of each diluted antibody was added to the above 384-well white plate, with two replicate wells per concentration gradient. The 384-well plate was incubated at 37°C with 5% CO₂ for 1 hour.
(2) TF-1 cells in the logarithmic growth phase were taken, washed twice with RPMI 1640 + 10% FBS medium, and adjusted to a density of 6×10⁵ cells/mL with RPMI 1640 + 10% FBS medium. Then, 10 µL of the cell suspension was aspirated and added to the above 384-well white plate, with 6×10³ cells per well. The plate was cultured at 37°C with 5% CO₂ for 18 hours.
(3) After the culture, Caspase-Glo 3/7 Reagent (purchased from Promega) and the plate were equilibrated to room temperature. Then, 40 µL of Caspase-Glo 3/7 Reagent was added to each well, and the plate was reacted at room temperature for 1 hour.
(4) The Luciferase fluorescence value was read using a BMG PLOARstar Omega multimode microplate reader.

Table 3 and Figures 4-1 to 4-6 (graphs showing the activity of chimeric antibodies detected by caspase assay) show the activity detection results of the caspase assay. It can be seen that both the chimeric antibodies of the present application and the positive control could block TL1A-induced caspase activation in a dose-dependent manner. From the curves and IC₅₀ values, the activities of the chimeric antibodies 12B3, 56C8 and 16H6 of the present application were superior to that of the positive control antibody 1D1, while the activities of the remaining chimeric antibodies were comparable to that of 1D1.

**Table 3: Activity detection results of chimeric antibodies by caspase assay**

| Chimeric antibody number | IC₅₀ (ng/mL) | |
|---|---|---|
| | Chimeric antibody | 1D1 |
| 5C6 | 214.4 | 216 |
| 28G7 | 310.9 | 216 |
| 16H6 | 169.5 | 216 |
| 51G9 | 152.9 | 216 |
| 69B1 | 174.5 | 216 |
| 12B3 | 120.8 | 216 |
| 56C8 | 161 | 216 |
| 13B8 | 212.1 | 216 |
| 70A3 | 281.5 | 176.3 |

### Example 6: Binding Activity Detection of Chimeric Antibodies

The binding of five preferred chimeric antibodies, 16H6, 12B3, 56C8, 69B1, and 13B8, as well as the positive control antibody 1D1, to human TL1A monomer and trimer was detected by ELISA according to the following steps.
(1) Coating: 96-well plates were coated with 1 µg/mL of target protein (human TL1A monomer or trimer) at 100 µL per well, and were incubated overnight at 4°C. The plates were then washed once with PBST.
(2) Blocking: Each well was blocked with PBST containing 2% BSA for 2 hours at room temperature, and then the plates were washed once with PBST.
(3) Primary antibody incubation: The antibodies to be tested were serially diluted in 3.5-fold gradients using PBST containing 0.5% BSA to final concentrations of 10 µg/mL, 2.86 µg/mL, 0.816 µg/mL, 0.233 µg/mL, 0.0666 µg/mL, 0.0190 µg/mL, 0.00544 µg/mL, 0.00155 µg/mL, 0.000444 µg/mL, 0.000127 µg/mL, and 0.0000363 µg/mL. Then, 100 µL per well of the diluted antibodies were added to the above 96-well plates, and the plates were incubated at 37°C for 1 hour, and then washed three times with PBST.
(4) Secondary antibody incubation: A secondary antibody, Anti-human-Fc-IgG-HRP (purchased from Thermo), diluted 1:30000 in PBST containing 0.5% BSA, was added to the above 96-well plates. The plates were incubated at 37°C for 1 hour, and then washed five times with PBST.
(5) Termination: TMB (purchased from Huzhou Shenke) was added to the above 96-well plates at 100 µL per well, and the plates were incubated at room temperature for 10 minutes for color development. Then, 50 µL per well of stop solution (1 M sulfuric acid) was added to terminate the reaction. The OD 450 and OD 630 values were read using a microplate reader.

Table 4 and Figures 5-1, 5-2 (graphs showing the binding activity of chimeric antibodies to human TL1A monomer), and Figures 6-1, 6-2 (graphs showing the binding activity of chimeric antibodies to human TL1A trimer) show the binding of the chimeric antibodies to human TL1A monomer and trimer. It can be seen that the chimeric antibodies of the present application all exhibited strong binding to both human TL1A monomer and trimer, and the binding activity was comparable to that of the positive control antibody 1D1.

**Table 4: Binding activity of chimeric antibodies to human TL1A monomer and trimer**

| Chimeric antibody number | EC50 (ng/mL) | |
|---|---|---|
| | Human TL1A monomer | Human TL1A trimer |
| Positive control antibody 1D1 | 11.80 | 13.78 |
| 16H6 | 8.317 | 11.89 |
| 12B3 | 6.593 | 9.195 |
| 56C8 | 8.729 | 14.13 |
| 69B1 | 8.604 | 12.22 |
| 13B8 | 9.851 | 11.10 |

### Example 7: Antibody Humanization

The preferred chimeric antibodies 16H6 and 12B3 were subjected to antibody humanization engineering. Humanization design was performed based on the amino acid sequences of the heavy and light chains of the murine antibody. The CDR region sequences of the original antibodies were retained. Different human antibody templates were selected for the heavy and light chains, respectively, based on the results of germline alignment and antibody structure modeling. Back-mutations were introduced into the framework regions after humanization to design candidate humanized antibody sequences. The heavy and light chains of the designed humanized antibodies were separately subjected to gene synthesis. The heavy chain genes were subcloned into the pcDNA3.4-hIgG1 expression vector, in which the heavy chain constant region was modified by L234A/L235A/G237A mutations to eliminate effector function. The light chain genes were subcloned into the pcDNA3.4-hIgKc expression vector. After the constructed vectors were verified by sequencing, CHO cells were transiently transfected with the vectors according to the method described in Example 3. The variable region and constant region sequences of the humanized antibodies are shown in Table 5.

**Table 5: Heavy chain and light chain amino acid sequences of humanized antibodies**

| Antibody number | Location | Amino acid sequence | SEQ ID NO. |
|---|---|---|---|
| 16H6-H1L1 | heavy chain variable region | EVQLVQSVAEVKKPGASVKVSCKASGYTFTSYWITWVRQAPG QGLEWIGNIFPGSGSTNYNEKFKGRVTLTVDTSSSTAYMELSSLR SEDTAVYYCSEGDYSFAYWGQGTLVTVSS | 54 |
| | light chain variable region | DIQMTQSPSSLSASVGDRVTITCRASQDINKYLNWYQQKPGGT VKLLIYSTSRLHSGVPSRFSGSGSGTDYSLTISNLQPEDIATYYCQ QGSMLPWTFGGGTKVEIK | 55 |
| 16H6-H1L2 | heavy chain variable region | identical to the heavy chain variable region of 16H6-H1L1 | 54 |
| | light chain variable region | DIQMTQSPSSLSASVGDRVTITCRASQDINKYLNWYQQKPGGT VKLLIYSTSRLHSGVPSRFSGSGSGTDYSLTISSLQPEDFATYYCQ QGSMLPWTFGGGTKVEIK | 56 |
| 16H6-H1L3 | heavy chain variable region | identical to the heavy chain variable region of 16H6-H1L1 | 54 |
| | light chain variable region | DIQMTQSPSSLSASVGDRVTITCRASQDINKYLNWYQQKPGGT VKLLLYSTSRLHSGVPSRFSGSGSGTDYSLTISSLQPEDFATYYC QQGSMLPWTFGGGTKVEIK | 57 |
| 16H6-H1L4 | heavy chain variable region | identical to the heavy chain variable region of 16H6-H1L1 | 54 |
| | light chain variable region | DIQMTQSPSSLSASVGDRVTITCRASQDINKYLNWYQQKPGGT VKLLIYSTSRLHSGVPSRFSGSGSGTDYSLTISSLQPEDVATYYCQ QGSMLPWTFGGGTKVEIK | 58 |
| 16H6-H2L1 | heavy chain variable region | EVQLVQSVAEFKKPGASVKMSCKASGYTFTSYWITWVRQAPG QGLEWIGNIFPGSGSTNYNEKFKGRVTLTVDTSSSTAYMELSSLR SEDTAVYYCSEGDYSFAYWGQGTLVTVSS | 59 |
| | light chain variable region | identical to the light chain variable region of 16H6-H1L1 | 55 |
| 16H6-H2L2 | heavy chain variable region | identical to the heavy chain variable region of 16H6-H2L1 | 59 |
| | light chain variable region | identical to the light chain variable region of 16H6-H1L2 | 56 |
| 16H6-H2L3 | heavy chain variable region | identical to the heavy chain variable region of 16H6-H2L1 | 59 |
| | light chain variable region | identical to the light chain variable region of 16H6-H1L3 | 57 |
| 16H6-H2L4 | heavy chain variable region | identical to the heavy chain variable region of 16H6-H2L1 | 59 |
| | light chain variable region | identical to the light chain variable region of 16H6-H1L4 | 58 |
| 16H6-H3L1 | heavy chain variable region | EVQLVQSGAEVKKPGASVKVSCKASGYTFTSYWITWVRQAPG QGLEWIGNIFPGSGSTNYNEKFKGRVTLTVDTSSSTAYMELSRL RSEDTAVYYCSEGDYSFAYWGQGTLVTVSS | 60 |
| | light chain variable region | identical to the light chain variable region of 16H6-H1L1 | 55 |
| 16H6-H3L2 | heavy chain variable region | identical to the heavy chain variable region of 16H6-H3L1 | 60 |
| | light chain variable region | identical to the light chain variable region of 16H6-H1L2 | 56 |
| 16H6-H3L3 | heavy chain variable region | identical to the heavy chain variable region of 16H6-H3L1 | 60 |
| | light chain variable region | identical to the light chain variable region of 16H6-H1L3 | 57 |
| 16H6-H3L4 | heavy chain variable region | identical to the heavy chain variable region of 16H6-H3L1 | 60 |
| | light chain variable region | identical to the light chain variable region of 16H6-H1L4 | 58 |
| 16H6-H4L1 | heavy chain variable region | EVQLVQSGAEVKKPGASVKVSCKASGYTFTSYWITWVRQAPG QRLEWIGNIFPGSGSTNYNEKFKGRVTLTVDTSSSTAYMELSSLR SEDTAVYYCSEGDYSFAYWGQGTLVTVSS | 61 |
| | light chain variable region | identical to the light chain variable region of 16H6-H1L1 | 55 |
| 16H6-H4L2 | heavy chain variable region | identical to the heavy chain variable region of 16H6-H4L1 | 61 |
| | light chain variable region | identical to the light chain variable region of 16H6-H1L2 | 56 |
| 16H6-H4L3 | heavy chain variable region | identical to the heavy chain variable region of 16H6-H4L1 | 61 |
| | light chain variable region | identical to the light chain variable region of 16H6-H1L3 | 57 |
| 16H6-H4L4 | heavy chain variable region | identical to the heavy chain variable region of 16H6-H4L1 | 61 |
| | light chain variable region | identical to the light chain variable region of 16H6-H1L4 | 58 |
| 12B3-H1L1 | heavy chain variable region | EVQLVQSGAEVKKPGASVKVSCKASGYPFTDFYMNWVRQAH GKSLEWIGEVNPDNGDTSHNQKFQGRVTLTVDKSTRTAYMELS SLRSEDTAVYYCARERVGYWFAYWGQGTLVTVSS | 62 |
| | light chain variable region | DIVMTQSPKFVSASVGDRVTITCRASQNVGSAVAWYQQKPGQS PKILIYSASNRYSGVPSRFSGSGSGTDFTLTISSLQPEDFADYFCQ QYSSFPLTFGQGTKLEIK | 63 |
| 12B3-H1L2 | heavy chain variable region | identical to the heavy chain variable region of 12B3-H1L1 | 62 |
| | light chain variable region | DIVMTQSPKFLSTSTGDRVTITCRASQNVGSAVAWYQQKPGKAP KILIYSASNRYSGVPSRFSGSGSGTDFTLTINNLLSEDFADYFCQQ YSSFPLTFGQGTKLEIK | 64 |
| 12B3-H1L4 | heavy chain variable region | identical to the heavy chain variable region of 12B3-H1L1 | 62 |
| | light chain variable region | DIQMTQSPSFLSASVGDRVTITCRASQNVGSAVAWYQQKPGQSP KILIYSASNRYSGVPSRFSGSGSGTEFTLTISSLLPEDFADYFCQQ YSSFPLTFGQGTKLEIK | 65 |
| 12B3-H1L5 | heavy chain variable region | identical to the heavy chain variable region of 12B3-H1L1 | 62 |
| | light chain variable region | DIVMTQSPKFLSTSTGDRVTITCKASQNVGSAVAWYQQRPGQSP KILIYSASNRYIGVPSRFSGSGSGTDFTLTINNLLSEDFADYFCQQ YSSFPLTFGQGTKLEIK | 66 |
| 12B3-H1L6 | heavy chain variable region | identical to the heavy chain variable region of 12B3-H1L1 | 62 |
| | light chain variable region | DIVMTQSPKFVSTSVGDRVTITCKASQNVGSAVAWYQQKPGQS PKILIYSASNRYIGVPSRFSGSGSGTDFTLTISSLLPEDFADYFCQQ YSSFPLTFGQGTKLEIK | 67 |
| 12B3-H1L8 | heavy chain variable region | identical to the heavy chain variable region of 12B3-H1L1 | 62 |
| | light chain variable region | DIQMTQSPSFLSTSVGDRVTITCKASQNVGSAVAWYQQKPGQSP KILIYSASNRYIGVPSRFSGSGSGTEFTLTISSLLPEDFADYFCQQY SSFPLTFGQGTKLEIK | 68 |
| 12B3-H2L1 | heavy chain variable region | EVQLVQSGAEVKKPGASVKVSCKASGYPFTDFYMNWVRQAH GKSLEWIGEVNPDNGDTSHNQEFQGRVTLTVDKSARTAYMELS SLRSEDMAVYYCARERVGYWFAYWGQGTLVTVSS | 69 |
| | light chain variable region | identical to the light chain variable region of 12B3-H1L1 | 63 |
| 12B3-H2L2 | heavy chain variable region | identical to the heavy chain variable region of 12B3-H2L1 | 69 |
| | light chain variable region | identical to the light chain variable region of 12B3-H1L2 | 64 |
| 12B3-H2L4 | heavy chain variable region | identical to the heavy chain variable region of 12B3-H2L1 | 69 |
| | light chain variable region | identical to the light chain variable region of 12B3-H1L4 | 65 |
| 12B3-H2L5 | heavy chain variable region | identical to the heavy chain variable region of 12B3-H2L1 | 69 |
| | light chain variable region | identical to the light chain variable region of 12B3-H1L5 | 66 |
| 12B3-H3L1 | heavy chain variable region | EVQLVQSGAEVKKPGASVKVSCKASGYPFTDFYMNWVRQAH GKSLEWMGEVNPDNGDTSHNQKFQGRVTLTVDKSTRTAYMEL SSLRSEDTAVYYCARERVGYWFAYWGQGTLVTVSS | 70 |
| | light chain variable region | identical to the light chain variable region of 12B3-H1L1 | 63 |
| 12B3-H3L2 | heavy chain variable region | identical to the heavy chain variable region of 12B3-H3L1 | 70 |
| | light chain variable region | identical to the light chain variable region of 12B3-H1L2 | 64 |
| 12B3-H3L4 | heavy chain variable region | identical to the heavy chain variable region of 12B3-H3L1 | 70 |
| | light chain variable region | identical to the light chain variable region of 12B3-H1L4 | 65 |
| 12B3-H3L5 | heavy chain variable region | identical to the heavy chain variable region of 12B3-H3L1 | 70 |
| | light chain variable region | identical to the light chain variable region of 12B3-H1L5 | 66 |
| 12B3-H3L6 | heavy chain variable region | identical to the heavy chain variable region of 12B3-H3L1 | 70 |
| | light chain variable region | identical to the light chain variable region of 12B3-H1L6 | 67 |
| 12B3-H3L8 | heavy chain variable region | identical to the heavy chain variable region of 12B3-H3L1 | 70 |
| | light chain variable region | identical to the light chain variable region of 12B3-H1L8 | 68 |
| 12B3-H4L1 | heavy chain variable region | EVQLVQSGAEVKKPGASVKVSCKASGYPFTDFYMNWVRQAH GKSLEWMGEVNPDNGDTSHNQKFQGRVTLTVDKSARTAYMEL SSLRSEDTAVYYCARERVGYWFAYWGQGTLVTVSS | 71 |
| | light chain variable region | identical to the light chain variable region of 12B3-H1L1 | 63 |
| 12B3-H4L2 | heavy chain variable region | identical to the heavy chain variable region of 12B3-H4L1 | 71 |
| | light chain variable region | identical to the light chain variable region of 12B3-H1L2 | 64 |
| 12B3-H4L4 | heavy chain variable region | identical to the heavy chain variable region of 12B3-H4L1 | 71 |
| | light chain variable region | identical to the light chain variable region of 12B3-H1L4 | 65 |
| 12B3-H4L5 | heavy chain variable region | identical to the heavy chain variable region of 12B3-H4L1 | 71 |
| | light chain variable region | identical to the light chain variable region of 12B3-H1L5 | 66 |
| 12B3-H4L6 | heavy chain variable region | identical to the heavy chain variable region of 12B3-H4L1 | 71 |
| | light chain variable region | identical to the light chain variable region of 12B3-H1L6 | 67 |
| 12B3-H4L8 | heavy chain variable region | identical to the heavy chain variable region of 12B3-H4L1 | 71 |
| | light chain variable region | identical to the light chain variable region of 12B3-H1L8 | 68 |
| 12B3-H6L1 | heavy chain variable region | EVQLVQSGAEVKKPGASVKVSCKASGYPFTDFYMNWVKQAH GKSLEWIGEVNPDNGDTSHNQEFEDRVTLTVDKSSRTAYMELSS LRSEDTAVYYCARERVGYWFAYWGQGTLVTVSS | 72 |
| | light chain variable region | identical to the light chain variable region of 12B3-H1L1 | 63 |
| 12B3-H6L4 | heavy chain variable region | identical to the heavy chain variable region of 12B3-H6L1 | 72 |
| | light chain variable region | identical to the light chain variable region of 12B3-H1L4 | 65 |
| 12B3-H6L6 | heavy chain variable region | identical to the heavy chain variable region of 12B3-H6L1 | 72 |
| | light chain variable region | identical to the light chain variable region of 12B3-H1L6 | 67 |
| 12B3-H6L8 | heavy chain variable region | identical to the heavy chain variable region of 12B3-H6L1 | 72 |
| | light chain variable region | identical to the light chain variable region of 12B3-H1L8 | 68 |
| 12B3-H7L1 | heavy chain variable region | EVQLVQSGAEVKKPGASVKVSCKASGYPFTDFYMNWVRQAH GKSLEWIGEVNPDNGDTSHNQEFEDRVTLTVDKSTRTAYMELSS LRSEDTAVYYCARERVGYWFAYWGQGTLVTVSS | 73 |
| | light chain variable region | identical to the light chain variable region of 12B3-H1L1 | 63 |
| 12B3-H7L4 | heavy chain variable region | identical to the heavy chain variable region of 12B3-H7L1 | 73 |
| | light chain variable region | identical to the light chain variable region of 12B3-H1L4 | 65 |
| 12B3-H7L6 | heavy chain variable region | identical to the heavy chain variable region of 12B3-H7L1 | 73 |
| | light chain variable region | identical to the light chain variable region of 12B3-H1L6 | 67 |
| 12B3-H7L8 | heavy chain variable region | identical to the heavy chain variable region of 12B3-H7L1 | 73 |
| | light chain variable region | identical to the light chain variable region of 12B3-H1L8 | 68 |
| 12B3-H8L1 | heavy chain variable region | EVQLVQSGAEVKKPGASVKVSCKASGYPFTDFYMNWVRQAH GKSLEWIGEVNPDNGDTSHNQEFEDRVTLTVDKSARTAYMELS SLRSEDTAVYYCARERVGYWFAYWGQGTLVTVSS | 74 |
| | light chain variable region | identical to the light chain variable region of 12B3-H1L1 | 63 |
| 12B3-H8L6 | heavy chain variable region | identical to the heavy chain variable region of 12B3-H8L1 | 74 |
| | light chain variable region | identical to the light chain variable region of 12B3-H1L6 | 67 |
| 12B3-H8L8 | heavy chain variable region | identical to the heavy chain variable region of 12B3-H8L1 | 74 |
| | light chain variable region | identical to the light chain variable region of 12B3-H1L8 | 68 |
| Candidate antibody heavy chain constant region | | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKKVEPKSCDKTHTCPPCPAPEAAGAPSVFLFPPKPKDT LMISRTPEVTCVVVDVSHEDPEVKFNWYVDGVEVHNAKTKPR EEQYNSTYRVVSVLTVLHQDWLNGKEYKCKVSNKALPAPIEKT ISKAKGQPREPQVYTLPPSREEMTKNQVSLTCLVKGFYPSDIAVE WESNGQPENNYKTTPPVLDSDGSFFLYSKLTVDKSRWQQGNVF SCSVMHEALHNHYTQKSLSLSPG | 75 |
| Candidate antibody light chain constant region | | | 76 |
| Wild-type heavy chain constant region (C-terminal Lys removed) | | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKKVEPKSCDKTHTCPPCPAPELLGGPSVFLFPPKPKDT LMISRTPEVTCVVVDVSHEDPEVKFNWYVDGVEVHNAKTKPR EEQYNSTYRVVSVLTVLHQDWLNGKEYKCKVSNKALPAPIEKT ISKAKGQPREPQVYTLPPSREEMTKNQVSLTCLVKGFYPSDIAVE WESNGQPENNYKTTPPVLDSDGSFFLYSKLTVDKSRWQQGNVF SCSVMHEALHNHYTQKSLSLSPG | 77 |
| 16H6-H4L1 | heavy chain | EVQLVQSGAEVKKPGASVKVSCKASGYTFTSYWITWVRQAPG QRLEWIGNIFPGSGSTNYNEKFKGRVTLTVDTSSSTAYMELSSLR SEDTAVYYCSEGDYSFAYWGQGTLVTVSSASTKGPSVFPLAPSS KSTSGGTAALGCLVKDYFPEPVTVSWNSGALTSGVHTFPAVLQS SGLYSLSSVVTVPSSSLGTQTYICNVNHKPSNTKVDKKVEPKSC DKTHTCPPCPAPEAAGAPSVFLFPPKPKDTLMISRTPEVTCVVV DVSHEDPEVKFNWYVDGVEVHNAKTKPREEQYNSTYRVVSVL TVLHQDWLNGKEYKCKVSNKALPAPIEKTISKAKGQPREPQVY TLPPSREEMTKNQVSLTCLVKGFYPSDIAVEWESNGQPENNYKT TPPVLDSDGSFFLYSKLTVDKSRWQQGNVFSCSVMHEALHNHY TQKSLSLSPG | 78 |
| | light chain | DIQMTQSPSSLSASVGDRVTITCRASQDINKYLNWYQQKPGGT VKLLIYSTSRLHSGVPSRFSGSGSGTDYSLTISNLQPEDIATYYCQ QGSMLPWTFGGGTKVEIKRTVAAPSVFIFPPSDEQLKSGTASVV CLLNNFYPREAKVQWKVDNALQSGNSQESVTEQDSKDSTYSLS STLTLSKADYEKHKVYACEVTHQGLSSPVTKSFNRGEC | 79 |
| 16H6-H4L3 | heavy chain | identical to the heavy chain of 16H6-H4L1 | 78 |
| | light chain | DIQMTQSPSSLSASVGDRVTITCRASQDINKYLNWYQQKPGGT VKLLLYSTSRLHSGVPSRFSGSGSGTDYSLTISSLQPEDFATYYC QQGSMLPWTFGGGTKVEIKRTVAAPSVFIFPPSDEQLKSGTASV VCLLNNFYPREAKVQWKVDNALQSGNSQESVTEQDSKDSTYS LSSTLTLSKADYEKHKVYACEVTHQGLSSPVTKSFNRGEC | 80 |
| 12B3-H8L1 | heavy chain | EVQLVQSGAEVKKPGASVKVSCKASGYPFTDFYMNWVRQAH GKSLEWIGEVNPDNGDTSHNQEFEDRVTLTVDKSARTAYMELS SLRSEDTAVYYCARERVGYWFAYWGQGTLVTVSSASTKGPSVF PLAPSSKSTSGGTAALGCLVKDYFPEPVTVSWNSGALTSGVHTF PAVLQSSGLYSLSSVVTVPSSSLGTQTYICNVNHKPSNTKVDKK VEPKSCDKTHTCPPCPAPEAAGAPSVFLFPPKPKDTLMISRTPEV TCVVVDVSHEDPEVKFNWYVDGVEVHNAKTKPREEQYNSTYR VVSVLTVLHQDWLNGKEYKCKVSNKALPAPIEKTISKAKGQPR EPQVYTLPPSREEMTKNQVSLTCLVKGFYPSDIAVEWESNGQPE NNYKTTPPVLDSDGSFFLYSKLTVDKSRWQQGNVFSCSVMHEA LHNHYTQKSLSLSPG | 81 |
| | light chain | DIVMTQSPKFVSASVGDRVTITCRASQNVGSAVAWYQQKPGQS PKILIYSASNRYSGVPSRFSGSGSGTDFTLTISSLQPEDFADYFCQ QYSSFPLTFGQGTKLEIKRTVAAPSVFIFPPSDEQLKSGTASVVCL LNNFYPREAKVQWKVDNALQSGNSQESVTEQDSKDSTYSLSST LTLSKADYEKHKVYACEVTHQGLSSPVTKSFNRGEC | 82 |
| 12B3-H7L1 | heavy chain | EVQLVQSGAEVKKPGASVKVSCKASGYPFTDFYMNWVRQAH GKSLEWIGEVNPDNGDTSHNQEFEDRVTLTVDKSTRTAYMELSS LRSEDTAVYYCARERVGYWFAYWGQGTLVTVSSASTKGPSVFP LAPSSKSTSGGTAALGCLVKDYFPEPVTVSWNSGALTSGVHTFP AVLQSSGLYSLSSVVTVPSSSLGTQTYICNVNHKPSNTKVDKKV EPKSCDKTHTCPPCPAPEAAGAPSVFLFPPKPKDTLMISRTPEVT CVVVDVSHEDPEVKFNWYVDGVEVHNAKTKPREEQYNSTYR VVSVLTVLHQDWLNGKEYKCKVSNKALPAPIEKTISKAKGQPR EPQVYTLPPSREEMTKNQVSLTCLVKGFYPSDIAVEWESNGQPE NNYKTTPPVLDSDGSFFLYSKLTVDKSRWQQGNVFSCSVMHEA LHNHYTQKSLSLSPG | 83 |
| | light chain | identical to the light chain of 12B3-H8L1 | 82 |
| 12B3-H8L8 | heavy chain | identical to the heavy chain of 12B3-H8L1 | 81 |
| | light chain | DIQMTQSPSFLSTSVGDRVTITCKASQNVGSAVAWYQQKPGQSP KILIYSASNRYIGVPSRFSGSGSGTEFTLTISSLLPEDFADYFCQQY SSFPLTFGQGTKLEIKRTVAAPSVFIFPPSDEQLKSGTASVVCLLN NFYPREAKVQWKVDNALQSGNSQESVTEQDSKDSTYSLSSTLT LSKADYEKHKVYACEVTHQGLSSPVTKSFNRGEC | 84 |

### Example 8: Activity Detection of Humanized Antibodies by Reporter Gene Assay

The activity of the above humanized antibodies against human TL1A trimer was detected by reporter gene assay according to the procedure described in Example 1. Table 6 and Figures 7-1 to 7-14 (graphs showing the activity of humanized antibodies against human TL1A trimer detected by reporter gene assay) show the activity detection results of the humanized antibodies against human TL1A trimer by reporter gene assay. It can be seen that all humanized antibodies of the present application could block the fluorescence signal of the TL1A trimer-induced reporter gene cell line in a dose-dependent manner. From the curves and IC₅₀ values, the activity of the humanized antibodies of the present application was superior to or comparable to that of the positive control antibody 1D1.

**Table 6: Activity detection results of humanized antibodies against human TL1A trimer by reporter gene assay**

| Humanized antibody number | IC₅₀ (ng/mL) | | Fold activity relative to 1D1 |
|---|---|---|---|
| | Humanized antibody | 1D1 | |
| 16H6-H1L1 | 1206 | 1724 | 143% |
| 16H6-H1L2 | 1202 | 1724 | 143% |
| 16H6-H1L3 | 1208 | 1724 | 143% |
| 16H6-H1L4 | 1152 | 1724 | 150% |
| 16H6-H2L1 | 1191 | 1721 | 145% |
| 16H6-H2L2 | 1225 | 1721 | 140% |
| 16H6-H2L3 | 1162 | 1721 | 148% |
| 16H6-H2L4 | 1189 | 1721 | 145% |
| 16H6-H3L1 | 1253 | 1838 | 147% |
| 16H6-H3L2 | 1390 | 1838 | 132% |
| 16H6-H3L3 | 1224 | 1838 | 150% |
| 16H6-H3L4 | 1372 | 1838 | 134% |
| 16H6-H4L1 | 1101 | 1937 | 176% |
| 16H6-H4L2 | 1180 | 1937 | 164% |
| 16H6-H4L3 | 1168 | 1937 | 166% |
| 16H6-H4L4 | 1206 | 1937 | 161% |
| 12B3-H1L1 | 1454 | 1494 | 103% |
| 12B3-H1L2 | 1412 | 1494 | 106% |
| 12B3-H1L4 | 1478 | 1494 | 101% |
| 12B3-H1L5 | 1454 | 1494 | 103% |
| 12B3-H1L6 | 1362 | 1657 | 122% |
| 12B3-H1L8 | 1171 | 1680 | 143% |
| 12B3-H2L1 | 1393 | 1551 | 111% |
| 12B3-H2L2 | 1406 | 1551 | 110% |
| 12B3-H2L4 | 1423 | 1551 | 109% |
| 12B3-H2L5 | 1449 | 1551 | 107% |
| 12B3-H3L1 | 1407 | 1575 | 112% |
| 12B3-H3L2 | 1457 | 1575 | 108% |
| 12B3-H3L4 | 1490 | 1575 | 106% |
| 12B3-H3L5 | 1446 | 1575 | 109% |
| 12B3-H3L6 | 1316 | 1545 | 117% |
| 12B3-H3L8 | 1313 | 1623 | 124% |
| 12B3-H4L1 | 1413 | 1551 | 110% |
| 12B3-H4L2 | 1349 | 1551 | 115% |
| 12B3-H4L4 | 1412 | 1551 | 110% |
| 12B3-H4L5 | 1403 | 1551 | 111% |
| 12B3-H4L6 | 1336 | 1611 | 121% |
| 12B3-H4L8 | 1317 | 1531 | 116% |
| 12B3-H6L1 | 1325 | 1657 | 125% |
| 12B3-H6L4 | 1364 | 1680 | 123% |
| 12B3-H6L6 | 1350 | 1657 | 123% |
| 12B3-H6L8 | 1337 | 1680 | 126% |
| 12B3-H7L1 | 1313 | 1545 | 118% |
| 12B3-H7L4 | 1304 | 1623 | 124% |
| 12B3-H7L6 | 1317 | 1545 | 117% |
| 12B3-H7L8 | 1308 | 1623 | 124% |
| 12B3-H8L1 | 1243 | 1611 | 130% |
| 12B3-H8L6 | 1318 | 1611 | 122% |
| 12B3-H8L8 | 1300 | 1531 | 118% |

### Example 9: Activity Detection of Humanized Antibodies by Caspase Assay

The activity of the humanized antibodies against human TL1A trimer was detected by caspase assay according to the procedure described in Example 5. Table 7 and Figures 8-1 to 8-4 (graphs showing the activity of humanized antibodies against human TL1A trimer detected by caspase assay) show the activity detection results of the humanized antibodies against human TL1A trimer by caspase assay. It can be seen that all humanized antibodies of the present application could block TL1A trimer-induced caspase activation in a dose-dependent manner. From the curves and IC₅₀ values, the activity of the humanized antibodies of the present application was superior to or comparable to that of the positive control antibody 1D1.

**Table 7: Activity detection results of humanized antibodies against human TL1A trimer by caspase assay**

| Humanized antibody number | IC₅₀ (ng/mL) | | Fold activity relative to 1D1 |
|---|---|---|---|
| | Humanized antibody | 1D1 | |
| 16H6-H3L1 | 182.1 | 156.8 | 86% |
| 16H6-H4L3 | 128.9 | 156.8 | 122% |
| 16H6-H4L1 | 93.24 | 100.7 | 108% |
| 16H6-H3L3 | 155.5 | 160.2 | 103% |
| 12B3-H1L8 | 105.6 | 156.8 | 148% |
| 12B3-H7L1 | 128.3 | 156.8 | 122% |
| 12B3-H8L1 | 130.1 | 156.8 | 121% |
| 12B3-H8L8 | 97.66 | 156.8 | 161% |

### Example 10: Activity Detection of Humanized Antibodies by Cytokine Assay

The activity of the humanized antibodies of the present application was detected by cytokine assay according to the following steps, in order to evaluate the blocking effect of the candidate antibodies on the release of IFN-γ from human primary PBMC cells co-induced by TL1A trimer, IL-12 and IL-18.
(1) Human IL-12 (purchased from Sino Biological) was diluted to 200 pg/mL with RPMI 1640 + 10% FBS medium, and 20 µL of the diluted solution was added per well to a 96-well clear flat-bottom plate.
(2) Human IL-18 (purchased from Sino Biological) was diluted to 270 ng/mL with RPMI 1640 + 10% FBS medium, and 20 µL of the diluted solution was added per well to the above 96-well clear flat-bottom plate.
(3) Human TL1A trimer (purchased from KACTUS) was diluted to 250 ng/mL with RPMI 1640 + 10% FBS medium, and 20 µL of the diluted solution was added per well to the above 96-well clear flat-bottom plate.
(4) Cryopreserved human PBMCs (purchased from Milecell Bio) were quickly thawed in a 37°C water bath, and then resuspended in 6 mL of RPMI 1640 + 10% FBS medium, followed by centrifugation at 400g for 8 minutes. The supernatant was discarded, and the cells were resuspended in RPMI 1640 + 10% FBS to a density of 5×10⁵ cells/mL. Then, 20 µL of the cell suspension was aspirated and added to the above 96-well clear flat-bottom plate, with 1×10⁴ cells per well. The 96-well clear flat-bottom plate was incubated at 37°C with 5% CO₂ for 1 hour.
(5) The positive control antibody 1D1 and the humanized antibodies were subjected to 2-fold serial dilutions starting from a maximum concentration of 2.5 µg/mL using RPMI 1640 + 10% FBS medium, for a total of 6 dilution gradients. The antibody concentrations were 2.5 µg/mL, 1.25 µg/mL, 0.625 µg/mL, 0.312 µg/mL, 0.156 µg/mL, and 0.0780 µg/mL, respectively. Then, 20 µL of each diluted antibody was added to the 96-well clear flat-bottom plate, with 4 replicate wells per concentration gradient. The plate was cultured at 37°C with 5% CO₂ for 72 hours.
(6) After the culture, the plate was centrifuged, and the culture supernatant was collected. The supernatant was diluted with an appropriate multiple using RPMI 1640 + 10% FBS medium. The human IFN-γ content in the culture supernatant was measured using a Human IFN-γ ELISA Kit (purchased from MULTI SCIENCES) following the manufacturer's instructions.

Table 8 and Figures 9-1 to 9-2 (graphs showing the activity of humanized antibodies against human TL1A trimer detected by cytokine assay) show the activity detection results of the humanized antibodies against human TL1A trimer by cytokine assay. It can be seen that all humanized antibodies of the present application could block TL1A trimer-induced IFN-γ release from human primary PBMC cells in a dose-dependent manner. From the curves and IC₅₀ values, the activity of the humanized antibodies of the present application was superior to or comparable to that of the positive control antibody 1D1.

**Table 8: Activity detection results of humanized antibodies against human TL1A trimer by cytokine assay**

| Humanized antibody number | IC₅₀ (ng/mL) | | Fold activity relative to 1D1 |
|---|---|---|---|
| | Humanized antibody | 1D1 | |
| 16H6-H4L1 | 95.39 | 164.1 | 172% |
| 16H6-H4L3 | 119.8 | 164.1 | 137% |
| 12B3-H8L1 | 125.0 | 164.1 | 131% |
| 12B3-H8L8 | 101.0 | 119.5 | 118% |
| 12B3-H7L1 | 104.2 | 119.5 | 110% |

### Example 11: In Vivo Efficacy of Humanized Antibodies in Rats

The in vivo efficacy of the humanized antibodies 16H6-H4L1 and 16H6-H4L3 of the present application, as well as the positive control antibody 1D1, was evaluated in a rat colitis model. Forty Sprague-Dawley rats (half male and half female) were divided into 5 groups as shown in Table 9. An acute colitis model was established by intrarectal administration of 2,4,6-trinitrobenzenesulfonic acid (TNBS) dissolved in ethanol via a catheter. The blank control group received only an equal volume of ethanol intrarectally. According to the protocol shown in Table 9, the animals were administered once on the day of model establishment (D0) after modeling, and once more on day 3 after modeling (D3). The blank control group and the model control group were administered only an equal volume of normal saline. The endpoint of the experiment was 7 days after modeling (D7). During this period, the disease activity index (DAI) was scored daily based on body weight, stool consistency, and the presence of blood in the stool. At the endpoint (D7), the animals were euthanized, and the colons were dissected and collected. The colon weight and length were measured. Macroscopic scoring of colon injury was performed by visual observation. Colon specimens were fixed in formalin, embedded in paraffin, sectioned, and subjected to colon pathological scoring by HE staining.

Figures 10-1 to 10-4 (graphs showing the in vivo efficacy results of humanized antibodies in rats) show the efficacy of the humanized antibodies in TNBS-induced rat colitis. It can be seen that compared with the model control group, the humanized antibodies 16H6-H4L1 and 16H6-H4L3 of the present application significantly reduced the colon weight/length/body weight ratio and significantly improved the colon macroscopic score. Furthermore, 16H6-H4L1 also significantly improved the colon pathological score, and was superior to the positive control antibody 1D1 (Statistical analysis of differences between groups was performed using t-test. *: P<0.05 compared to model control; **: P<0.01 compared to model control; ***: P<0.001 compared to model control).

**Table 9: Study protocol for in vivo efficacy of humanized antibodies in rats**

| Group | Number of animals | Drug | Dose (mg/kg) | Route of administration | Frequency of administration |
|---|---|---|---|---|---|
| Blank control group | 8 animals ( (half male and half female) | Saline | / | i.v. | D0, D3 |
| Model control group | 8 animals (half male and half female) | Saline | / | i.v. | D0, D3 |
| Positive control group | 8 animals (half male and half female) | 1D1 | 50 | i.v. | D0, D3 |
| Candidate molecule group 1 | 8 animals (half male and half female) | 16H6-H4L1 | 50 | i.v. | D0, D3 |
| Candidate molecule group 2 | 8 animals (half male and half female) | 16H6-H4L3 | 50 | i.v. | D0, D3 |

The sequences of the present application are listed in Table 10 below.

**Table 10: Sequences of the present application**

| SEQ ID NO. | Antibody region | Sequence |
|---|---|---|
| 1 | 16H6 HCDR1 | GYTFTSYW |
| 2 | 16H6 HCDR2 | IFPGSGST |
| 3 | 16H6 HCDR3 | SEGDYSFAY |
| 4 | 16H6 LCDR1 | QDINKY |
| 5 | 16H6 LCDR2 | STS |
| 6 | 16H6 LCDR3 | QQGSMLPWT |
| 7 | 12B3 HCDR1 | GYPFTDFY |
| 8 | 12B3 HCDR2 | VNPDNGDT |
| 9 | 12B3 HCDR3 | ARERVGYWFAY |
| 10 | 12B3 LCDR1 | QNVGSA |
| 11 | 12B3 LCDR2 | SAS |
| 12 | 12B3 LCDR3 | QQYSSFPLT |
| 13 | 56C8 HCDR1 | GFNIKDDY |
| 14 | 56C8 HCDR2 | IDPANGNT |
| 15 | 56C8 HCDR3 | ASGRNYGTYWHFDV |
| 16 | 56C8 LCDR1 | QDINRY |
| 17 | 56C8 LCDR2 | RAN |
| 18 | 56C8 LCDR3 | LQYDEFPYT |
| 19 | 5C6 HCDR1 | GYSFTGYT |
| 20 | 5C6, 28G7 HCDR2 | INPYSGGT |
| 21 | 5C6 HCDR3 | ARISYRYEAMDC |
| 22 | 5C6, 28G7 LCDR1 | SSVSY |
| 23 | 5C6, 28G7 LCDR2 | DTS |
| 24 | 5C6 LCDR3 | QQWSSDPYT |
| 25 | 28G7 HCDR1 | SYSFTGYT |
| 26 | 28G7 HCDR3 | ARIGGRVDAMDY |
| 27 | 28G7 LCDR3 | QQWSSNPYT |
| 28 | 70A3 HCDR1 | GYSITSDY |
| 29 | 70A3 HCDR2 | IYYNGGT |
| 30 | 70A3 HCDR3 | AKAGSSYVGLDY |
| 31 | 70A3 LCDR1 | QDISNY |
| 32 | 70A3 LCDR2 | YTS |
| 33 | 70A3 LCDR3 | QQGRTLPWT |
| 34 | 51G9 HCDR1 | GYSITSGYY |
| 35 | 51G9 HCDR2 | ISSDGRS |
| 36 | 51G9 HCDR3 | VRGGLRWDFES |
| 37 | 51G9 LCDR1 | KSVSTSGYNY |
| 38 | 51G9 LCDR2 | LAS |
| 39 | 51G9 LCDR3 | QHSRELPPT |
| 40 | 69B1 HCDR1 | GYTFTGYW |
| 41 | 69B1 HCDR2 | ILPGDDSA |
| 42 | 69B1 HCDR3 | ARRAYYPYDAMDY |
| 43 | 69B1 LCDR1 | QSIVHRNTYTY |
| 44 | 69B1 LCDR2 | GVS |
| 45 | 69B1 LCDR3 | FQSTHVPYT |
| 46 | 13B8 HCDR1 | GFSLTDYA |
| 47 | 13B8 HCDR2 | IYRSGST |
| 48 | 13B8 HCDR3 | AKNGDGFSWYFDV |
| 49 | 13B8 LCDR2 | GNIHTY |
| 50 | 13B8 LCDR2 | NAK |
| 51 | 13B8 LCDR3 | QHFWGNPPT |
| 52 | 1D1 heavy chain | QVQLVQSGAEVKKPGASVKVSCKASGYDFTYYGISWVRQAPG |
| | | QGLEWMGWISTYNGNTHYARMLQGRVTMTTDTSTRTAYMEL RSLRSDDTAVYYCARENYYGSGAYRGGMDVWGQGTTVTVSS ASTKGPSVFPLAPSSKSTSGGTAALGCLVKDYFPEPVTVSWNSG ALTSGVHTFPAVLQSSGLYSLSSVVTVPSSSLGTQTYICNVNHK PSNTKVDKKVEPKSCDKTHTCPPCPAPEAAGAPSVFLFPPKPKD TLMISRTPEVTCVVVDVSHEDPEVKFNWYVDGVEVHNAKTKP REEQYNSTYRVVSVLTVLHQDWLNGKEYKCKVSNKALPAPIE KTISKAKGQPREPQVYTLPPSREEMTKNQVSLTCLVKGFYPSDI AVEWESNGQPENNYKTTPPVLDSDGSFFLYSKLTVDKSRWQQ GNVFSCSVMHEALHNHYTQKSLSLSPG |
| 53 | 1D1 light chain | EIVLTQSPATLSLSPGERATLSCRASQSVSSYLAWYQQKPGQAP RLLIYDASNRATGIPARFSGSGSGTDFTLTISSLEPEDFAVYYCQ QRSNWPWTFGQGTKVEIKRTVAAPSVFIFPPSDEQLKSGTASVV CLLNNFYPREAKVQWKVDNALQSGNSQESVTEQDSKDSTYSLS STLTLSKADYEKHKVYACEVTHQGLSSPVTKSFNRGEC |
| 54 | heavy chain variable regions of 16H6-H1L1, 16H6-H1L2, 16H6-H1L3, and 16H6-H1L4 | EVQLVQSVAEVKKPGASVKVSCKASGYTFTSYWITWVRQAPG QGLEWIGNIFPGSGSTNYNEKFKGRVTLTVDTSSSTAYMELSSLR SEDTAVYYCSEGDYSFAYWGQGTLVTVSS |
| 55 | light chain variable regions of 16H6-H1L1, 16H6-H2L1, 16H6-H3L1, and 16H6-H4L1 | DIQMTQSPSSLSASVGDRVTITCRASQDINKYLNWYQQKPGGT VKLLIYSTSRLHSGVPSRFSGSGSGTDYSLTISNLQPEDIATYYCQ QGSMLPWTFGGGTKVEIK |
| 56 | light chain variable regions of 16H6-H1L2, 16H6-H2L2, 16H6-H3L2, and16H6-H4L2 | DIQMTQSPSSLSASVGDRVTITCRASQDINKYLNWYQQKPGGT VKLLIYSTSRLHSGVPSRFSGSGSGTDYSLTISSLQPEDFATYYCQ QGSMLPWTFGGGTKVEIK |
| 57 | light chain variable regions of 16H6-H1L3, 16H6-H2L3, 16H6-H3L3, and 16H6-H4L3 | DIQMTQSPSSLSASVGDRVTITCRASQDINKYLNWYQQKPGGT VKLLLYSTSRLHSGVPSRFSGSGSGTDYSLTISSLQPEDFATYYC QQGSMLPWTFGGGTKVEIK |
| 58 | light chain variable regions of 16H6-H1L4, 16H6-H2L4, 16H6-H3L4, and 16H6-H4L4 | DIQMTQSPSSLSASVGDRVTITCRASQDINKYLNWYQQKPGGT VKLLIYSTSRLHSGVPSRFSGSGSGTDYSLTISSLQPEDVATYYCQ QGSMLPWTFGGGTKVEIK |
| 59 | heavy chain variable regions of 16H6-H2L1, 16H6-H2L2, 16H6-H2L3, and 16H6-H2L4 | EVQLVQSVAEFKKPGASVKMSCKASGYTFTSYWITWVRQAPG QGLEWIGNIFPGSGSTNYNEKFKGRVTLTVDTSSSTAYMELSSLR SEDTAVYYCSEGDYSFAYWGQGTLVTVSS |
| 60 | heavy chain variable regions of 16H6-H3L1, 16H6-H3L2, 16H6-H3L3, and 16H6-H3L4 | EVQLVQSGAEVKKPGASVKVSCKASGYTFTSYWITWVRQAPG QGLEWIGNIFPGSGSTNYNEKFKGRVTLTVDTSSSTAYMELSRL RSEDTAVYYCSEGDYSFAY WGQGTLVTVSS |
| 61 | heavy chain variable regions of 16H6-H4L1, 16H6-H4L2, 16H6-H4L3, and 16H6-H4L4 | EVQLVQSGAEVKKPGASVKVSCKASGYTFTSYWITWVRQAPG QRLEWIGNIFPGSGSTNYNEKFKGRVTLTVDTSSSTAYMELSSLR SEDTAVYYCSEGDYSFAY WGQGTLVTVSS |
| 62 | heavy chain variable regions of 12B3-H1L1, 12B3-H1L2, 12B3-H1L4, 12B3-H1L5, 12B3-H1L6, and 12B3-H1L8 | EVQLVQSGAEVKKPGASVKVSCKASGYPFTDFYMNWVRQAH GKSLEWIGEVNPDNGDTSHNQKFQGRVTLTVDKSTRTAYMELS SLRSEDTAVYYCARERVGYWFAYWGQGTLVTVSS |
| 63 | light chain variable regions of 12B3-H1L1, 12B3-H2L1, 12B3-H3L1, 12B3-H4L1, 12B3-H6L1, 12B3-H7L1, and 12B3-H8L1 | DIVMTQSPKFVSASVGDRVTITCRASQNVGSAVAWYQQKPGQS PKILIYSASNRYSGVPSRFSGSGSGTDFTLTISSLQPEDFADYFCQ QYSSFPLTFGQGTKLEIK |
| 64 | light chain variable regions of 12B3-H1L2, 12B3-H2L2, 12B3-H3L2, and 12B3-H4L2 | DIVMTQSPKFLSTSTGDRVTITCRASQNVGSAVAWYQQKPGKAP KILIYSASNRYSGVPSRFSGSGSGTDFTLTINNLLSEDFADYFCQQ YSSFPLTFGQGTKLEIK |
| 65 | light chain variable regions of 12B3-H1L4, 12B3-H2L4, 12B3-H3L4, 12B3-H4L4, 12B3-H6L4, and 12B3-H7L4 | DIQMTQSPSFLSASVGDRVTITCRASQNVGSAVAWYQQKPGQSP KILIYSASNRYSGVPSRFSGSGSGTEFTLTISSLLPEDFADYFCQQ YSSFPLTFGQGTKLEIK |
| 66 | light chain variable regions of 12B3-H1L5, 12B3-H2L5, 12B3-H3L5, and 12B3-H4L5 | DIVMTQSPKFLSTSTGDRVTITCKASQNVGSAVAWYQQRPGQSP KILIYSASNRYIGVPSRFSGSGSGTDFTLTINNLLSEDFADYFCQQ YSSFPLTFGQGTKLEIK |
| 67 | light chain variable regions of 12B3-H1L6, 12B3-H3L6, 12B3-H4L6, 12B3-H6L6, 12B3-H7L6, and 12B3-H8L6 | DIVMTQSPKFVSTSVGDRVTITCKASQNVGSAVAWYQQKPGQS PKILIYSASNRYIGVPSRFSGSGSGTDFTLTISSLLPEDFADYFCQQ YSSFPLTFGQGTKLEIK |
| 68 | light chain variable regions of 12B3-H1L8, 12B3-H3L8, 12B3-H4L8, 12B3-H6L8, 12B3-H7L8, and 12B3-H8L8 | DIQMTQSPSFLSTSVGDRVTITCKASQNVGSAVAWYQQKPGQSP KILIYSASNRYIGVPSRFSGSGSGTEFTLTISSLLPEDFADYFCQQY SSFPLTFGQGTKLEIK |
| 69 | heavy chain variable regions of 12B3-H2L1, 12B3-H2L2, 12B3-H2L4, and 12B3-H2L5 | EVQLVQSGAEVKKPGASVKVSCKASGYPFTDFYMNWVRQAH GKSLEWIGEVNPDNGDTSHNQEFQGRVTLTVDKSARTAYMELS SLRSEDMAVYYCARERVGYWFAYWGQGTLVTVSS |
| 70 | heavy chain variable regions of 12B3-H3L1, 12B3-H3L2, 12B3-H3L4, 12B3-H3L5, 12B3-H3L6, and 12B3-H3L8 | EVQLVQSGAEVKKPGASVKVSCKASGYPFTDFYMNWVRQAH GKSLEWMGEVNPDNGDTSHNQKFQGRVTLTVDKSTRTAYMEL SSLRSEDTAVYYCARERVGYWFAYWGQGTLVTVSS |
| 71 | heavy chain variable regions of 12B3-H4L1, 12B3-H4L2, 12B3-H4L4, 12B3-H4L5, 12B3-H4L6, and 12B3-H4L8 | EVQLVQSGAEVKKPGASVKVSCKASGYPFTDFYMNWVRQAH GKSLEWMGEVNPDNGDTSHNQKFQGRVTLTVDKSARTAYMEL SSLRSEDTAVYYCARERVGYWFAYWGQGTLVTVSS |
| 72 | heavy chain variable regions of 12B3-H6L1, 12B3-H6L4, 12B3-H6L6, and 12B3-H6L8 | EVQLVQSGAEVKKPGASVKVSCKASGYPFTDFYMNWVKQAH GKSLEWIGEVNPDNGDTSHNQEFEDRVTLTVDKSSRTAYMELSS LRSEDTAVYYCARERVGYWFAYWGQGTLVTVSS |
| 73 | heavy chain variable regions of 12B3-H7L1, 12B3-H7L4, 12B3-H7L6, and 12B3-H7L8 | EVQLVQSGAEVKKPGASVKVSCKASGYPFTDFYMNWVRQAH GKSLEWIGEVNPDNGDTSHNQEFEDRVTLTVDKSTRTAYMELSS LRSEDTAVYYCARERVGYWFAYWGQGTLVTVSS |
| 74 | heavy chain variable regions of 12B3-H8L1, 12B3-H8L6, and 12B3-H8L8 | EVQLVQSGAEVKKPGASVKVSCKASGYPFTDFYMNWVRQAH GKSLEWIGEVNPDNGDTSHNQEFEDRVTLTVDKSARTAYMELS SLRSEDTAVYYCARERVGYWFAYWGQGTLVTVSS |
| 75 | Candidate antibody heavy chain constant region | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKKVEPKSCDKTHTCPPCPAPEAAGAPSVFLFPPKPKDT LMISRTPEVTCVVVDVSHEDPEVKFNWYVDGVEVHNAKTKPR EEQYNSTYRVVSVLTVLHQDWLNGKEYKCKVSNKALPAPIEKT ISKAKGQPREPQVYTLPPSREEMTKNQVSLTCLVKGFYPSDIAVE WESNGQPENNYKTTPPVLDSDGSFFLYSKLTVDKSRWQQGNVF SCSVMHEALHNHYTQKSLSLSPG |
| 76 | Candidate antibody light chain constant region | |
| 77 | Wild-type heavy chain constant region (C-terminal Lys removed) | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKKVEPKSCDKTHTCPPCPAPELLGGPSVFLFPPKPKDT LMISRTPEVTCVVVDVSHEDPEVKFNWYVDGVEVHNAKTKPR EEQYNSTYRVVSVLTVLHQDWLNGKEYKCKVSNKALPAPIEKT ISKAKGQPREPQVYTLPPSREEMTKNQVSLTCLVKGFYPSDIAVE WESNGQPENNYKTTPPVLDSDGSFFLYSKLTVDKSRWQQGNVF SCSVMHEALHNHYTQKSLSLSPG |
| 78 | heavy chains of 16H6-H4L1, 16H6-H4L2, 16H6-H4L3, and 16H6-H4L4 | EVQLVQSGAEVKKPGASVKVSCKASGYTFTSYWITWVRQAPG QRLEWIGNIFPGSGSTNYNEKFKGRVTLTVDTSSSTAYMELSSLR SEDTAVYYCSEGDYSFAYWGQGTLVTVSSASTKGPSVFPLAPSS KSTSGGTAALGCLVKDYFPEPVTVSWNSGALTSGVHTFPAVLQS SGLYSLSSVVTVPSSSLGTQTYICNVNHKPSNTKVDKKVEPKSC DKTHTCPPCPAPEAAGAPSVFLFPPKPKDTLMISRTPEVTCVVV DVSHEDPEVKFNWYVDGVEVHNAKTKPREEQYNSTYRVVSVL TVLHQDWLNGKEYKCKVSNKALPAPIEKTISKAKGQPREPQVY TLPPSREEMTKNQVSLTCLVKGFYPSDIAVEWESNGQPENNYKT TPPVLDSDGSFFLYSKLTVDKSRWQQGNVFSCSVMHEALHNHY TQKSLSLSPG |
| 79 | light chains of 16H6-H4L1, 16H6-H1L1, 16H6-H2L1, and 16H6-H3L1 | DIQMTQSPSSLSASVGDRVTITCRASQDINKYLNWYQQKPGGT VKLLIYSTSRLHSGVPSRFSGSGSGTDYSLTISNLQPEDIATYYCQ QGSMLPWTFGGGTKVEIKRTVAAPSVFIFPPSDEQLKSGTASVV CLLNNFYPREAKVQWKVDNALQSGNSQESVTEQDSKDSTYSLS STLTLSKADYEKHKVYACEVTHQGLSSPVTKSFNRGEC |
| 80 | light chains of 16H6-H4L3, 16H6-H1L3, 16H6-H2L3, and 16H6-H3L3 | DIQMTQSPSSLSASVGDRVTITCRASQDINKYLNWYQQKPGGT VKLLLYSTSRLHSGVPSRFSGSGSGTDYSLTISSLQPEDFATYYC QQGSMLPWTFGGGTKVEIKRTVAAPSVFIFPPSDEQLKSGTASV |
| | | VCLLNNFYPREAKVQWKVDNALQSGNSQESVTEQDSKDSTYS LSSTLTLSKΛDYEKHKVYΛCEVTHQGLSSPVTKSFNRGEC |
| 81 | heavy chains of 12B3-H8L1, 12B3-H8L6, and 12B3-H8L8 | EVQLVQSGAEVKKPGASVKVSCKASGYPFTDFYMNWVRQAH GKSLEWIGEVNPDNGDTSHNQEFEDRVTLTVDKSARTAYMELS SLRSEDTAVYYCARERVGYWFAYWGQGTLVTVSSASTKGPSVF PLAPSSKSTSGGTAALGCLVKDYFPEPVTVSWNSGALTSGVHTF PAVLQSSGLYSLSSVVTVPSSSLGTQTYICNVNHKPSNTKVDKK VEPKSCDKTHTCPPCPAPEAAGAPSVFLFPPKPKDTLMISRTPEV TCVVVDVSHEDPEVKFNWYVDGVEVHNAKTKPREEQYNSTYR VVSVLTVLHQDWLNGKEYKCKVSNKALPAPIEKTISKAKGQPR EPQVYTLPPSREEMTKNQVSLTCLVKGFYPSDIAVEWESNGQPE NNYKTTPPVLDSDGSFFLYSKLTVDKSRWQQGNVFSCSVMHEA LHNHYTQKSLSLSPG |
| 82 | light chains of 12B3-H8L1, 12B3-H1L1, 12B3-H2L1, 12B3-H3L1, 12B3-H4L1, 12B3-H6L1, and 12B3-H7L1 | DIVMTQSPKFVSASVGDRVTITCRASQNVGSAVAWYQQKPGQS PKILIYSASNRYSGVPSRFSGSGSGTDFTLTISSLQPEDFADYFCQ QYSSFPLTFGQGTKLEIKRTVAAPSVFIFPPSDEQLKSGTASVVCL LNNFYPREAKVQWKVDNALQSGNSQESVTEQDSKDSTYSLSST LTLSKADYEKHKVYACEVTHQGLSSPVTKSFNRGEC |
| 83 | heavy chains of 12B3-H7L1, 12B3-H7L4, 12B3-H7L6, and 12B3-H7L8 | EVQLVQSGAEVKKPGASVKVSCKASGYPFTDFYMNWVRQAH GKSLEWIGEVNPDNGDTSHNQEFEDRVTLTVDKSTRTAYMELSS LRSEDTAVYYCARERVGYWFAYWGQGTLVTVSSASTKGPSVFP LAPSSKSTSGGTAALGCLVKDYFPEPVTVSWNSGALTSGVHTFP AVLQSSGLYSLSSVVTVPSSSLGTQTYICNVNHKPSNTKVDKKV EPKSCDKTHTCPPCPAPEAAGAPSVFLFPPKPKDTLMISRTPEVT CVVVDVSHEDPEVKFNWYVDGVEVHNAKTKPREEQYNSTYRV VSVLTVLHQDWLNGKEYKCKVSNKALPAPIEKTISKAKGQPRE PQVYTLPPSREEMTKNQVSLTCLVKGFYPSDIAVEWESNGQPEN NYKTTPPVLDSDGSFFLYSKLTVDKSRWQQGNVFSCSVMHEAL HNHYTQKSLSLSPG |
| 84 | light chains of 12B3-H8L8, 12B3-H1L8, 12B3-H3L8, 12B3-H4L8, 12B3-H6L8, and 12B3-H7L8 | DIQMTQSPSFLSTSVGDRVTITCKASQNVGSAVAWYQQKPGQSP KILIYSASNRYIGVPSRFSGSGSGTEFTLTISSLLPEDFADYFCQQY SSFPLTFGQGTKLEIKRTVAAPSVFIFPPSDEQLKSGTASVVCLLN NFYPREAKVQWKVDNALQSGNSQESVTEQDSKDSTYSLSSTLT LSKADYEKHKVYACEVTHQGLSSPVTKSFNRGEC |

All patents, patent applications, and other publications are hereby expressly incorporated by reference into the present application for the purpose of description and disclosure. Such publications are provided solely because their disclosure predates the filing date of the present application. All statements regarding the dates of these documents or representations of the contents of these documents are based on the information available to the applicant and do not constitute any admission as to the correctness of the dates or contents of these documents. Furthermore, any reference to these publications in the present application shall not be construed as an acknowledgement that such publication forms part of the common general knowledge in the art in any country.

Those skilled in the art will recognize that the scope of the present application is not limited to the various specific embodiments and examples described above, but can be subjected to various modifications, substitutions, or recombinations without departing from the spirit of the present application, all of which fall within the protection scope of the present application.

## Claims

1. An anti-TL1A antibody or an antigen-binding fragment thereof, comprising:
(a) a heavy chain variable region comprising the following complementarity determining regions:
HCDR1, comprising the amino acid sequence set forth in SEQ ID NO: 1, 7, 13, 19, 25, 28, 34, 40 or 46, or an amino acid sequence having at least 80% identity to the amino acid sequence set forth in SEQ ID NO: 1, 7, 13, 19, 25, 28, 34, 40 or 46, or an amino acid sequence obtained by substitution, deletion or addition of one or more amino acids in the amino acid sequence set forth in SEQ ID NO: 1, 7, 13, 19, 25, 28, 34, 40 or 46 and having the same or similar function to the amino acid sequence set forth in SEQ ID NO: 1, 7, 13, 19, 25, 28, 34, 40 or 46;
HCDR2, comprising the amino acid sequence set forth in SEQ ID NO: 2, 8, 14, 20, 29, 35, 41 or 47, or an amino acid sequence having at least 80% identity to the amino acid sequence set forth in SEQ ID NO: 2, 8, 14, 20, 29, 35, 41 or 47, or an amino acid sequence obtained by substitution, deletion or addition of one or more amino acids in the amino acid sequence set forth in SEQ ID NO: 2, 8, 14, 20, 29, 35, 41 or 47 and having the same or similar function to the amino acid sequence set forth in SEQ ID NO: 2, 8, 14, 20, 29, 35, 41 or 47; and
HCDR3, comprising the amino acid sequence set forth in SEQ ID NO: 3, 9, 15, 21, 26, 30, 36, 42 or 48, or an amino acid sequence having at least 80% identity to the amino acid sequence set forth in SEQ ID NO: 3, 9, 15, 21, 26, 30, 36, 42 or 48, or an amino acid sequence obtained by substitution, deletion or addition of one or more amino acids in the amino acid sequence set forth in SEQ ID NO: 3, 9, 15, 21, 26, 30, 36, 42 or 48 and having the same or similar function to the amino acid sequence set forth in SEQ ID NO: 3, 9, 15, 21, 26, 30, 36, 42 or 48; and
(b) a light chain variable region comprising the following complementarity determining regions:
LCDR1, comprising the amino acid sequence set forth in SEQ ID NO: 4, 10, 16, 22, 31, 37, 43 or 49, or an amino acid sequence having at least 80% identity to the amino acid sequence set forth in SEQ ID NO: 4, 10, 16, 22, 31, 37, 43 or 49, or an amino acid sequence obtained by substitution, deletion or addition of one or more amino acids in the amino acid sequence set forth in SEQ ID NO: 4, 10, 16, 22, 31, 37, 43 or 49 and having the same or similar function to the amino acid sequence set forth in SEQ ID NO: 4, 10, 16, 22, 31, 37, 43 or 49;
LCDR2, comprising the amino acid sequence set forth in SEQ ID NO: 5, 11, 17, 23, 32, 38, 44 or 50, or an amino acid sequence having at least 80% identity to the amino acid sequence set forth in SEQ ID NO: 5, 11, 17, 23, 32, 38, 44 or 50, or an amino acid sequence obtained by substitution, deletion or addition of one or more amino acids in the amino acid sequence set forth in SEQ ID NO: 5, 11, 17, 23, 32, 38, 44 or 50 and having the same or similar function to the amino acid sequence set forth in SEQ ID NO: 5, 11, 17, 23, 32, 38, 44 or 50; and
LCDR3, comprising the amino acid sequence set forth in SEQ ID NO: 6, 12, 18, 24, 27, 33, 39, 45 or 51, or an amino acid sequence having at least 80% identity to the amino acid sequence set forth in SEQ ID NO: 6, 12, 18, 24, 27, 33, 39, 45 or 51, or an amino acid sequence obtained by substitution, deletion or addition of one or more amino acids in the amino acid sequence set forth in SEQ ID NO: 6, 12, 18, 24, 27, 33, 39, 45 or 51 and having the same or similar function to the amino acid sequence set forth in SEQ ID NO: 6, 12, 18, 24, 27, 33, 39, 45 or 51.

2. The anti-TL1A antibody or the antigen-binding fragment thereof according to claim 1, comprising:
(1) HCDR1, HCDR2, HCDR3, LCDR1, LCDR2 and LCDR3, wherein the HCDR1, HCDR2 and HCDR3 have the amino acid sequences set forth in SEQ ID NOs: 1, 2 and 3, respectively, and the LCDR1, LCDR2 and LCDR3 have the amino acid sequences set forth in SEQ ID NOs: 4, 5 and 6, respectively;
(2) HCDR1, HCDR2, HCDR3, LCDR1, LCDR2 and LCDR3, wherein the HCDR1, HCDR2 and HCDR3 have the amino acid sequences set forth in SEQ ID NOs: 7, 8 and 9, respectively, and the LCDR1, LCDR2 and LCDR3 have the amino acid sequences set forth in SEQ ID NOs: 10, 11 and 12, respectively;
(3) HCDR1, HCDR2, HCDR3, LCDR1, LCDR2 and LCDR3, wherein the HCDR1, HCDR2 and HCDR3 have the amino acid sequences set forth in SEQ ID NOs: 13, 14 and 15, respectively, and the LCDR1, LCDR2 and LCDR3 have the amino acid sequences set forth in SEQ ID NOs: 16, 17 and 18, respectively;
(4) HCDR1, HCDR2, HCDR3, LCDR1, LCDR2 and LCDR3, wherein the HCDR1, HCDR2 and HCDR3 have the amino acid sequences set forth in SEQ ID NOs: 19, 20 and 21, respectively, and the LCDR1, LCDR2 and LCDR3 have the amino acid sequences set forth in SEQ ID NOs: 22, 23 and 24, respectively;
(5) HCDR1, HCDR2, HCDR3, LCDR1, LCDR2 and LCDR3, wherein the HCDR1, HCDR2 and HCDR3 have the amino acid sequences set forth in SEQ ID NOs: 25, 20 and 26, respectively, and the LCDR1, LCDR2 and LCDR3 have the amino acid sequences set forth in SEQ ID NOs: 22, 23 and 27, respectively;
(6) HCDR1, HCDR2, HCDR3, LCDR1, LCDR2 and LCDR3, wherein the HCDR1, HCDR2 and HCDR3 have the amino acid sequences set forth in SEQ ID NOs: 28, 29 and 30, respectively, and the LCDR1, LCDR2 and LCDR3 have the amino acid sequences set forth in SEQ ID NOs: 31, 32 and 33, respectively;
(7) HCDR1, HCDR2, HCDR3, LCDR1, LCDR2 and LCDR3, wherein the HCDR1, HCDR2 and HCDR3 have the amino acid sequences set forth in SEQ ID NOs: 34, 35 and 36, respectively, and the LCDR1, LCDR2 and LCDR3 have the amino acid sequences set forth in SEQ ID NOs: 37, 38 and 39, respectively;
(8) HCDR1, HCDR2, HCDR3, LCDR1, LCDR2 and LCDR3, wherein the HCDR1, HCDR2 and HCDR3 have the amino acid sequences set forth in SEQ ID NOs: 40, 41 and 42, respectively, and the LCDR1, LCDR2 and LCDR3 have the amino acid sequences set forth in SEQ ID NOs: 43, 44 and 45, respectively; or
(9) HCDR1, HCDR2, HCDR3, LCDR1, LCDR2 and LCDR3, wherein the HCDR1, HCDR2 and HCDR3 have the amino acid sequences set forth in SEQ ID NOs: 46, 47 and 48, respectively, and the LCDR1, LCDR2 and LCDR3 have the amino acid sequences set forth in SEQ ID NOs: 49, 50 and 51, respectively;
preferably, the anti-TL1A antibody or the antigen-binding fragment thereof comprises:
(1) HCDR1, HCDR2, HCDR3, LCDR1, LCDR2 and LCDR3, wherein the HCDR1, HCDR2 and HCDR3 have the amino acid sequences set forth in SEQ ID NOs: 1, 2 and 3, respectively, and the LCDR1, LCDR2 and LCDR3 have the amino acid sequences set forth in SEQ ID NOs: 4, 5 and 6, respectively;
(2) HCDR1, HCDR2, HCDR3, LCDR1, LCDR2 and LCDR3, wherein the HCDR1, HCDR2 and HCDR3 have the amino acid sequences set forth in SEQ ID NOs: 7, 8 and 9, respectively, and the LCDR1, LCDR2 and LCDR3 have the amino acid sequences set forth in SEQ ID NOs: 10, 11 and 12, respectively;
(3) HCDR1, HCDR2, HCDR3, LCDR1, LCDR2 and LCDR3, wherein the HCDR1, HCDR2 and HCDR3 have the amino acid sequences set forth in SEQ ID NOs: 13, 14 and 15, respectively, and the LCDR1, LCDR2 and LCDR3 have the amino acid sequences set forth in SEQ ID NOs: 16, 17 and 18, respectively;
(4) HCDR1, HCDR2, HCDR3, LCDR1, LCDR2 and LCDR3, wherein the HCDR1, HCDR2 and HCDR3 have the amino acid sequences set forth in SEQ ID NOs: 40, 41 and 42, respectively, and the LCDR1, LCDR2 and LCDR3 have the amino acid sequences set forth in SEQ ID NOs: 43, 44 and 45, respectively; or
(5) HCDR1, HCDR2, HCDR3, LCDR1, LCDR2 and LCDR3, wherein the HCDR1, HCDR2 and HCDR3 have the amino acid sequences set forth in SEQ ID NOs: 46, 47 and 48, respectively, and the LCDR1, LCDR2 and LCDR3 have the amino acid sequences set forth in SEQ ID NOs: 49, 50 and 51, respectively; and
more preferably, the anti-TL1A antibody or the antigen-binding fragment thereof comprises:
(1) HCDR1, HCDR2, HCDR3, LCDR1, LCDR2 and LCDR3, wherein the HCDR1, HCDR2 and HCDR3 have the amino acid sequences set forth in SEQ ID NOs: 1, 2 and 3, respectively, and the LCDR1, LCDR2 and LCDR3 have the amino acid sequences set forth in SEQ ID NOs: 4, 5 and 6, respectively;
(2) HCDR1, HCDR2, HCDR3, LCDR1, LCDR2 and LCDR3, wherein the HCDR1, HCDR2 and HCDR3 have the amino acid sequences set forth in SEQ ID NOs: 7, 8 and 9, respectively, and the LCDR1, LCDR2 and LCDR3 have the amino acid sequences set forth in SEQ ID NOs: 10, 11 and 12, respectively; or
(3) HCDR1, HCDR2, HCDR3, LCDR1, LCDR2 and LCDR3, wherein the HCDR1, HCDR2 and HCDR3 have the amino acid sequences set forth in SEQ ID NOs: 13, 14 and 15, respectively, and the LCDR1, LCDR2 and LCDR3 have the amino acid sequences set forth in SEQ ID NOs: 16, 17 and 18, respectively.

3. The anti-TL1A antibody or the antigen-binding fragment thereof according to claim 1 or 2, wherein the anti-TL1A antibody or the antigen-binding fragment thereof comprises:
(1) a heavy chain variable region and a light chain variable region, wherein the heavy chain variable region comprises the amino acid sequence set forth in SEQ ID NO: 61 or an amino acid sequence having at least 80% identity to the amino acid sequence set forth in SEQ ID NO: 61, and the light chain variable region comprises the amino acid sequence set forth in SEQ ID NO: 55 or an amino acid sequence having at least 80% identity to the amino acid sequence set forth in SEQ ID NO: 55;
(2) a heavy chain variable region and a light chain variable region, wherein the heavy chain variable region comprises the amino acid sequence set forth in SEQ ID NO: 54 or an amino acid sequence having at least 80% identity to the amino acid sequence set forth in SEQ ID NO: 54, and the light chain variable region comprises the amino acid sequence set forth in SEQ ID NO: 55 or an amino acid sequence having at least 80% identity to the amino acid sequence set forth in SEQ ID NO: 55;
(3) a heavy chain variable region and a light chain variable region, wherein the heavy chain variable region comprises the amino acid sequence set forth in SEQ ID NO: 54 or an amino acid sequence having at least 80% identity to the amino acid sequence set forth in SEQ ID NO: 54, and the light chain variable region comprises the amino acid sequence set forth in SEQ ID NO: 56 or an amino acid sequence having at least 80% identity to the amino acid sequence set forth in SEQ ID NO: 56;
(4) a heavy chain variable region and a light chain variable region, wherein the heavy chain variable region comprises the amino acid sequence set forth in SEQ ID NO: 54 or an amino acid sequence having at least 80% identity to the amino acid sequence set forth in SEQ ID NO: 54, and the light chain variable region comprises the amino acid sequence set forth in SEQ ID NO: 57 or an amino acid sequence having at least 80% identity to the amino acid sequence set forth in SEQ ID NO: 57;
(5) a heavy chain variable region and a light chain variable region, wherein the heavy chain variable region comprises the amino acid sequence set forth in SEQ ID NO: 54 or an amino acid sequence having at least 80% identity to the amino acid sequence set forth in SEQ ID NO: 54, and the light chain variable region comprises the amino acid sequence set forth in SEQ ID NO: 58 or an amino acid sequence having at least 80% identity to the amino acid sequence set forth in SEQ ID NO: 58;
(6) a heavy chain variable region and a light chain variable region, wherein the heavy chain variable region comprises the amino acid sequence set forth in SEQ ID NO: 59 or an amino acid sequence having at least 80% identity to the amino acid sequence set forth in SEQ ID NO: 59, and the light chain variable region comprises the amino acid sequence set forth in SEQ ID NO: 55 or an amino acid sequence having at least 80% identity to the amino acid sequence set forth in SEQ ID NO: 55;
(7) a heavy chain variable region and a light chain variable region, wherein the heavy chain variable region comprises the amino acid sequence set forth in SEQ ID NO: 59 or an amino acid sequence having at least 80% identity to the amino acid sequence set forth in SEQ ID NO: 59, and the light chain variable region comprises the amino acid sequence set forth in SEQ ID NO: 56 or an amino acid sequence having at least 80% identity to the amino acid sequence set forth in SEQ ID NO: 56;
(8) a heavy chain variable region and a light chain variable region, wherein the heavy chain variable region comprises the amino acid sequence set forth in SEQ ID NO: 59 or an amino acid sequence having at least 80% identity to the amino acid sequence set forth in SEQ ID NO: 59, and the light chain variable region comprises the amino acid sequence set forth in SEQ ID NO: 57 or an amino acid sequence having at least 80% identity to the amino acid sequence set forth in SEQ ID NO: 57;
(9) a heavy chain variable region and a light chain variable region, wherein the heavy chain variable region comprises the amino acid sequence set forth in SEQ ID NO: 59 or an amino acid sequence having at least 80% identity to the amino acid sequence set forth in SEQ ID NO: 59, and the light chain variable region comprises the amino acid sequence set forth in SEQ ID NO: 58 or an amino acid sequence having at least 80% identity to the amino acid sequence set forth in SEQ ID NO: 58;
(10) a heavy chain variable region and a light chain variable region, wherein the heavy chain variable region comprises the amino acid sequence set forth in SEQ ID NO: 60 or an amino acid sequence having at least 80% identity to the amino acid sequence set forth in SEQ ID NO: 60, and the light chain variable region comprises the amino acid sequence set forth in SEQ ID NO: 55 or an amino acid sequence having at least 80% identity to the amino acid sequence set forth in SEQ ID NO: 55;
(11) a heavy chain variable region and a light chain variable region, wherein the heavy chain variable region comprises the amino acid sequence set forth in SEQ ID NO: 60 or an amino acid sequence having at least 80% identity to the amino acid sequence set forth in SEQ ID NO: 60, and the light chain variable region comprises the amino acid sequence set forth in SEQ ID NO: 56 or an amino acid sequence having at least 80% identity to the amino acid sequence set forth in SEQ ID NO: 56;
(12) a heavy chain variable region and a light chain variable region, wherein the heavy chain variable region comprises the amino acid sequence set forth in SEQ ID NO: 60 or an amino acid sequence having at least 80% identity to the amino acid sequence set forth in SEQ ID NO: 60, and the light chain variable region comprises the amino acid sequence set forth in SEQ ID NO: 57 or an amino acid sequence having at least 80% identity to the amino acid sequence set forth in SEQ ID NO: 57;
(13) a heavy chain variable region and a light chain variable region, wherein the heavy chain variable region comprises the amino acid sequence set forth in SEQ ID NO: 60 or an amino acid sequence having at least 80% identity to the amino acid sequence set forth in SEQ ID NO: 60, and the light chain variable region comprises the amino acid sequence set forth in SEQ ID NO: 58 or an amino acid sequence having at least 80% identity to the amino acid sequence set forth in SEQ ID NO: 58;
(14) a heavy chain variable region and a light chain variable region, wherein the heavy chain variable region comprises the amino acid sequence set forth in SEQ ID NO: 61 or an amino acid sequence having at least 80% identity to the amino acid sequence set forth in SEQ ID NO: 61, and the light chain variable region comprises the amino acid sequence set forth in SEQ ID NO: 56 or an amino acid sequence having at least 80% identity to the amino acid sequence set forth in SEQ ID NO: 56;
(15) a heavy chain variable region and a light chain variable region, wherein the heavy chain variable region comprises the amino acid sequence set forth in SEQ ID NO: 61 or an amino acid sequence having at least 80% identity to the amino acid sequence set forth in SEQ ID NO: 61, and the light chain variable region comprises the amino acid sequence set forth in SEQ ID NO: 57 or an amino acid sequence having at least 80% identity to the amino acid sequence set forth in SEQ ID NO: 57;
(16) a heavy chain variable region and a light chain variable region, wherein the heavy chain variable region comprises the amino acid sequence set forth in SEQ ID NO: 61 or an amino acid sequence having at least 80% identity to the amino acid sequence set forth in SEQ ID NO: 61, and the light chain variable region comprises the amino acid sequence set forth in SEQ ID NO: 58 or an amino acid sequence having at least 80% identity to the amino acid sequence set forth in SEQ ID NO: 58;
(17) a heavy chain variable region and a light chain variable region, wherein the heavy chain variable region comprises the amino acid sequence set forth in SEQ ID NO: 62 or an amino acid sequence having at least 80% identity to the amino acid sequence set forth in SEQ ID NO: 62, and the light chain variable region comprises the amino acid sequence set forth in SEQ ID NO: 63 or an amino acid sequence having at least 80% identity to the amino acid sequence set forth in SEQ ID NO: 63;
(18) a heavy chain variable region and a light chain variable region, wherein the heavy chain variable region comprises the amino acid sequence set forth in SEQ ID NO: 62 or an amino acid sequence having at least 80% identity to the amino acid sequence set forth in SEQ ID NO: 62, and the light chain variable region comprises the amino acid sequence set forth in SEQ ID NO: 64 or an amino acid sequence having at least 80% identity to the amino acid sequence set forth in SEQ ID NO: 64;
(19) a heavy chain variable region and a light chain variable region, wherein the heavy chain variable region comprises the amino acid sequence set forth in SEQ ID NO: 62 or an amino acid sequence having at least 80% identity to the amino acid sequence set forth in SEQ ID NO: 62, and the light chain variable region comprises the amino acid sequence set forth in SEQ ID NO: 65 or an amino acid sequence having at least 80% identity to the amino acid sequence set forth in SEQ ID NO: 65;
(20) a heavy chain variable region and a light chain variable region, wherein the heavy chain variable region comprises the amino acid sequence set forth in SEQ ID NO: 62 or an amino acid sequence having at least 80% identity to the amino acid sequence set forth in SEQ ID NO: 62, and the light chain variable region comprises the amino acid sequence set forth in SEQ ID NO: 66 or an amino acid sequence having at least 80% identity to the amino acid sequence set forth in SEQ ID NO: 66;
(21) a heavy chain variable region and a light chain variable region, wherein the heavy chain variable region comprises the amino acid sequence set forth in SEQ ID NO: 62 or an amino acid sequence having at least 80% identity to the amino acid sequence set forth in SEQ ID NO: 62, and the light chain variable region comprises the amino acid sequence set forth in SEQ ID NO: 67 or an amino acid sequence having at least 80% identity to the amino acid sequence set forth in SEQ ID NO: 67;
(22) a heavy chain variable region and a light chain variable region, wherein the heavy chain variable region comprises the amino acid sequence set forth in SEQ ID NO: 62 or an amino acid sequence having at least 80% identity to the amino acid sequence set forth in SEQ ID NO: 62, and the light chain variable region comprises the amino acid sequence set forth in SEQ ID NO: 68 or an amino acid sequence having at least 80% identity to the amino acid sequence set forth in SEQ ID NO: 68;
(23) a heavy chain variable region and a light chain variable region, wherein the heavy chain variable region comprises the amino acid sequence set forth in SEQ ID NO: 69 or an amino acid sequence having at least 80% identity to the amino acid sequence set forth in SEQ ID NO: 69, and the light chain variable region comprises the amino acid sequence set forth in SEQ ID NO: 63 or an amino acid sequence having at least 80% identity to the amino acid sequence set forth in SEQ ID NO: 63;
(24) a heavy chain variable region and a light chain variable region, wherein the heavy chain variable region comprises the amino acid sequence set forth in SEQ ID NO: 69 or an amino acid sequence having at least 80% identity to the amino acid sequence set forth in SEQ ID NO: 69, and the light chain variable region comprises the amino acid sequence set forth in SEQ ID NO: 64 or an amino acid sequence having at least 80% identity to the amino acid sequence set forth in SEQ ID NO: 64;
(25) a heavy chain variable region and a light chain variable region, wherein the heavy chain variable region comprises the amino acid sequence set forth in SEQ ID NO: 69 or an amino acid sequence having at least 80% identity to the amino acid sequence set forth in SEQ ID NO: 69, and the light chain variable region comprises the amino acid sequence set forth in SEQ ID NO: 65 or an amino acid sequence having at least 80% identity to the amino acid sequence set forth in SEQ ID NO: 65;
(26) a heavy chain variable region and a light chain variable region, wherein the heavy chain variable region comprises the amino acid sequence set forth in SEQ ID NO: 69 or an amino acid sequence having at least 80% identity to the amino acid sequence set forth in SEQ ID NO: 69, and the light chain variable region comprises the amino acid sequence set forth in SEQ ID NO: 66 or an amino acid sequence having at least 80% identity to the amino acid sequence set forth in SEQ ID NO: 66;
(27) a heavy chain variable region and a light chain variable region, wherein the heavy chain variable region comprises the amino acid sequence set forth in SEQ ID NO: 70 or an amino acid sequence having at least 80% identity to the amino acid sequence set forth in SEQ ID NO: 70, and the light chain variable region comprises the amino acid sequence set forth in SEQ ID NO: 63 or an amino acid sequence having at least 80% identity to the amino acid sequence set forth in SEQ ID NO: 63;
(28) a heavy chain variable region and a light chain variable region, wherein the heavy chain variable region comprises the amino acid sequence set forth in SEQ ID NO: 70 or an amino acid sequence having at least 80% identity to the amino acid sequence set forth in SEQ ID NO: 70, and the light chain variable region comprises the amino acid sequence set forth in SEQ ID NO: 64 or an amino acid sequence having at least 80% identity to the amino acid sequence set forth in SEQ ID NO: 64;
(29) a heavy chain variable region and a light chain variable region, wherein the heavy chain variable region comprises the amino acid sequence set forth in SEQ ID NO: 70 or an amino acid sequence having at least 80% identity to the amino acid sequence set forth in SEQ ID NO: 70, and the light chain variable region comprises the amino acid sequence set forth in SEQ ID NO: 65 or an amino acid sequence having at least 80% identity to the amino acid sequence set forth in SEQ ID NO: 65;
(30) a heavy chain variable region and a light chain variable region, wherein the heavy chain variable region comprises the amino acid sequence set forth in SEQ ID NO: 70 or an amino acid sequence having at least 80% identity to the amino acid sequence set forth in SEQ ID NO: 70, and the light chain variable region comprises the amino acid sequence set forth in SEQ ID NO: 66 or an amino acid sequence having at least 80% identity to the amino acid sequence set forth in SEQ ID NO: 66;
(31) a heavy chain variable region and a light chain variable region, wherein the heavy chain variable region comprises the amino acid sequence set forth in SEQ ID NO: 70 or an amino acid sequence having at least 80% identity to the amino acid sequence set forth in SEQ ID NO: 70, and the light chain variable region comprises the amino acid sequence set forth in SEQ ID NO: 67 or an amino acid sequence having at least 80% identity to the amino acid sequence set forth in SEQ ID NO: 67;
(32) a heavy chain variable region and a light chain variable region, wherein the heavy chain variable region comprises the amino acid sequence set forth in SEQ ID NO: 70 or an amino acid sequence having at least 80% identity to the amino acid sequence set forth in SEQ ID NO: 70, and the light chain variable region comprises the amino acid sequence set forth in SEQ ID NO: 68 or an amino acid sequence having at least 80% identity to the amino acid sequence set forth in SEQ ID NO: 68;
(33) a heavy chain variable region and a light chain variable region, wherein the heavy chain variable region comprises the amino acid sequence set forth in SEQ ID NO: 71 or an amino acid sequence having at least 80% identity to the amino acid sequence set forth in SEQ ID NO: 71, and the light chain variable region comprises the amino acid sequence set forth in SEQ ID NO: 63 or an amino acid sequence having at least 80% identity to the amino acid sequence set forth in SEQ ID NO: 63;
(34) a heavy chain variable region and a light chain variable region, wherein the heavy chain variable region comprises the amino acid sequence set forth in SEQ ID NO: 71 or an amino acid sequence having at least 80% identity to the amino acid sequence set forth in SEQ ID NO: 71, and the light chain variable region comprises the amino acid sequence set forth in SEQ ID NO: 64 or an amino acid sequence having at least 80% identity to the amino acid sequence set forth in SEQ ID NO: 64;
(35) a heavy chain variable region and a light chain variable region, wherein the heavy chain variable region comprises the amino acid sequence set forth in SEQ ID NO: 71 or an amino acid sequence having at least 80% identity to the amino acid sequence set forth in SEQ ID NO: 71, and the light chain variable region comprises the amino acid sequence set forth in SEQ ID NO: 65 or an amino acid sequence having at least 80% identity to the amino acid sequence set forth in SEQ ID NO: 65;
(36) a heavy chain variable region and a light chain variable region, wherein the heavy chain variable region comprises the amino acid sequence set forth in SEQ ID NO: 71 or an amino acid sequence having at least 80% identity to the amino acid sequence set forth in SEQ ID NO: 71, and the light chain variable region comprises the amino acid sequence set forth in SEQ ID NO: 66 or an amino acid sequence having at least 80% identity to the amino acid sequence set forth in SEQ ID NO: 66;
(37) a heavy chain variable region and a light chain variable region, wherein the heavy chain variable region comprises the amino acid sequence set forth in SEQ ID NO: 71 or an amino acid sequence having at least 80% identity to the amino acid sequence set forth in SEQ ID NO: 71, and the light chain variable region comprises the amino acid sequence set forth in SEQ ID NO: 67 or an amino acid sequence having at least 80% identity to the amino acid sequence set forth in SEQ ID NO: 67;
(38) a heavy chain variable region and a light chain variable region, wherein the heavy chain variable region comprises the amino acid sequence set forth in SEQ ID NO: 71 or an amino acid sequence having at least 80% identity to the amino acid sequence set forth in SEQ ID NO: 71, and the light chain variable region comprises the amino acid sequence set forth in SEQ ID NO: 68 or an amino acid sequence having at least 80% identity to the amino acid sequence set forth in SEQ ID NO: 68;
(39) a heavy chain variable region and a light chain variable region, wherein the heavy chain variable region comprises the amino acid sequence set forth in SEQ ID NO: 72 or an amino acid sequence having at least 80% identity to the amino acid sequence set forth in SEQ ID NO: 72, and the light chain variable region comprises the amino acid sequence set forth in SEQ ID NO: 63 or an amino acid sequence having at least 80% identity to the amino acid sequence set forth in SEQ ID NO: 63;
(40) a heavy chain variable region and a light chain variable region, wherein the heavy chain variable region comprises the amino acid sequence set forth in SEQ ID NO: 72 or an amino acid sequence having at least 80% identity to the amino acid sequence set forth in SEQ ID NO: 72, and the light chain variable region comprises the amino acid sequence set forth in SEQ ID NO: 65 or an amino acid sequence having at least 80% identity to the amino acid sequence set forth in SEQ ID NO: 65;
(41) a heavy chain variable region and a light chain variable region, wherein the heavy chain variable region comprises the amino acid sequence set forth in SEQ ID NO: 72 or an amino acid sequence having at least 80% identity to the amino acid sequence set forth in SEQ ID NO: 72, and the light chain variable region comprises the amino acid sequence set forth in SEQ ID NO: 67 or an amino acid sequence having at least 80% identity to the amino acid sequence set forth in SEQ ID NO: 67;
(42) a heavy chain variable region and a light chain variable region, wherein the heavy chain variable region comprises the amino acid sequence set forth in SEQ ID NO: 72 or an amino acid sequence having at least 80% identity to the amino acid sequence set forth in SEQ ID NO: 72, and the light chain variable region comprises the amino acid sequence set forth in SEQ ID NO: 68 or an amino acid sequence having at least 80% identity to the amino acid sequence set forth in SEQ ID NO: 68;
(43) a heavy chain variable region and a light chain variable region, wherein the heavy chain variable region comprises the amino acid sequence set forth in SEQ ID NO: 73 or an amino acid sequence having at least 80% identity to the amino acid sequence set forth in SEQ ID NO: 73, and the light chain variable region comprises the amino acid sequence set forth in SEQ ID NO: 63 or an amino acid sequence having at least 80% identity to the amino acid sequence set forth in SEQ ID NO: 63;
(44) a heavy chain variable region and a light chain variable region, wherein the heavy chain variable region comprises the amino acid sequence set forth in SEQ ID NO: 73 or an amino acid sequence having at least 80% identity to the amino acid sequence set forth in SEQ ID NO: 73, and the light chain variable region comprises the amino acid sequence set forth in SEQ ID NO: 65 or an amino acid sequence having at least 80% identity to the amino acid sequence set forth in SEQ ID NO: 65;
(45) a heavy chain variable region and a light chain variable region, wherein the heavy chain variable region comprises the amino acid sequence set forth in SEQ ID NO: 73 or an amino acid sequence having at least 80% identity to the amino acid sequence set forth in SEQ ID NO: 73, and the light chain variable region comprises the amino acid sequence set forth in SEQ ID NO: 67 or an amino acid sequence having at least 80% identity to the amino acid sequence set forth in SEQ ID NO: 67;
(46) a heavy chain variable region and a light chain variable region, wherein the heavy chain variable region comprises the amino acid sequence set forth in SEQ ID NO: 73 or an amino acid sequence having at least 80% identity to the amino acid sequence set forth in SEQ ID NO: 73, and the light chain variable region comprises the amino acid sequence set forth in SEQ ID NO: 68 or an amino acid sequence having at least 80% identity to the amino acid sequence set forth in SEQ ID NO: 68;
(47) a heavy chain variable region and a light chain variable region, wherein the heavy chain variable region comprises the amino acid sequence set forth in SEQ ID NO: 74 or an amino acid sequence having at least 80% identity to the amino acid sequence set forth in SEQ ID NO: 74, and the light chain variable region comprises the amino acid sequence set forth in SEQ ID NO: 63 or an amino acid sequence having at least 80% identity to the amino acid sequence set forth in SEQ ID NO: 63;
(48) a heavy chain variable region and a light chain variable region, wherein the heavy chain variable region comprises the amino acid sequence set forth in SEQ ID NO: 74 or an amino acid sequence having at least 80% identity to the amino acid sequence set forth in SEQ ID NO: 74, and the light chain variable region comprises the amino acid sequence set forth in SEQ ID NO: 67 or an amino acid sequence having at least 80% identity to the amino acid sequence set forth in SEQ ID NO: 67; or
(49) a heavy chain variable region and a light chain variable region, wherein the heavy chain variable region comprises the amino acid sequence set forth in SEQ ID NO: 74 or an amino acid sequence having at least 80% identity to the amino acid sequence set forth in SEQ ID NO: 74, and the light chain variable region comprises the amino acid sequence set forth in SEQ ID NO: 68 or an amino acid sequence having at least 80% identity to the amino acid sequence set forth in SEQ ID NO: 68.

4. The anti-TL1A antibody or the antigen-binding fragment thereof according to any one of claims 1 to 3, wherein, compared to the amino acid sequence set forth in any one of SEQ ID NOs: 61, 54, 59, 60, 62, 69, 70, 71, 72, 73, 74, 55, 56, 57, 58, 63, 64, 65, 66, 67 and 68, the differing amino acids in the amino acid sequence having at least 80% identity are located mainly or entirely in an FR region.

5. The anti-TL1A antibody or the antigen-binding fragment thereof according to any one of claims 1 to 4, wherein the anti-TL1A antibody or the antigen-binding fragment thereof further comprises a heavy chain constant region and/or a light chain constant region;
preferably, the heavy chain constant region is a heavy chain constant region of IgG, IgA, IgM, IgE or IgD or a variant thereof; the heavy chain constant region is preferably a heavy chain constant region of IgG or a variant thereof; the heavy chain constant region is more preferably a heavy chain constant region of human IgG1 or a variant thereof; and
preferably, the light chain constant region is a constant region of a human κ chain or λ chain or a variant thereof.

6. The anti-TL1A antibody or the antigen-binding fragment thereof according to claim 5, wherein the heavy chain constant region comprises a full-length heavy chain constant region or a fragment thereof, and the fragment is optionally selected from a CH1 region, a CH2 region, a CH3 region and an Fc region.

7. The anti-TL1A antibody or the antigen-binding fragment thereof according to claim 5, wherein the Fc region is a human IgG1 Fc region; preferably, the human IgG1 Fc region further has any one or more mutations selected from the group consisting of 329A, 329G, 329Y, 331S, 236F, 236R, 238A, 238E, 238G, 238H, 238I, 238V, 238W, 238Y, 248A, 254D, 254E, 254G, 254H, 254I, 254N, 254P, 254Q, 254T, 254V, 264S, 265H, 265K, 265S, 265Y, 265A, 267G, 267H, 267I, 267K, 434I, 438G, 439E, 439H, 439Q, 440A, 440D, 440E, 440F, 440M, 440T and 440V, according to Kabat numbering.

8. The anti-TL1A antibody or the antigen-binding fragment thereof according to any one of claims 5 to 7, wherein the heavy chain constant region comprises the amino acid sequence set forth in SEQ ID NO: 77 or 75, or an amino acid sequence having at least 80% identity thereto; and/or
the light chain constant region comprises the amino acid sequence set forth in SEQ ID NO: 76, or an amino acid sequence having at least 80% identity thereto.

9. The anti-TL1A antibody or the antigen-binding fragment thereof according to any one of claims 1 to 8, wherein the anti-TL1A antibody or the antigen-binding fragment thereof comprises:
a heavy chain, wherein the heavy chain comprises a heavy chain variable region and a heavy chain constant region, the heavy chain variable region comprises an amino acid sequence set forth in any one of SEQ ID NOs: 61, 54, 59, 60, 62, 69, 70, 71, 72, 73 and 74 or an amino acid sequence having at least 80% identity to the amino acid sequence set forth in any one of SEQ ID NOs: 61, 54, 59, 60, 62, 69, 70, 71, 72, 73 and 74, and the heavy chain constant region comprises the amino acid sequence set forth in SEQ ID NO: 75 or an amino acid sequence having at least 80% identity to the amino acid sequence set forth in SEQ ID NO: 75; and
a light chain, wherein the light chain comprises a light chain variable region and a light chain constant region, the light chain variable region comprises an amino acid sequence set forth in any one of SEQ ID NOs: 55, 56, 57, 58, 63, 64, 65, 66, 67 and 68 or an amino acid sequence having at least 80% identity to the amino acid sequence set forth in any one of SEQ ID NOs: 55, 56, 57, 58, 63, 64, 65, 66, 67 and 68, and the light chain constant region comprises the amino acid sequence set forth in SEQ ID NO: 76 or an amino acid sequence having at least 80% identity to the amino acid sequence set forth in SEQ ID NO: 76; and
preferably, the anti-TL1A antibody or the antigen-binding fragment thereof comprises:
(1) a heavy chain comprising the amino acid sequence set forth in SEQ ID NO: 78 or an amino acid sequence having at least 80% identity to the amino acid sequence set forth in SEQ ID NO: 78, and
a light chain comprising the amino acid sequence set forth in SEQ ID NO: 79 or an amino acid sequence having at least 80% identity to the amino acid sequence set forth in SEQ ID NO: 79;
(2) a heavy chain comprising the amino acid sequence set forth in SEQ ID NO: 78 or an amino acid sequence having at least 80% identity to the amino acid sequence set forth in SEQ ID NO: 78, and
a light chain comprising the amino acid sequence set forth in SEQ ID NO: 80 or an amino acid sequence having at least 80% identity to the amino acid sequence set forth in SEQ ID NO: 80;
(3) a heavy chain comprising the amino acid sequence set forth in SEQ ID NO: 81 or an amino acid sequence having at least 80% identity to the amino acid sequence set forth in SEQ ID NO: 81, and
a light chain comprising the amino acid sequence set forth in SEQ ID NO: 82 or an amino acid sequence having at least 80% identity to the amino acid sequence set forth in SEQ ID NO: 82;
(4) a heavy chain comprising the amino acid sequence set forth in SEQ ID NO: 83 or an amino acid sequence having at least 80% identity to the amino acid sequence set forth in SEQ ID NO: 83, and
a light chain comprising the amino acid sequence set forth in SEQ ID NO: 82 or an amino acid sequence having at least 80% identity to the amino acid sequence set forth in SEQ ID NO: 82; or
(5) a heavy chain comprising the amino acid sequence set forth in SEQ ID NO: 81 or an amino acid sequence having at least 80% identity to the amino acid sequence set forth in SEQ ID NO: 81, and
a light chain comprising the amino acid sequence set forth in SEQ ID NO: 84 or an amino acid sequence having at least 80% identity to the amino acid sequence set forth in SEQ ID NO: 84.

10. The anti-TL1A antibody or the antigen-binding fragment thereof according to any one of claims 1 to 9, wherein the antibody is a murine antibody, a monkey antibody, a rabbi antibody, a chimeric antibody, a humanized antibody, or a fully human antibody; and/or
the anti-TL1A antibody is selected from a monoclonal antibody, a polyclonal antibody, a neutralizing antibody, an antagonistic antibody, an anti-idiotypic antibody, an afucosylated antibody, a monospecific antibody, a multispecific antibody, and a nanobody, and the antigen-binding fragment is at least one selected from a Fab fragment, a Fab' fragment, a F(ab')2 fragment, an Fd fragment, an Fv fragment, a dAb fragment, an isolated CDR region, and an scFv; and/or
the anti-TL1A antibody is a monoclonal antibody or a multispecific antibody; preferably, the multispecific antibody is a bispecific antibody, a trispecific antibody or a tetraspecific antibody.

11. A nucleic acid molecule, wherein the nucleic acid molecule encodes the anti-TL1A antibody or the antigen-binding fragment thereof according to any one of claims 1 to 10.

12. A recombinant vector, comprising the nucleic acid molecule of claim 11.

13. A host cell, comprising the nucleic acid molecule of claim 11 or the recombinant vector of claim 12.

14. The anti-TL1A antibody or the antigen-binding fragment thereof according to any one of claims 1 to 10, the nucleic acid molecule of claim 11, the recombinant vector of claim 12, or the host cell of claim 13, for use in preventing and/or treating a TL1A-mediated related disease or disorder;
preferably, the disease or disorder is prevented, alleviated, ameliorated or inhibited by eliminating, inhibiting or reducing TL1A activity;
preferably, the disease is an immune-related or inflammatory disease;
preferably, the disease or disorder is at least one selected from the group consisting of inflammatory bowel disease (IBD), irritable bowel syndrome (IBS), systemic sclerosis-associated interstitial lung disease, idiopathic pulmonary fibrosis, virus-induced pulmonary fibrosis, asthma, chronic obstructive pulmonary disease (COPD), allergic pulmonary inflammation, neurodegenerative disease, cancer, diabetes, arthritis, rheumatoid arthritis, juvenile rheumatoid arthritis, osteoarthritis, psoriatic arthritis, ankylosing spondylitis, multiple sclerosis, transplant rejection, graft-versus-host disease (GVHD), spondyloarthropathy, primary sclerosing cholangitis, primary biliary cirrhosis, atherosclerosis, bladder syndrome, interstitial cystitis, urinary and bowel dysfunction, sepsis, uveitis, encephalomyelitis, myasthenia gravis, systemic lupus erythematosus, cutaneous lupus erythematosus, autoimmune thyroiditis, atopic dermatitis, eczematous dermatitis, psoriasis, Sjögren's syndrome, scleroderma, and vasculitis;
preferably, the inflammatory bowel disease comprises Crohn's disease and ulcerative colitis; and
more preferably, the disease or disorder is inflammatory bowel disease.

15. A kit for detecting TL1A, comprising the anti-TL1A antibody or the antigen-binding fragment thereof according to any one of claims 1 to 10, and/or the nucleic acid molecule of claim 11.
